Europäisches Patentamt

European Patent Office

Office européen des brevets

(19)

(11) **EP 1 700 914 A1**

(12)

# EUROPEAN PATENT APPLICATION
published in accordance with Art. 158(3) EPC

(43) Date of publication:
**13.09.2006 Bulletin 2006/37**

(51) Int Cl.:
*C12N 15/09* (2006.01)    *C12Q 1/02* (2006.01)
*C12Q 1/68* (2006.01)    *A01K 67/027* (2006.01)

(21) Application number: **04819768.5**

(22) Date of filing: **19.11.2004**

(86) International application number:
**PCT/JP2004/017307**

(87) International publication number:
**WO 2005/054463 (16.06.2005 Gazette 2005/24)**

(84) Designated Contracting States:
**DE FR GB**

(30) Priority: **21.11.2003 JP 2003393175**

(71) Applicants:
  • **Osaka Industrial Promotion Organization
    Osaka-shi, Osaka 540-0029 (JP)**
  • **National Institute of Radiological Sciences
    Chiba-shi,
    Chiba-ken 263-8555 (JP)**

(72) Inventors:
  • **TAKEDA, Junji
    2-1, Yamadaoka, Suita-shi, Osaka 5650871 (JP)**

  • **HORIE, Kyoji
    2-2, Yamadaoka, Suita-shi, Osaka 5650871 (JP)**
  • **YUSA, Kosuke
    2-2, Yamadaoka, Suita-shi, Osaka 5650871 (JP)**
  • **ISHIHARA, Hiroshi
    Anagawa, Inage-ku, Chiba 2638555 (JP)**

(74) Representative: **Harding, Charles Thomas
    D Young & Co
    120 Holborn
    London EC1N 2DY (GB)**

(54) **DEVELOPMENT OF MAMMALIAN GENOME MODIFICATION TECHNIQUE USING RETROTRANSPOSON**

(57)     To comprehensively modify genome, it is intended to develop a transposition system of the copy and paste type which has an improved efficiency. This object has been achieved by the finding that an LTR retrotransposon is partly usable in a transposition system. Namely, a technique of efficiently transferring a foreign gene into a cell by using a transposon. More specifically speaking, a complete IPA element and a functional promoter sequence are found out. It is clarified that, without a combination of them, a retrotransposon cannot exert its function.

**Description**

TECHNICAL FIELD

The present invention is related to a system, kit and composition for introducing a foreign nucleic acid molecule into a cell, and for modifying a genome. Further, the present invention is related to the production of a transgenic organism and a composition, kit and system used therefor. Hereinafter, the detailed description of the present invention is provided.

BACKGROUND ART

Transgenic organisms are an important technology. Due to their broad applications, transgenic organisms are receiving notable present attention within the scientific community. However, methods for efficiently producing transgenic organisms are not currently well developed, and therefore, development of such methods for efficiently producing transgenic organisms are of note.

Recently, production of transgenic biological organisms using transposons have been attempted. A transposon (or alternatively called "transposable element") is a nucleic acid molecule or sequence with repeated sequences in parallel. Transposase is an enzyme that promotes integration of a different nucleic acid into another nucleic acid molecule. Usually, the sequence encoding the enzyme transposase exists within the transposon.

DNA-type transposons are found in a relatively broad range of biological organisms, and the phenomenon attained thereby is believed to be ubiquitous. Transposition is achieved by a cut-and-paste mechanism. Transposons have also been found in vertebrates (Radice, A.D. et al., 1994.Mol. Gen. Genet. 244,606-612). Further, a number of transposons have been isolated from a variety of organisms such as fish, amphibia, mammals and the like, including those belonging to the super families Tc1/mariner, hAT(hobo/Ac/Tam) and the like (Oosumi et al., 1995. Nature 378, 873; Ivics et al., 1995. Mol. Gen. Genet. 247, 312-322; Koga et al. 1996. Nature 383, 30; Lam et al., 1996. J. Mol. Biol. 257,359-366, and Lam, W. L. et al., Proc. Natl. Acad. Sci. USA 93, 10870-10875). Transposases are known to catalyze or facilitate excision from the original location of a transposon and reintegration thereof (Plasterk, RHA., 1999, TIG 15: 326-332; Plasterk RHA., 1996 Curr. Top. Microbiol. Immunol. 204, 125-143). An autonomous element of transposons can express active transposases, which are a transacting factor, and thus have the property of the transposon *per se* and can also transpose. A non autonomous element may be a *cis*-acting element, subsequently known as a inverted terminal repeat sequence. Some inverted repeat sequences may also include one or more tandem repeat sequences. Such a sequence is included in a terminal inverted repeat sequence and may be used for transposition from another element in the presence of a complementary transposase.

A method for introducing DNA into a cell is known, and includes for example, DNA aggregation reagents (e.g. calcium phosphate, polyethylene glycol and the like), lipid-containing reagents (e.g., liposome, multi-lamellar vesicle and the like), and virus mediated methods, and the like. These methods have their own deficiencies. For example, DNA aggregation reagents and virus mediated methods, have deficiencies where the size of the DNA insert is limited, and the amount of nucleic acids to be introduced is limited. The promotion of integration of the transgene is not always advantageous.

There is still a demand for a method for introducing DNA into a cell. In particular, efficient integration of a nucleic acid fragment of a variety of sizes into the nucleic acid of a cell, specifically a method for promoting integration of DNA into the genome of a cell. DNA-type transposon has also be developed for use in transposition systems, in which transposon transposition frequency is only about 5-6 % in hepatocytes with the introduced gene therein. As such, the efficiency of the method for gene introduction is low, and does not allow systematic preparation of transgenic animals. Furthermore, in conventional methods, it was difficult to randomly introduce mutations into a number of genes in the body of an individual animal, and resulting expression level was also low. As such, it was necessary to design a general method for inducing mutagenesis.

However, a DNA-type transposon as described above has a tendency to focus its transposition sites in the vicinity of the sites before the transposition. It is not possible to increase transposition and the number of copies of transposons before transposition in each cell, due to the properties of the cut and paste mechanisms. On the other hand, retrotransposon, an RNA-type transposon is also of note. It is understood that using RNA-type transposon, transposition achieved thereby does not depend on the site before transposition, and thus transposition shall occur in a wide range of site within the genome. Accordingly, it is advantageous for the purpose of exhaustive modification of the genome. Further, due to properties of copy and paste mechanisms, the sequence before the transposition is also copied when the transposition occurs. As such, it is possible to increase transposition beyond the number of the copies before the transposition.

Until now, it has been attempted to develop a transposition system with a retrotransposon using the LINE1 vector (see, non-patent literature 1, patent literatures 1 and 2). However, systems using LINE1 is accompanied by a high frequency of deletion of vectors on insertion of a transposition into the genome, and thus there is a problem of having limitation in terms of scope of applications.

Retrotransposons have a category called LTR-type, further to this, there is a retrotransposon, so-called intracisternal A particle (IAP) (see, Patent Reference 2). IAP vectors conventionally used to date have a deletion in the genes essential for transposition, and thus a vector comprising a complete retrotransposon has not yet been achieved, and thus there was a defect or problem in that it is difficult to control transposition (non-patent literature 2).

[non-patent literature 1]

Ostertag, E. M., et al., Nat Genet. 32, 655-660,2002

[non-patent literature 2]

Heidmann O., et al., Cell 64, 159-170, 1991

[patent literature 1]

United States Patent No. 6150160

[patent literature 2]

United States Patent Application Laid-Open Publication No. 2003-0121063

DISCLOSURE OF THE INVENTION

(Problems to be solved by the invention)

It is an object of the present invention to develop a more efficient copy & paste type transposition system so as to exhaustively modify a genome. It is also an object of the present invention to develop a system enabling easy observation of transposition activity of a retrotransposon.

(Means for solving the problems)

The above-mentioned objects have been solved by in part as a result of extensive inventive study, by discovering that LTR-type retrotransposon can be used in a transposition system. The present invention also solved the problem partially by success in development of a system in which a specific promoter is inserted to allow transposition activity of an LTR-type transposon.

The present invention is related to technology of efficiently introducing a foreign gene into a cell using a transposon. More specifically, the above-mentioned problem has been solved by discovering a full competent IAP element and a functional promoter sequence, and combining the same to allow detection of a functional retrotransposon for the first time.

As such, the present invention provides the following:

1. An isolated nucleic acid construct comprising a nucleic acid sequence encoding an LTR-type retrotransposon.

2. A nucleic acid construct according to Item 1 wherein the LTR-type retrotransposon comprises Intracisternal A particle (IAP)-type retrotransposon.

3. A nucleic acid construct according to Item 1 wherein the retrotransposon comprises a full-length IAP element.

4. A nucleic acid construct according to Item 1 wherein the retrotransposon encodes a polypeptide having a function.

5. A nucleic acid construct according to Item 1 wherein the function comprises at least one activity selected from the group consisting of transcription activity, reverse transcription activity and integrase activity.

6. A nucleic acid construct according to Item 1 wherein the retrotransposon is an IAP element and at least one domain selected from the group consisting of LTR, gag, pol and tRNA binding site is conserved against SEQ ID NO: 1.

7. A nucleic acid construct according to Item 1, wherein the retrotransposon is from an animal.

8. A nucleic acid construct according to Item 1, wherein the retrotransposon is from a mammal.

9. A nucleic acid construct according to Item 1, wherein the retrotransposon is from a rodent or primate.

10. A nucleic acid construct according to Item 1, wherein the retrotransposon is from a mouse.

11. A nucleic acid construct according to Item 1 wherein the retrotransposon is an IAP element, wherein the nucleic acid thereof has at least one feature selected from the group consisting of repeat of a sequence of tccgggacgagaaaa in the tRNA binding site immediately located at LTR at the 5' side, and inclusion of two or more repeat sequences ttgcttcttgctctc in the R region.

12. A nucleic acid construct according to Item 1 wherein the retrotransposon comprises:

(a) a polynucleotide having a base sequence set forth in SEQ ID NO: 1 or a fragment sequence thereof;
(b) a polynucleotide encoding a polypeptide consisting of an amino acid sequence set forth in SEQ ID NO: 2, or 3 and 4, or a fragment thereof;
(c) a polynucleotide encoding a variant polypeptide consisting of an amino acid sequence set forth in SEQ ID NO: 2, or 3 and 4 with at least one mutation selected from consisting of at least one amino acid substitution, addition and deletion, or a fragment thereof, which possesses a biological activity;
(d) a polynucleotide being a splice variant or allelic variant of the base sequence set forth in SEQ ID NO: 1, or a fragment thereof;
(e) a polynucleotide encoding a species homolog of a polypeptide consisting of an amino acid sequence set forth in SEQ ID NO: 2, or 3 and 4, or a fragment thereof;
(f) a polynucleotide which is hybridizes to any of polynucleotides (a) through (e) or the complement thereof under stringent conditions, and encoding a polypeptide having a biological activity; or
(g) a polynucleotide having at least 70 % identity to any of polynucleotides (a) through (e) or the complement thereof under stringent conditions, and encoding a polypeptide having a biological activity.

13. A nucleic acid construct according to Item 1 wherein the nucleic acid sequence encoding the retrotransposon comprises SEQ ID NO: 1.

14. A nucleic acid construct according to Item 1 further comprising a promoter sequence.

15. A nucleic acid construct according to Item 14 wherein the promoter sequence has an activity of 0.1 rlu or greater when determined by a luciferase assay *in vitro*.

16. A nucleic acid construct according to Item 14 wherein the promoter sequence is selected from the group consisting of CMV, CA and the variants thereof.

17. A nucleic acid construct according to Item 14 wherein the promoter sequence partially substitutes a portion of 5'LTR of the LTR-type retrotransposon.

18. A nucleic acid construct according to Item 17 wherein the promoter sequence substitutes an entirety or portion of U3 region in the 5' LTR in the LTR-type retrotransposon.

19. A nucleic acid construct according to Item 14 wherein the promoter sequence is operably linked to the retrotransposon.

20. A nucleic acid construct according to Item 14 wherein the promoter sequence is located in frame to a transcription initiation site of the retrotransposon at the transcription initiation site of the promoter sequence.

21. A nucleic acid construct according to Item 1 further comprising a sequence encoding a foreign gene.

22. A nucleic acid construct according to Item 21 wherein the sequence encoding the foreign gene is placed within the retrotransposon.

23. A nucleic acid construct according to Item 21 wherein the foreign gene provides a host with a distinguishable property.

24. A nucleic acid construct according to Item 23 wherein the distinguishable property is selected from the group consisting of antibiotic resistance, complement of nutrition, enzymatic activity and fluorescence.

25. A nucleic acid construct according to Item 21, wherein the foreign gene is selected from the group consisting of neo, GFP, hyg, puro, zeo, bsr, lacZ, CFP, YFP, RFP, BFP and hrGFP.

26. A nucleic acid construct according to Item 21, wherein the foreign gene is composed such that the foreign gene is first expressed only after transcription, reverse transcription and insertion into the genome is subjected to.

27. A nucleic acid construct according to Item 21, wherein the foreign gene comprises an intron sequence.

28. A nucleic acid construct according to Item 27, wherein the intron sequence is located in the same transcription direction (forward) with respect to the retrotransposon.

29. A nucleic acid construct according to Item 27, wherein the intron sequence is located between a splice donor sequence and a splice acceptor sequence.

30. A nucleic acid construct according to Item 1 for use in genomic modification.

31. A nucleic acid construct according to Item 15 which is for confirming whether or not the retrotransposon has transposition ability.

32. A nucleic acid construct according to Item 21 which is for transposing the foreign gene.

33. A nucleic acid construct according to Item 21 which is used for introducing the foreign gene into a host.

34. A nucleic acid construct according to Item 33 wherein the host comprises a eukaryotic organism.

35. A nucleic acid construct according to Item 33 wherein the host comprises a mammal.

36. A nucleic acid construct according to Item 33 wherein the host comprises a rodent or a primate.

37. A nucleic acid construct according to Item 33 wherein the host is a mouse.

38. A vector comprising a nucleic acid construct according to any one of Items 1-37.

39. A composition comprising a nucleic acid construct according to any one of Items 1-37, and a carrier.

40. A cell comprising a nucleic acid construct according to any one of Items 1-37.

41. An organism comprising a nucleic acid construct according to any one of Items 1-37, or a portion thereof.

42. A method for modifying a genome in a cell, comprising the steps of:

A) providing a nucleic acid construct comprising an LTR-type retrotransposon;
B) introducing the nucleic acid construct into the cell;
C) culturing the cell for a predetermined period of time; and
D) selecting a cell with a genome modified by means of the nucleic acid construct.

43. A method according to Item 42, further comprising a promoter having an activity of 0.1 rlu or greater as determined by a luciferase assay *in vitro*, wherein the predetermined period of time is sufficient for transcription, reverse transcription and insertion into the genome.

43. A method according to Item 42, wherein the promoter sequence is located in frame to a transcription initiation site of the retrotransposon at the transcription initiation site of the promoter sequence.

45. A method according to Item 42, wherein the nucleic acid construct comprises a foreign gene located in an operable manner in the retrotransposon, and the selection is achieved by the expression of the foreign gene.

46. A method according to Item 42, wherein the foreign gene is located in the reverse direction with respect to the transcription direction of the retrotransposon, and comprises a splice donor sequence and splice acceptor sequence, and an intron sequence located cis-direction sandwiched therebetween, wherein said predetermined period of time is sufficient for achieving transcription, reverse transcription and insertion into the genome, and wherein the selection is achieved by the expression of the foreign gene.

47. A method according to Item 46, wherein the foreign gene encodes an agent selected from the group consisting of antibiotic resistance gene, nutrient supplement agent, enzyme and fluorophore, and the selection is achieved by the property of the cell expressing the agent.

48. A method according to Item 42, wherein the LTR-type retrotransposon comprises an IAP element.

49. A method according to Item 42, wherein the LTR-type retrotransposon comprises a full-length IAP element.

50. A method according to Item 42, wherein the selection is achieved by confirming the transposed sequence by means of ligation mediated PCR.

51. A method according to Item 42, wherein the introduction comprises a format selected from the group consisting of transfection, transformation and transduction.

52. A method according to Item 42, wherein the introduction is achieved in the presence of at least one substance selected from the group consisting of cationic lipids and polyamine reagents.

53. A method according to Item 42, wherein the cells comprises a eukaryotic cell.

54. A method according to Item 42, wherein the cells comprises a mammalian cell.

55. A method according to Item 42, wherein the cells comprises a rodent or primate cell.

56. A method according to Item 42, wherein the retrotransposon is derived from a eukaryotic cell.

57. A method according to Item 42, wherein the retrotransposon is derived from a mammalian cell.

58. A method according to Item 42, wherein the retrotransposon is derived from a rodent or primate cell.

59. A method according to Item 42, wherein the cell is of the same species as that of the natural host of the retrotransposon.

60. A method according to Item 42, wherein the cell is of a different species as that of the natural host of the retrotransposon.

61. A method for assaying transposition activity of a retrotransposon, comprising the steps of:

A) providing a nucleic acid construct comprising a nucleic acid sequence encoding a retrotransposon to be assayed, and a promoter sequence having activity of at least 0.1 rlu as determined by a luciferase assay *in vitro*;
B) introducing the nucleic acid construct into the cell;
C) culturing the cell for a predetermined period of time; and
D) detecting the transposition by means of nucleic acid construct.

62. A method according to Item 61, wherein the detection comprises the step of ligation mediated PCR.

63. A method according to Item 61, wherein the detection comprises the step of comparing a genomic database and the sequence obtained by the ligation mediated PCR.

64. A method for producing the transgenic organism, comprising the steps of:

A) providing a nucleic acid construct comprising a nucleic acid sequence encoding a LTR-type retrotransposon;
B) introducing the nucleic acid construct into a germ-line cell of a desired biological organism;
C) selecting a germ-line cell with the genome thereof modified in the germ-line cell; and
D) regenerating the germ-line cell with the genome thereof modified into a biological organism.

65. A kit for modifying the genome of a cell, comprising:

A) a nucleic acid construct comprising a nucleic acid sequence encoding a LTR-type retrotransposon;

B) means for introducing the nucleic acid construct into a germ-line cell of a desired biological organism; and

C) means for selecting a germ-line cell with the genome thereof modified in the germ-line cell.

66. A kit according to Item 65, wherein the means for introducing the nucleic acid construct into the cell comprises transfection reagent.

67. A kit according to Item 66, wherein the transfection reagent is selected from the group consisting of cationic macromolecule, cationic lipid, polyamine reagent, polyimine reagent, and calcium phosphate.

68. A kit according to Item 66, wherein the transfection reagent is selected from the group consisting of cationic lipid and polyamine reagent.

69. A kit according to Item 65, wherein the means for selection comprises at least one of means for detection corresponding to one selected from the group consisting of a PCR primer, antibiotic resistance, complement of nutrition, enzymatic activity and fluorescence.

70. A kit for assaying transposition activity of a retrotransposon, comprising:

A) a nucleic acid construct comprising a nucleic acid sequence encoding a LTR-type retrotransposon, and a promoter having an activity of 0.1 rlu or greater as determined by a luciferase assay *in vitro;*
B) means for introducing the nucleic acid construct into the cell; and
C) means for detecting transposition by the nucleic acid construct.

71. A kit according to Item 54, wherein the means for detecting comprises at least one means selected from means for detection of at least one of the group consisting of PCR primer, antibiotic resistance, complement of nutrition, enzymatic activity and fluorescence.

72. A kit for producing a. transgenic organism, comprising:

A) a nucleic acid construct comprising a nucleic acid sequence encoding an LTR-type retrotransposon;
B) means for introducing the nucleic acid construct into a germ-line cell of a desired organism;
C) means for selecting a germ-line cell with the genome thereof modified in the germ-line cell; and
D) means for regenerating the germ-line with the genome thereof modified into an organism.

73. A kit according to Item 72, wherein the means for regenerating the organism comprises an organism as a host.

74. A promoter comprising a cytomegalovirus enhancer and avian beta-actin promoter, wherein at least one of the cytomegalovirus enhancer and the avian beta-actin promoter comprises a sequence shorter than the native full-length thereof.

75. A promoter according to Item 74, wherein the shorter sequence is due to the deletion of a sequence downstream of the transcription initiation site.

76. A promoter according to Item 74, wherein all the sequence is downstream of the transcription initiation site is deleted.

77. A promoter according to Item 74, wherein a portion of a sequence is downstream of the transcription initiation site and the promoter region.

78. A promoter according to Item 74, wherein the cytomegalovirus enhancer comprises a sequence set forth in SEQ ID NO: 36 and a variant thereof.

79. A promoter according to Item 74, wherein the avian beta-actin promoter comprises a sequence set forth in SEQ ID NO: 8 or a variant thereof.

80. A promoter according to Item 74, comprising the sequence set forth in SEQ ID NO: 6.

81. A promoter according to Item 74, comprising the sequence set forth in SEQ ID NO: 7.

82. Use of an LTR-type retrotransposon for genomic modification.

83. Use of a promoter having an activity of 0.1 rlu or greater as determined by a luciferase assay *in vitro*, for modification of a genome.

84. Use of a promoter having an activity of 0.1 rlu or greater as determined by a luciferase assay *in vitro*, for confirmation of an LTR-type retrotransposon.

Accordingly, these and other advantages of the present invention will be evidently understood by those skilled in the art in view of the drawings attached hereto and in view of the following detailed description of the present invention.

EFFECTS OF THE INVENTION

The present invention allows unexpectedly use of an LTR-type retrotransposon for a system for artificial promoting transposition. Accordingly, such a system is used in an application to allow modification of a genome of a cell, organism and the like, introduction of a gene, production of a transgenic mouse, and the like. Furthermore, it is believed that the utility of the present invention is extremely large and vast. The present invention could surprisingly provide a system in which transposition activity of an LTR-type retrotransposon can be readily detected. Accordingly, the present invention attains an effect in which such a system is used to confirm transposition activity of an LTR-type retrotransposon and to readily identify an LTR-type retrotransposon which can be used in the production of transgenic mice or the like.

BRIEF DESCRIPTION OF THE DRAWINGS

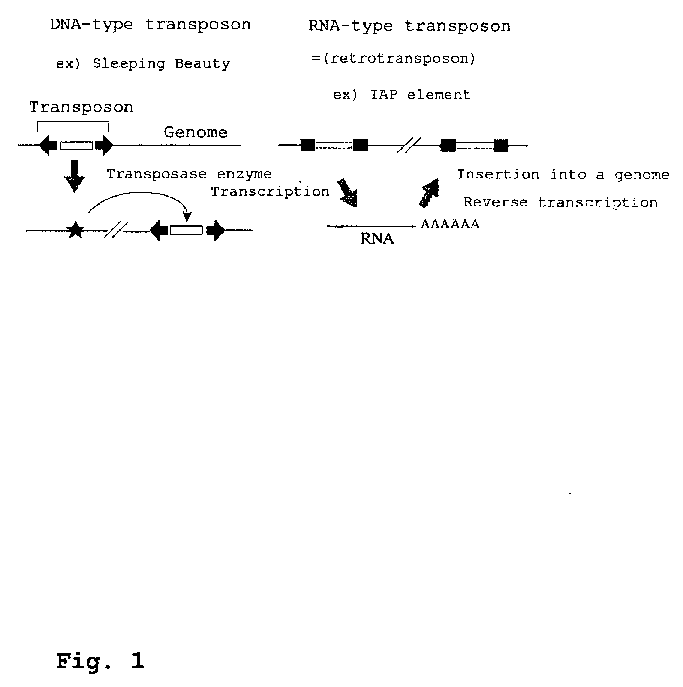

Figure **1** depicts a schematic comparison between a DNA-type transposon and RNA-type transposon (retrotransposon).

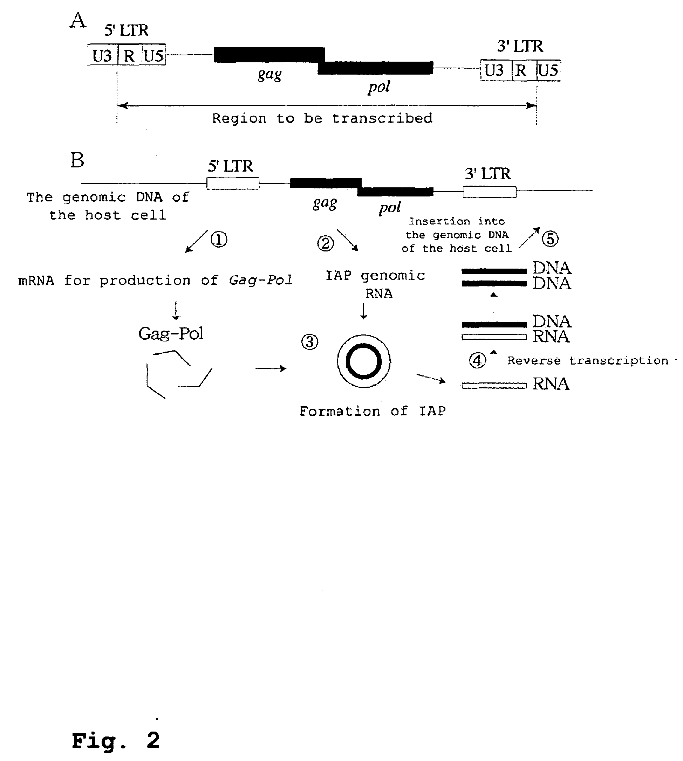

Figure **2A** depicts the structure of the IAP. Figure **2B** depicts the life cycle of an IAP.

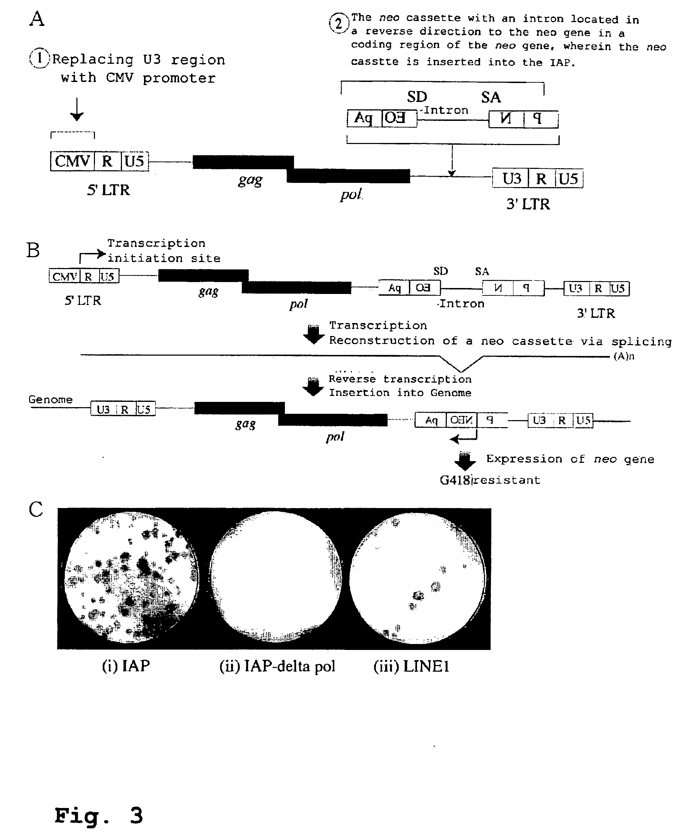

Figure **3A** depicts the construction of the vector of the invention. Figure **3B** depicts an assay method for IAP activity. Figure **3C** depicts an example of appearance frequency of G418 resistant colonies.

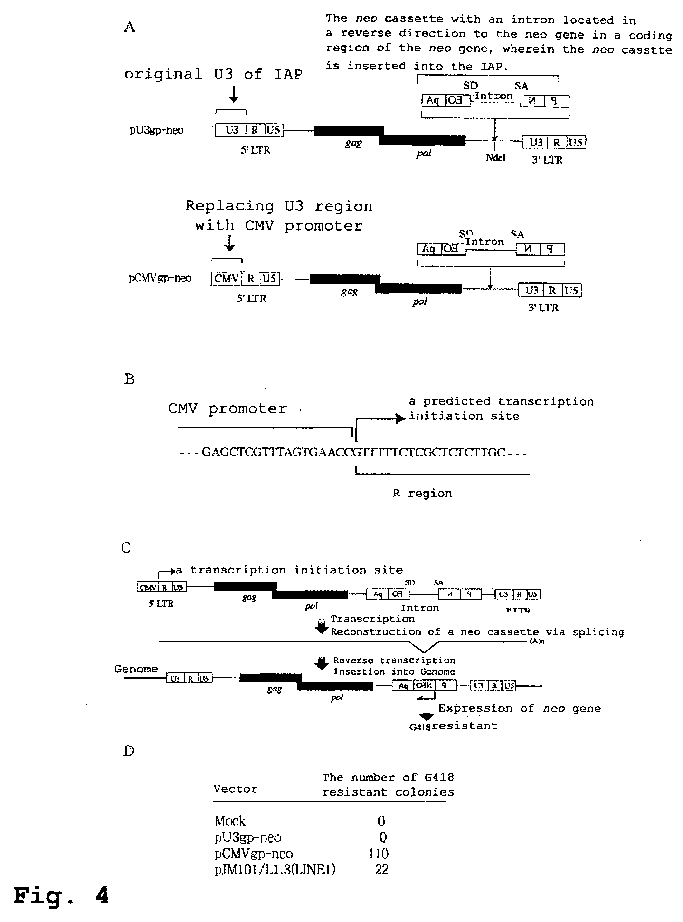

Figure **4** depicts schematic exemplification of efficient transposition by means of modification in the promoter region of the IAP. (A) the structure of a vector used in Example 1; (B) the sequence of a junctional portion between the CMV promoter and the R region; (C) principle of detection of transposition; and (D) detection of transposition by means of transfection into NIH3T3 cell.

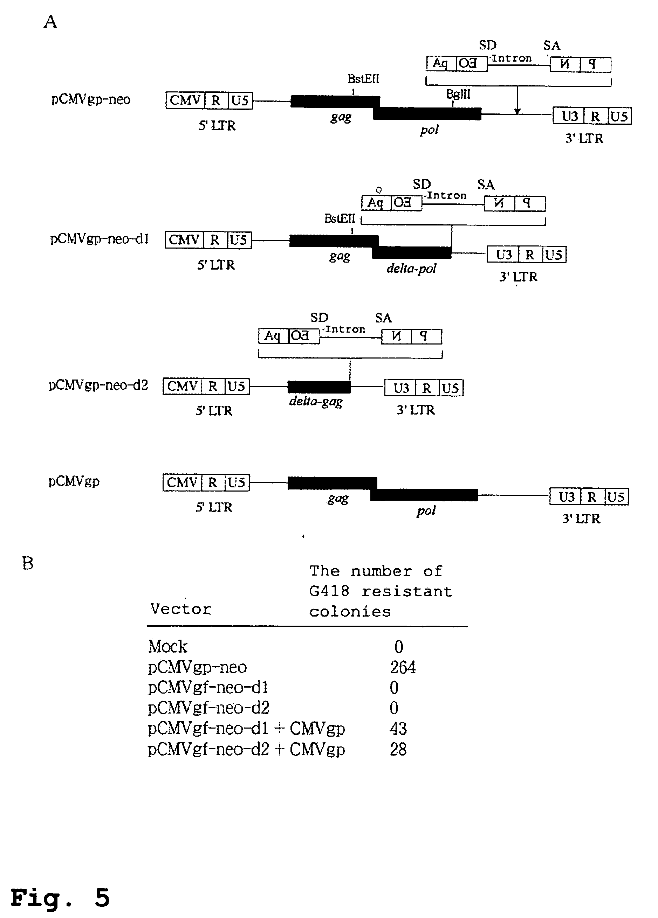

Figure **5** depicts confirmatory examples in which an IAP that can be used in the present invention has a complete transposition capability, and transposition can be controlled by separating *gag-pol* expression units. (A) shows the structures of a variety vectors used in Example 1. (B) shows an assay of activities of each vector by transfection into HeLa cells.

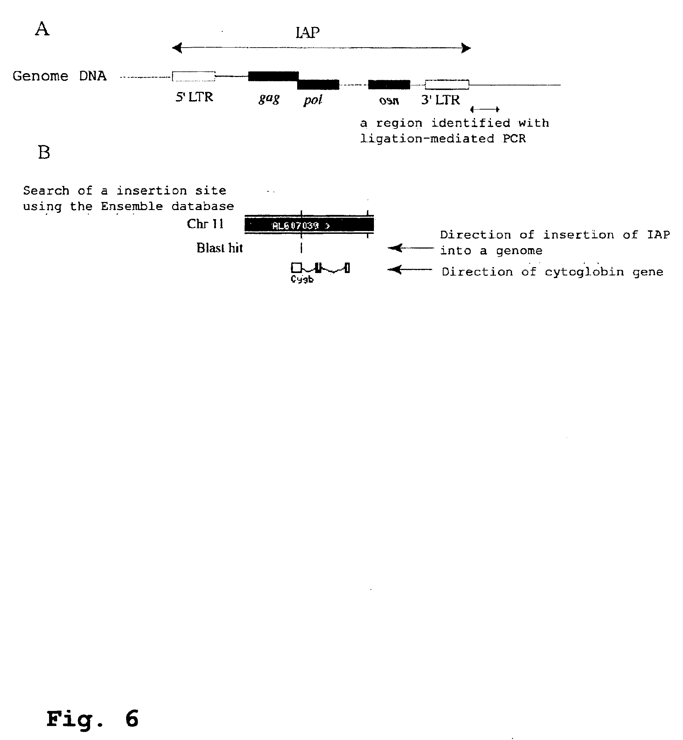

Figure **6** depicts examples in which an IAP vector is inserted into a gene. (A) shows site in which the 3' LTR downstream base sequence in the Figure has been determined by ligation-mediated PCR. (B) shows the results for which the sequence determined in (A) was searched using the Ensemble database

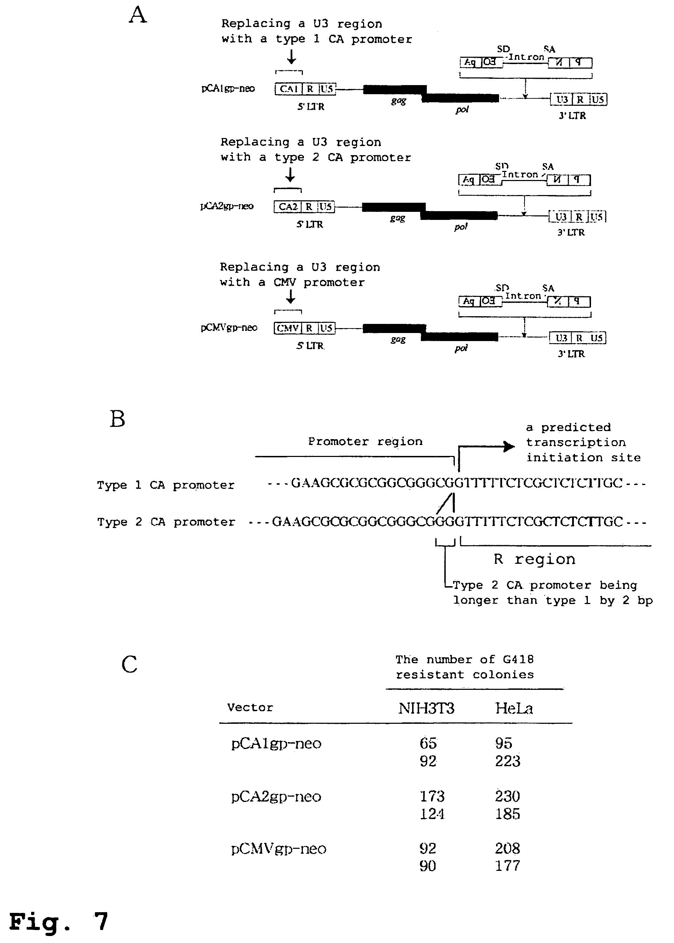

Figure **7** shows effects of the CA promoter. (A) shows the structures of two CA-containing vectors (pCA1gp-neo, pCA2gp-neo) and pCMVgp-neo. (B) shows the sequence of juncture sites of two CA promoters shown in (A) and the R region. (C) shows the comparison of CA1, CA2 and CMV promoters.

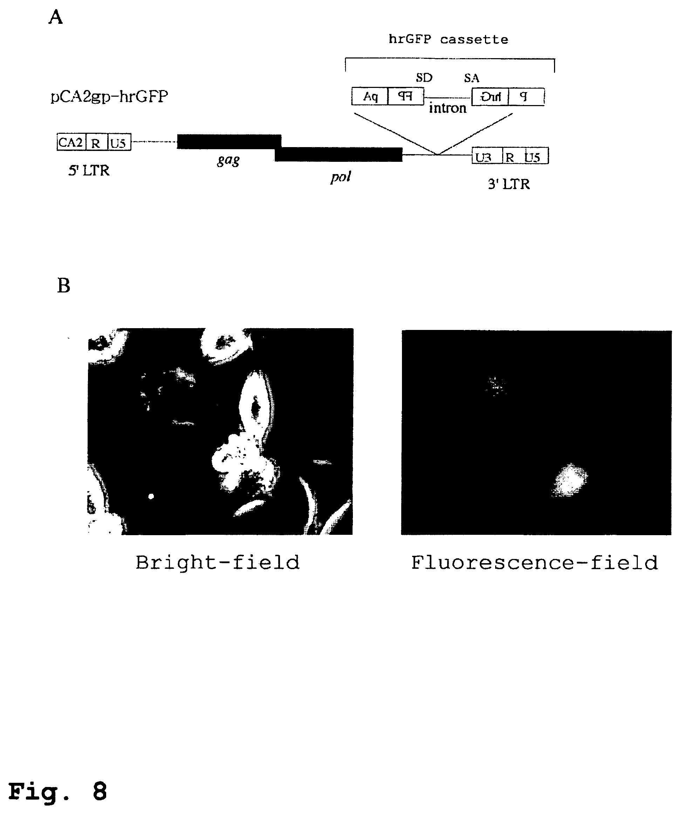

Figure **8** depicts a visualized example of transposition using GFP. (A) shows the structure of the vector used. (B) depicts the expression of GFP associated with the transposition.

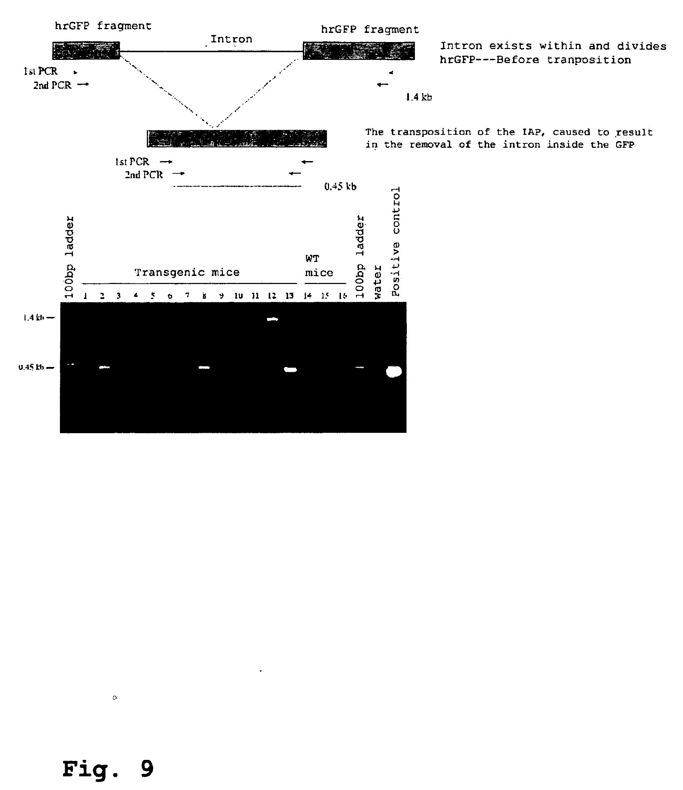

Figure **9** depicts the examples of recombination in a mouse individual. Transgenic mice having pCA2gp-hrGFP depicted in Figure **8** were produced and PCR conducted using DNA from tail of the mice as a template and the primers shown in the figure. The transposition of the IAP is caused to result in the removal of the intron inside the GFP. As such it is predicted that 0.45kb band will appear. As further shown in the figure, in three lines out of thirteen, 0.45kb band is detected and it was proved that the transposition has been caused in the body of the mice.

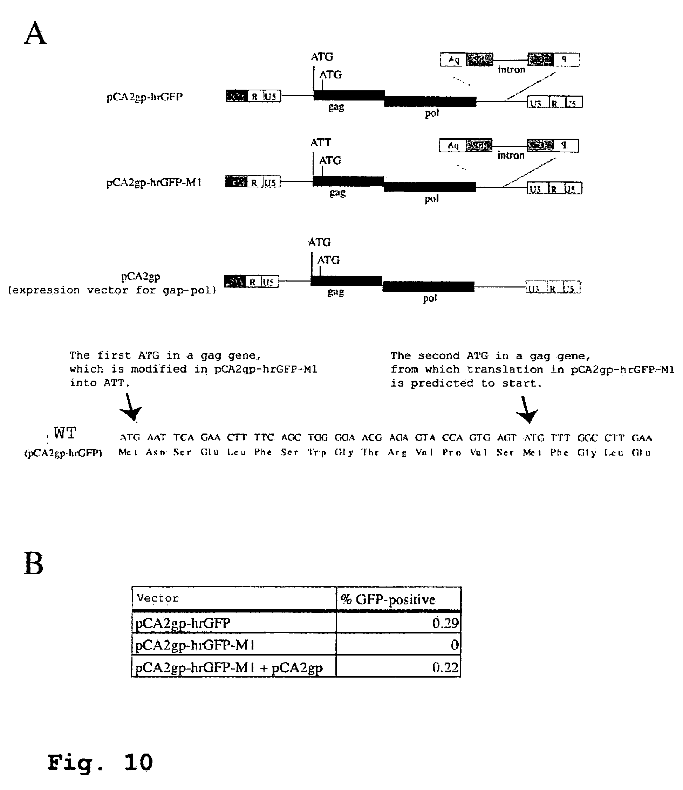

Figure **10** depicts verification that the first 15 amino acids of the GAG protein are preferable for the transposition. (A) depicts the structure of the vector. It is believed that in comparison with pCA2gp-hrGFP which showed autonomous transposition in Figure **8,** gpCA2hrGFP-M1 has introduced mutations in the initiation codon of the gag gene, and subsequently resulted in the initiation of the translation of the second ATG, fifteen amino acids downstream thereto. (B) Shows a study of transposition efficiency: Using the vector of (A), HeLa cells were transfected with the three combinations shown therein, and analyzed for the ratio of GFP-positive cells by FACS after seven days. As a result, pCA2hrGFP-M1, in which a mutation is introduced in the ATG at the original translation initiation site of the *gag* gene, has attenuated the transposition ability. However, a similar vector that has been transfected with pCA2gp, an expression vector of the *gag-pol* full length, has recovered its transposition ability. Hence, the fifteen amino acids from the translation initiation site of the GAG protein is preferable for its transposition ability.

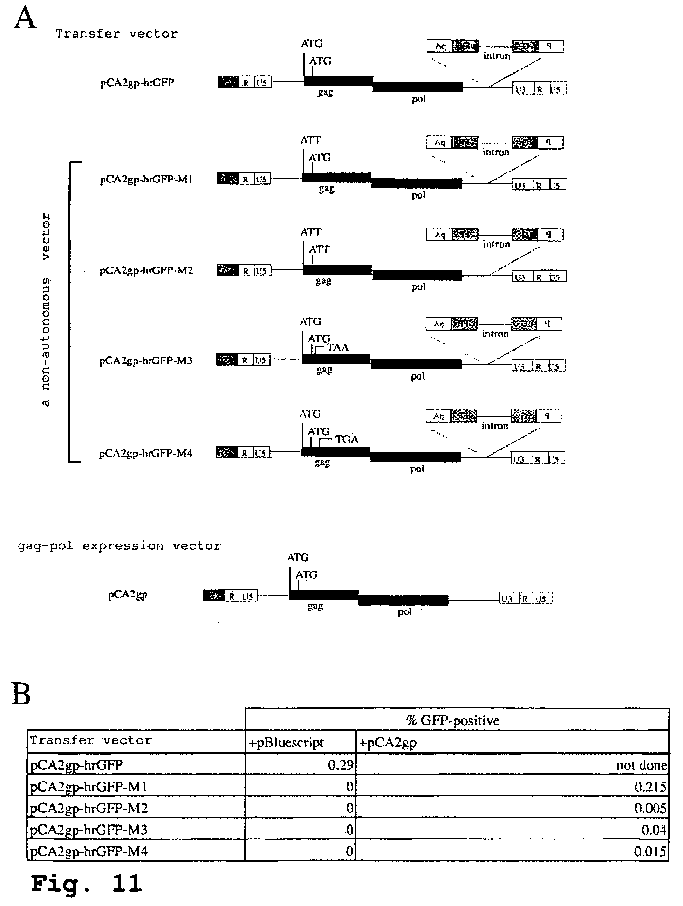

Figure **11** shows that it is preferable that the GAG protein is translated from per se in the transposition of a non-autonomous vector. (A) The structure of the vectors: The first three vectors and the *gag-pol* expression vector are the same as Figure **10.** In pCA2gp-hrGFP-M2 and pCA2gp-hrGFP-M3, a terminator codon has been introduced immediately downstream of the second ATG of the *gag* gene, and thus the GAG protein can only be expressed as a short fragment. Four vectors having a mutation in the GAG protein cannot cause transposition *per se*, and thus has been referred to as a non-autonomous vector. (B) The study of transposition efficiency: The transfer vector of (A) has been transfected into a HeLa cell in the presence of the *gag-pol* expression vector (pCA2gp), or in the absence thereof (using pBluescript, instead), and subsequently analyzed with FACS with the appearance frequency of the GFP positive cells after seven days. As a result, in three non-autonomous vectors, in which the translation of the GAG protein has been suppressed, transposition has been significantly reduced even in the presence of the *gag-pol* expression vector. On the other hand, it was observed that only pCA2gp-hrGFP-M1, in which the full length translation is caused after the first fifteen amino acids, transposition was at a higher rate in the presence of the *gag-pol* expression vector. Hence, it was shown that it was preferable that GAG _protein is translated from per se, for the transposition of a non-autonomous vector.

DESCRIPTION OF THE SEQUENCE LISTING

SEQ ID NO: 1: IAP sequence actually used in the Examples for exemplification.
SEQ ID NO: 2: IAP sequence amino acid sequence (gag #1)
SEQ ID NO: 3: IAP sequence amino acid sequence (gag #2)
SEQ ID NO: 4: IAP sequence amino acid sequence (pol)
SEQ ID NO: 5: CMV promoter sequence
SEQ ID NO: 6: CA1 promoter sequence (without the R region and with two bases deletion in the promoter region in addition thereto)
SEQ ID NO: 7: CA2 promoter sequence (without the R region)
SEQ ID NO: 8: avian beta-actin promoter sequence
SEQ ID NO: 9: forward primer sequence for isolation of the IAP element used in Example 1
SEQ ID NO: 10: reverse primer sequence for isolation of the IAP element used in Example 1
SEQ ID NO: 11: forward primer sequence for isolation of the full length of the IAP element used in Example 1
SEQ ID NO: 12: reverse primer sequence for isolation of the full length of the IAP element used in Example 1
SEQ ID NO: 13: forward primer sequence related to the CMV promoter used in Example 1 (c).
SEQ ID NO: 14: reverse primer sequence related to the CMV promoter used in Example 1 (c).
SEQ ID NO: 15: forward primer sequence related to the R region of the IAP used in Example 1 (c).
SEQ ID NO: 16: reverse primer sequence related to the R region of the IAP used in Example 1 (c).
SEQ ID NO: 17: a linking sequence of a linker DNA used in Example 3.
SEQ ID NO: 18: a linking sequence of a linker DNA used in Example 3.
SEQ ID NO: 19: a linker specific primer for use in the first round in Example 3 (forward).
SEQ ID NO: 20: a linker specific primer for use in the first round in Example 3 (reverse).
SEQ ID NO: 21: a linker specific primer for use in the second round in Example 3 (forward).
SEQ ID NO: 22: a linking sequence of neo cassette specific primer for use in the second round in Example 3 (reverse).
SEQ ID NO: 23: an alternative linking sequence of *neo* cassette specific primer for use in the second round in Example 3 (reverse).
SEQ ID NO: 24: a primer 5' upstream until the transcription initiation site of chicken beta-actin promoter used in Example 4.

SEQ ID NO: 25: a primer 3' of chicken beta-actin promoter used in Example 4.

SEQ ID NO: 26: an alternative primer 3' of chicken beta-actin promoter used in Example 4.

SEQ ID NO: 27: a primer of the 5' upstream from the 5' terminus of the R region of the IAP to the downstream of the U5 region used in Example 4.

SEQ ID NO: 28: a primer of the 3' side from the 5' terminus of the R region of the IAP to the downstream of the U5 region used in Example 4.

SEQ ID NO: 29: an alternative primer of the 3' side from the 5' terminus of the R region of the IAP to the downstream of the U5 region used in Example 4.

SEQ ID NO: 30: gamma globin intron sequence

SEQ ID NO: 31: a sequence of the tRNA binding site of the full length IAP

SEQ ID NO: 32: a repeat sequence of the R region of the full length IAP

SEQ ID NO: 33: a specific sequence for the full length IAP (tRNA binding site)

SEQ ID NO: 34: a tandem repeat sequence specific for the full length IAP

SEQ ID NO: 35: a repeat sequence of the R region found in the full length IAP

SEQ ID NO: 36: cytomegalovirus (CMV) enhancer sequence

SEQ ID NO: 37: a sequence in the sense direction of 1st primer used in Example 8 (AGGGCTGCGGCAAGGGCAACATCCTGTTCG).

SEQ ID NO: 38: a sequence in the antisense direction of 1st primer used in Example 8 (GCCGCCGTCCTCCACGTAGGTCTTCTCCAG).

SEQ ID NO: 39: a sequence in the sense direction of 2nd primer used in Example 8 (GGCAACCAGCTGGTGCAGATCCGCGTGACC).

SEQ ID NO: 40: a sequence in the antisense direction of 2nd primer used in Example 8 (GTCCTTCACCACGCCCTTGCTCTTCATCAG).

BEST MODE FOR CARRYING OUT THE INVENTION

Hereinafter the present invention is described.

It should be understood throughout the present specification, that expression of a singular form includes the concept of their plurality unless otherwise mentioned. Specifically, articles for a singular form (e.g., "a", "an", "the", etc. in English, and articles, adjectives, etc. in other languages) include the concept of their plurality unless otherwise mentioned. It should be also understood that the terms as used herein have definitions typically used in the art unless otherwise mentioned. Thus, unless otherwise defined, all scientific and technical terms have the same meanings as those generally used by those skilled in the art to which the present invention pertain. If there is contradiction, the present specification (including the definition) precedes.

(Definitions and Description of Terms)

Hereinafter, the definitions of terms specifically used herein are listed.

"Transposon" as used herein refers to a nucleic acid molecule or nucleic acid sequence which is capable of moving (transposition) from one site to another on a chromosome. Typically, a transposon is a DNA segment (DNA transposon). DNA transposons (hereinafter simply referred to as "transposons") are activated by a transposase enzyme and are subsequently transposed. Transposons include, but are not limited to, for example, SB transposon (Acc. No. L48685; SEQ ID NO: 1), and those included in the sequences set forth in SEQ ID NOs: 10-19, and the like.

As used herein, "DNA type" transposon refers to a transposon for use in transposing a DNA. Usual transposons are of DNA type. In an embodiment, the present invention may be practiced by using a DNA-type transposon.

As used herein, the term "retrotransposon" collectively refers to any DNA in which a DNA sequence in a certain site of the genome is once transcribed into an RNA and then reverse transcribed into complementary DNA (cDNA) by means of a reverse transcriptase to be re-inserted into another site of the genome. Such a phenomenon is also referred to as a retrotransposition. It is roughly classified into two groups: a group of retaining reverse transcriptase and a group of failing to retain reverse transcriptase.

Retrotransposons are classified into three groups according to a certain classification method. The first group is a type in which free DNA is incorporated into the genome caused by reverse transcription by a reverse transcriptase, after the sequence of transposition factor is transcribed. Ways of the transposition is similar to that to be integrated into the genome by means of a retrovirus such as HIV, and the type of this transposition factors are deeply related to a retrovirus. These groups have long terminal repeats (LTRs) at both termini of the sequence thereof, and thus are called an LTR-type retrotransposon. There are about 450,000 copies, or 8% of the human genome. It includes the Drosophila copia element, murine intracisternal A particle (IAP) element, and the like. Those having an LTR is sub classified into those encoding the env gene and those not encoding by the same (for example, Drosophila copia element, murine intracisternal A particle (IAP) element, and the like). Those having such transposition activity are called retrotransposon or an RNA-type transposon, and the related phenomenon of the transposition is called retrotransposition.

The second group is the same as the first group in that the second group itself encodes a reverse transcriptase. However, differs in that the second group will not result in a free DNA as reverse transcription product, but reverse transcription and the incorporation into the genome occur in parallel. This group is called LINE (Long INterspersed Element) for historical reasons. This group occupies 20 % of the human genome, corresponding to about 900,000 copies. It includes, but is not limited to, for example human L1 element, and the like.

The third group is greatly different from the first two groups in that the third group has no reverse transcriptase by itself. This group is also different from the two other retrotransposon in terms of transcription mechanism in addition to absence of translation product. Retrotransposons having reverse transcriptase is expected to cause transcription due to RNA polymerase II similar to a general mRNA, whereas the group (which is called Short INterspersed Element (SINE) as opposed to LINE) is similar to tRNA, and causes transcription by RNA polymerase III. This group is believed to occupy 13 % in the human genome, corresponding to about 1,500,000 copies, and thus to be present at the most in terms of copy number. Such a reverse transcriptase non-containing group includes, for example, pseudogene caused by reverse transcription from an mRNA, which was transcribed from a DNA sequence encoding a protein by means of an RNA polymerase, pseudogene of intranuclear low molecular RNA, interspersed short repeat sequence so called SINE, which is transcribed by an RNA polymerase, and the like. SINE includes a number of examples such as the Alu family present in the human genome, and those derived from tRNA and the like.

Accordingly, as used herein "RNA-type transposon" or "retrotransposon" are interchangeably used to refer to a retroposon having transposition activity. As used herein, it is intended that retrotransposon does not include retroviruses.

Figure **1** depicts a comparison between a DNA-type transposon and an RNA-type transposon (retrotransposon). In a DNA-type transposon, a transposon is inserted into another site after removal of a transposon from the genome, and thus the number of mutations allowable for introduction, cannot exceed the copy number of the transposons before transposition. Further, it has a property of being amenable of being transposable in the vicinity of the sites before transposition. On the other hand, in an RNA-type transposon, an RNA transposon transcribed has been inserted into the genome via reverse transcription, the number of mutations allowable for introduction and does not depend on the copy number of the retrotransposon before transposition, and it is possible that transposition occurs in the entire genome. As such, retrotransposons have the possibility of enhancing the exhaustiveness of mutations to be introduced.

As used herein the term "LTR-type" retrotransposon refers to a retrotransposon having a LTR (long terminal repeat) in the construct thereof. Such an LTR-type retrotransposon includes, but is not limited to, for example, IAP elements, early transposons (ETn), virus-like 30S RNA (VL30) element, and the like.

As used herein the term "LTR" refers to a sequence consisting of one hundred to one thousand base pairs having repeats at both sides of a provirus DNA such as a retrovirus, retrotransposon and the like. LTR consists of respective regions of transcription of a virus gene, U3, R and U5 relating to reverse transcription thereof, and the incorporation into the host DNA, respectively. IR sequence (inverted repeat region) present at 5' and 3' termini of the provirus are 4-20 base pairs in length. U3 comprises an enhancer sequence and a promoter sequence for transcription.

As used herein "non-LTR-type" retrotransposon refers to a retrotransposon having no LTR in the structure thereof. Non-LTR-type retrotransposon includes, but is not limited to, for example, L1 (LINE 1) and the like.

As used herein the term "intercisternal A particle" or "IAP" refers to a particle found as a particle classified as type A which has been discovered to be present in the cellular cistern by means of electron microscopy.

As used herein the term "IAP"-type retrotransposon, "IAP DNA element", "IAP RNA element", "IAP sequence", "IAP element" and "IAP nucleotide sequence", are interchangeably used to refer to a molecule having retrotransposon activity found in IAP. As used herein, unless otherwise stated, IAP is interchangeably used with IAP element. When specifically stated, it refers to an "IAP sequence" depending on the status with respect to the description of gene engineering and the devices thereof. Accordingly, an IAP retrotransposon is a type of an LTR-type retrotransposon found within the mouse genome. Our discovery of IAP-retrotransposons was from radiation induced bone marrow leukemia cells from C3H mouse, wherein the IAP-retrotransposons were in several hundreds to several thousands copies. Such examples of clones include: GeneBank Accession Numbers: AB099818; AB099819; AB099820; AB099821; AB099822; AB099823; AB099824; AB099825; AB099826; AB099827; AB099828; AH012499; Z36947; AB026817; D63766; D63767; AH007468; AF097546; AF097545; U79727; U79726; S80638; M58326; M59201 and the like, and it is understood that those skilled in the art will be able to obtain an appropriate clone based on the sequence information available from such known exemplary sequences and the like.

Figure **2** depicts an overview of IAP.

(A) The structure of an IAP element: There are two long terminal repeat (LTR) at both ends, and the *gag* and *pol* genes are located therebetween in a different reading frame. In contrast to a retrovirus, there is no functional env gene. The LTR consists of the U3, R and U5 regions similar to a usual retrovirus. The U3 region of the 5' LTR acts as a promoter, and the sequence in the R region of the 3' LTR functions as a poly A addition signal. Accordingly, transcription occurs in the region shown in the Figure from the 5' side upstream of the R region of the 5' LTR to the 3' terminal of the R region of the 3' LTR. The U3 of the 5' LTR and the U5 of the 3' LTR are not transcribed, but in the case of reverse transcription, these regions are copied from the other side of the LTR, and thus the full length IAP element is reconstructed after the insertion into the genome.

(B) The life cycle of IAP: After transcription of the region as described in (A), the portion thereof functions as (1) mRNA for production of *Gag-Pol,* and the remaining transcription product functions as (2) IAP genomic RNA. Combining *Gag-Pol* and the IAP genomic RNA, particulate IAP is produced (3). Particulate formation occurs on the membrane of endoplasmic reticulum (ER), and the construct of the produced IAP is released into the ER. The IAP construct is activated by an unknown mechanism, and transcribed from IAP RNA to IAP DNA (4) to insert into the genomic DNA of the host cell (5).

As used herein the term "full length" relating to a retrotransposon, refers to having a sequence corresponding to at least LTR (including the R region), *gag, pol* and tRNA binding sites. In particular, with respect to IAP element, there are thousands of clones and some of them have conventionally been reported to be "full length" by Mietz, J. A., et al., J. Virol. 61, 3020-3029, 1987. However, it was not known to date as to whether or not such a full length can be used in a transposition system.

As used herein, having "functionality" in relation to a retrotransposon, refers to having transposition activity.

As used herein, the term "consensus sequence" relating to a retrotransposon, refers to a minimal sequence necessary for having functionality. With respect to IAP element, consensus sequences include: amongst the sequence set forth in SEQ ID NO: 1, at least one domain selected from the group consisting of LTR (the LTR region at the 5' side: SEQ ID NO:1 positions 1-443; herein positions 1-225 correspond to the U3 region, positions 226-384 correspond to the R region, and positions 385-443 correspond to the U5 region. On the 3' side of the LTR region, positions 6876-7318 of SEQ ID NO: 1, herein positions 6876-7089 correspond to the U3 region, positions 7090 to 7259 correspond to the R region, and positions 7260-7318 correspond to the U5 region); *gag* (SEQ ID NO: 1, positions 670-2427 (*gag* #1), or positions 2430-3203 (gag #2), *pol* (positions 3440-5854 of SEQ ID NO: 1) and tRNA binding site (SEQ ID NO: 1: positions 444-463).

As used herein the term "transcription activity" refers to an activity of transcribing a DNA into an RNA (in particular, mRNA).

As used herein the term "reverse transcription activity" refers to an activity of transcribing an RNA to a DNA. Accordingly, it refers to an activity in a "reverse" direction in terms of the transcription activity.

As used herein the term "promoter activity" is a level of activating transcription. Promoter activity is expressed as rlu (relative unit) as expressed herein to observe an activity in a luciferase assay *in vitro.* As used herein, the activity of the CMV promoter is expressed as having an activity of 1 rlu as observed in the above-mentioned *in vitro* system.

As used herein the term "cytomegalovirus" or "CMV" is interchangeably used to refer to a multiparticular virus, belonging to Cucumovirus group. It consists of three types of viral particles, which are all globular polyhedron, having diameter about 29 nm. The genome thereof consists of three single stranded RNAs. The virus is a plant virus whose host range is extremely broad, and is distributed all over the world as a major pathogen and viral diseases of a number of crops such as cucumber, tomatoes and the like. The promoter of Cytonmegalovirus is a sequence having transcription promoting activity present in the RNA encoding the above-mentioned protein of the Cytomegalovirus. The CMV promoters include, but are not limited to, for example, the sequence set forth in SEQ ID NO: 5.

As used herein the term "CAG" promoter refers to a promoter comprising a Cytomegalovirus enhancer (preferably, Cytomegalovirus early immediate enhancer) and avian (chicken) beta-actin promoter related intron sequence. CAG promoter is described in, for example, Kosuga M.et al.,Cell Transplant.2000 Sep-Oct;9(5):675-680. A typical CAG promoter includes, but are not limited to, those comprising the sequences set forth in SEQ ID NO: 36 and 38.

As used herein the term "CA" promoter refers to a promoter in which an intron sequence and a portion of exon sequence is depleted from the CAP promoter, and is thus never conventionally present. The sequence which can be deleted from the CA promoter mainly include intron sequences, and are preferably in cases for regulating transcription initiation site. For example, CA promoters include, but are not limited to, e.g., those set forth in SEQ ID NO: 6 (CA1) and SEQ ID NO: 7 (CA2) and the like.

As used herein the term "cytomegalovirus enhancer" or "CMV enhancer" refers to an enhancer found in CMV, and typically includes, but is not limited to, one set forth in SEQ ID NO: 36 and the like, for example. This enhancer is reported to generally have very potent activity, and can be used in combination with a promoter. In particular, it is herein used as an element constituting the CAG.

As used herein the term "avian beta-actin promoter" or "chicken beta-actin promoter" are interchangeably used to refer to a promoter found in the beta-actin gene of an avian species (chicken), and typically includes one set forth in SEQ ID NO: 8. This promoter is believed to have potent activity in general, and can be combined with an enhancer. In particular, it is also used as an element constituting CAG.

As used herein, the term "in frame" refers to a way of location of nucleic acid sequences, and specifically refers to that the initiation site of translation or transcription or the translation frame is adapted. In the case of transcription location, transcription initiation site and a promoter sequence are directly linked.

As used herein, the term "reverse" refers to a way of location of nucleic acid sequences, and specifically refers to a nucleic acid sequence encoding a gene that is located in a reverse direction against the nucleic acid sequence encoding another gene in terms of translation or transcription. In the case that location is in reverse, when one nucleic acid sequence is transcribed under the effect of a promoter, the other nucleic acid sequence located in reverse will not be transcribed.

As used herein, the term "forward" refers to a way of location of nucleic acid sequences, and specifically refers to a nucleic acid sequence encoding a gene that is located in the same direction against the nucleic acid sequence encoding another gene in terms of translation or transcription.

As used herein the term "distinguishable property", as used in terms of a foreign gene, refers to a property of an expressed gene product in which the expression thereof can be confirmed by way of any means such as physical, chemical, biological, biochemical means or the like.

As used herein the term "intron sequence" refers to a sequence which lies inside a gene or the transcript thereof, but is not included in a final RNA product having a function, produced therefrom. Such an intron sequence can be readily identified by those skilled in the art by identifying the sequence which is present in a gene sequence in the genome but does not exist in mRNA or cDNA thereof. Typically, an intron sequence includes, but is not limited to, for example, intron of gamma-globin (SEQ ID NO: 30).

As used herein the term "splice donor" refers to a sequence rendering a sequence to be spliced to an acceptor, in a series of reactions of removing an intron portion in an RNA molecule with a nucleic acid sequence encoding a gene made by transcription, and linking the sequences of exons flanking thereto. There are some common sequences known as splice-donor sequence, such as, but are not limited to, for example, GARAGT (R refers to purine).

As used herein the term "splice acceptor" refers to a sequence of receiving a sequence to be spliced from a donor, in a series of reactions of removing an intron portion in an RNA molecule with a nucleic acid sequence encoding a gene made by transcription, and linking the sequences of exons flanking thereto. There are some common sequences known as splice acceptor sequence, such as, but are not limited to, for example, $(Y)_n$NCAG (n>11, N is any base).

Splice donor and splice acceptor are preferably selected as insertion sites when an intron sequence is inserted into a sequence encoding the foreign gene of the present invention.

As used herein the term "modification of the genome" refers to modification of a gene in a nucleic acid sequence of the genome, which in particular is functional.

As used herein the term "transposition" refers to transfer of a certain unit of sequence from a site on a nucleic acid sequence of the genome or the like to another site.

It is possible to verify as discussed below in detail, for example, to confirm whether or not the retrotransposon has the full transposition ability. A schematic drawing is shown in Figure **3**.

The exemplary assay is described using Figure **3A** and **3B**. (A) The structure of vectors. (1) The U3 region of the 5' LTR, an IAP promoter, is replaced with the CMV promoter in order to enhance the transcription of the IAP in a variety of cultured cells. (2) The *neo* cassette with an intron located in a reverse direction to the neo gene in a coding region of the *neo* gene, is inserted into the IAP. The direction of the intron after the insertion is the same as the IAP, but the *neo* gene is located in a reverse direction with respect to the IAP, which is shown as reversed letters corresponding to the *neo* cassette within the Figure. SD: splice donor; SA splice acceptor. (B) Assay method of IAP activity: When an IAP vector is transfected into a cell, the intron inserted into the *neo* gene by splicing after transcription, is resected to reconstitute the coding region of the *neo* gene, rendering the cell G418-resistant.

As used herein the term "introduction" refers to, as used in terms of a nucleic acid, that a nucleic acid molecule is transferred inside a cell.

Ligation-mediated PCR refers to a reaction of amplification of the genomic region surrounding a particular sequence by means of PCR. The genomic DNA is linked to a linker DNA after the resection of the genomic DNA by means of restriction enzymes and PCR is conducted using primers specific to the linker and a primer set inside the sequence of interest.

In the present invention, any retrotransposons may be used for transgenic organisms, whether endogenous or exogenous without limit, and preferably, an exogenous retrotransposons can be used.

Target sequences of retrotransposons include any sequences.

Retrotransposons are classified as autonomous type in which mainly an enzyme catalyzing self transcription, and constitutive protein of the particle of the retrotransposon are encoded therein, and non-autonomous type which lacks the same. What is preferred to be used in the present invention, is a autonomous type. Such a autonomous type can be confirmed to be autonomous type only by means of the system of the present invention. The present invention, for the first time, has provided a system for confirming whether or not it is of such a autonomous type. This can be explained by being able to provide a system for modifying the genome for the first time.

A variety of nucleic acid sequences (for example, marker gene, a sequence for regulating expression of a gene, and a desired gene, and the like) may be inserted in to the portion to be sandwiched between the retrotransposon sequences. Thus, a transposon construct in combination with a variety of elements as necessary in addition to the retrotransposon sequence can be constructed. In the present invention, cells to be targeted for introducing a necessary gene for a transposon construct or for inducing transposition, include cells that have the potential to allow differentiation of an individual of a biological organism (preferably non-human biological organism), including, for example, a stem cell or a fertilized cell.

The transgenic biological organism of the present invention includes founder (not only the first generation but also those lineages established based on the founder are of course encompassed by the present invention) having either or both a transposon construct and a transposase. Further, organs, tissues, eggs, sperms and fertilized eggs derived from the transgenic biological organism lineage of the present invention, established cell lines established from a lineage of the transgenic biological organism, cloned individuals produced from the transgenic biological organisms, are also encompassed by the present invention. Transposon constructs of the present invention may be constructed by combining a transposon sequence with a variety of other elements and are subsequently introduced into a stem cell or fertilized egg or the like.

DNA-type transposons can transfer from a first position to a second position on the DNA in the presence of a transposase enzyme (which is called mobility). Any mobile cut-and-paste type transposon has two basic components, such as, being derived from an active transposase and a DNA sequence recognized by a transposase and capable of transfer. Transfer of a DNA sequence allows transfer of an intervening nucleic acid between a DNA sequence recognized thereby.

On the other hand, a retrotransposon allows insertion of its sequence by replication to a site far from the origin by means of transcription, the reverse transcription and the insertion into the genome. Therefore, it can be classed as a copy-and-paste type transposition. Further, when conducting genome modification, not only modifications in the vicinity of the target, but also the effects are also attained such that insertion of modifications in an exhaustive manner or universal manner in the entire genome. Accordingly, the appearance of the genome modification tool using an efficient retrotransposon system leads to greater effects and utility in a variety of fields.

As used herein the term "foreign gene" refers to a gene which is intended to be introduced via gene transfer of the present invention or a nucleic acid molecule encoding the same. Such a foreign gene is derived from a host of different origin from the host which the introduction is intended or the same host. As long as introduction is intended, the nucleic acid sequence encoding the foreign gene may encode any protein. In one embodiment, the protein encoded by the nucleic acid sequence is a marker protein such as GFP, chloramphenicol acetyltransferase (CAT), β-galactosidase (lacZ), and luciferase (LUC). In another embodiment, the protein encoded by the nucleic acid is a growth hormone, for example, insulin-like growth factors (IGFs) to promote growth in a transgenic animal.

In one embodiment of a transgenic animal, the protein encoded by the nucleic acid fragment is a product of isolation from a cell. It should be noted that transgenic animals as bioreactors are known, for example , proteins can be produced in quantity in milk, urine, blood or eggs. Promoters are known that subsequently promote protein expression in milk, urine, blood or eggs and these include, but are not limited to, the casein promoter, the mouse urinary protein promoter, beta-globin promoter and the ovalbumin promoter, respectively. Recombinant proteins are produced by means of other methods for producing a protein in a cell. Nucleic acids encoding these or other proteins can be incorporated into the nucleic acid fragment of this invention and subsequently introduced into a cell. Efficient incorporation of the nucleic acid fragments into the DNA of a cell occurs when a composition of the present invention is present. Where the cell is part of a tissue or part of a transgenic animal, large amounts of recombinant protein can be obtained.

(Cells and Biology)

The term "cell" is herein used in its broadest sense in the art, referring to a structural unit of a tissue present in a multicellular organism, which is capable of self replicating, has genetic information and a mechanism for expressing it, and is surrounded by a membrane structure that isolates the living body from the outside. Cells used herein may be either naturally-occurring cells or artificially modified cells (e.g., fusion cells, genetically modified cells, etc), as long as the cell has a chemical receptor or is capable of having such a nucleic acid molecule introduced therein. Examples of cell sources include, but are not limited to, a single-cell culture; the embryo, blood, or a body tissue of a normally-grown transgenic animal, a mixture of cells derived from normally-grown cell lines, and the like. Preferably, a cell which is easily transformed or transfected is used. Cells used in the present invention are preferably cells which easily introduces a nucleic acid there into. For a purpose of reproduction, it is preferable to use reproductive cells. Alternatively, an ES cell may be used.

Cells used herein may be derived from any organism (e.g., any unicellular organisms (e.g., bacteria and yeast) or any multi-cellular organisms (e.g., animals (e.g., vertebrates and invertebrates), plants (e.g., monocotyledons and dicotyledons, etc.)). For example, cells used herein are derived from a vertebrate (e.g., Myxiniformes, Petronyzoniformes, Chondrichthyes, Osteichthyes, amphibian, reptilian, avian, mammalian, etc.), more preferably mammalian (e.g., monotremata, marsupialia, edentate, dermoptera, chiroptera, carnivore, insectivore, proboscidea, perissodactyla, artiodactyla, tubulidentata, pholidota, sirenia, cetacean, primates, rodentia, lagomorpha, etc.). In one embodiment, cells derived from Primates (e.g., chimpanzee, Japanese monkey, human) are used. Particularly, without limitation, cells derived from a human are used. The above-described cells may be either stem cells or somatic cells. Also, the cells may be adherent cells, suspended cells, tissue forming cells, and mixtures thereof.

Any organ may be targeted by the present invention. A tissue or cell targeted by the present invention may also be derived from any organ. As used herein, the term "organ" refers to a morphologically independent structure localized at a particular portion of an individual organism in which a certain function is performed. In multi-cellular organisms (e.g., animals, plants), an organ consists of several tissues spatially arranged in a particular manner, each tissue being composed of a number of different cells. An example of such an organ includes an organ relating to the vascular system. In one embodiment, organs targeted by the present invention include, but are not limited to, skin, blood vessel, cornea, kidney, heart, liver, umbilical cord, intestine, nerve, lung, placenta, pancreas, brain, peripheral limbs, retina, and the like. In plant, "organ" includes, but is not limited to: callus, root, caulome, stem, stalk, leaf, flower, seed, embryo, germ, fruit, albumen and the like.

As used herein, the term "tissue" refers to an aggregate of cells having substantially the same function and/or form in a multi-cellular organism. "Tissue" is typically an aggregate of cells of the same origin, but may be an aggregate of cells of different origins as long as the cells have the same function and/or form. Therefore, when stem cells of the present invention are used to regenerate a tissue, the tissue may be composed of an aggregate of cells of two or more different origins. Typically, a tissue constitutes a part of an organ. Animal tissues are separated into epithelial tissue, connective tissue, muscular tissue, nervous tissue, and the like, on a morphological, functional, or developmental basis. Plant tissues are roughly separated into meristematic tissue and permanent tissue according to the developmental stage of the cells constituting the tissue. Alternatively, tissues may be separated into single tissues and composite tissues according to the type of cells constituting the tissue. Thus, tissues are separated into various categories.

As used herein, the term "stem cell" refers to a cell capable of self replication and exibiting pluripotent behavior. Typically, stem cells can be used o regenerate an injured tissue. Stem cells used herein may be, but are not limited to, embryonic stem (ES) cells or tissue stem cells (also called tissular stem cell, tissue-specific stem cell, or somatic stem cells). Accordingly, a stem cell may be directly used in the present invention.

As used herein, the term "somatic cell" refers to any cell other than a germ cell, such as an egg, a sperm, or the like, which does not transfer its DNA to the next generation. Typically, somatic cells have limited or no pluripotency. Somatic cells used herein may be naturally-occurring or genetically modified.

As used herein, the term "isolated" means that naturally accompanying material is at least reduced, or preferably substantially completely eliminated, in normal circumstances. Therefore, the term "isolated cell" refers to a cell substantially free from other accompanying substances (e.g., other cells, proteins, nucleic acids, etc.) in natural circumstances. The term "isolated" in relation to nucleic acids or polypeptides means that, for example, the nucleic acids or the polypeptides are substantially free from cellular substances or culture media when they are produced by recombinant DNA techniques; or precursory chemical substances or other chemical substances when they are subsequently chemically synthesized. Isolated nucleic acids are preferably free from sequences naturally flanking the nucleic acid within an organism from which the nucleic acid is derived (i.e., sequences positioned at the 5' terminus and the 3' terminus of the nucleic acid).

As used herein, the term "established" in relation to cells refers to a state of a cell in which a particular property (pluripotency) of the cell is maintained and the cell undergoes stable proliferation under culture conditions. Therefore, established stem cells maintain pluripotency. In the present invention, such an established cell is preferably used since such a cell provides a stabilized result.

As used herein, the term "differentiated cell" refers to a cell having a specialized function and form (e.g., muscle cells, neurons, etc.). Unlike stem cells, differentiated cells have no or little pluripotency. Examples of differentiated cells include epidermal cells, pancreatic parenchymal cells, pancreatic duct cells, hepatic cells, blood cells, cardiac muscle cells, skeletal muscle cells, osteoblasts, skeletal myoblasts, neurons, vascular endothelial cells, pigment cells, smooth muscle cells, fat cells, bone cells, cartilage cells, and the like.

As used herein the term "a living body" refers to a form of a biological organism which may be present as a single individual capable of existing as a living organism.

(Biochemistry and Molecular Biology)

As used herein, the term "gene" refers to an element defining a genetic trait. A gene is typically arranged in a given sequence on a chromosome. A gene which defines the primary structure of a protein is called a structural gene. A gene which regulates the expression of a structural gene is called a regulatory gene (e.g., promoter). Genes herein include structural genes and regulatory genes unless otherwise specified. Therefore, the term "cyclin gene" typically includes the structural gene cyclin and its subsequent promoter. As used herein, "gene" may refer to a "polynucleotide", "oligonucleotide", "nucleic acid", and a "nucleic acid molecule" and/or "protein", "polypeptide", "oligopeptide" and "peptide". As used herein, "gene product" includes a "polynucleotide", "oligonucleotide", a "nucleic acid" and a "nucleic acid molecule" and/or "protein", "polypeptide", "oligopeptide" and a "peptide", which are subsequent expression products of a gene. Those skilled in the art understand what a gene product is, according to the context used with the present invention. Accordingly, gene used herein usually includes not only double-stranded DNA but also each single-stranded DNA, such as sense chain and antisense chain constituting thereof. Therefore, the genes of the present invention include any of double-stranded DNA including human genome DNA, and single-stranded DNA (sense chain) including cDNA, as well as a single stranded DNA (antisense) having a sequence complementary to the sense chain, as well as fragments thereof.

As used herein, the term "homology" in relation to a sequence (e.g., a nucleic acid sequence, an amino acid sequence, etc.) refers to the proportion of identity between two or more gene sequences. Therefore, the greater the homology between two given genes is, the greater is the identity or similarity between their sequences. Whether or not two genes have homology is determined by comparing their sequences directly or by a hybridization method under stringent conditions. When two gene sequences are directly compared with each other, these genes have homology if the DNA sequences of the genes have representatively at least 50% identity, preferably at least 70% identity, more preferably at least 80%, 90%, 95%, 96%, 97%, 98%, or 99% identity with each other. As used herein, the term "similarity" in relation to a sequence (e.g., a nucleic acid sequence, an amino acid sequence, or the like) refers to the proportion of identity between two or more sequences when conservative substitution is regarded as positive (identical) in the above-described homology. Therefore, homology and similarity differ from each other in the presence of conservative substitutions. If no conservative substitutions are present, homology and similarity have the same value.

The similarity, identity and homology of amino acid sequences and base sequences are herein compared using FASTA with the default parameters. Alternatively, an identity search may be conducted, for example, using NCBI's BLAST 2.2.9 (published May 12, 2004). As used herein, the value of identity usually refers to the value as a result of alignment with the BLAST as described above using the default parameters. If the change of parameters results in higher values, then the highest value is employed herein as the value of the identity. When a plurality of regions are evaluated for identity, the highest value is employed herein as the value of the identity.

The terms "protein", "polypeptide", "oligopeptide" and "peptide" as used herein have the same meaning and refer to an amino acid polymer having any length. This polymer may be a straight, branched or cyclic chain. An amino acid may be a naturally-occurring or non-naturally-occurring amino acid, or a variant amino acid. The term may include those assembled into a composite of a plurality of polypeptide chains. The term also includes a naturally-occurring or artificially modified amino acid polymer. Such modification includes, for example, disulfide bond formation, glycosylation, lipidation (acylation), acetylation, phosphorylation, or any other manipulation or modification (e.g., conjugation with a labeling moiety). This definition encompasses a polypeptide containing at least one amino acid analog (e.g., non-naturally-occurring amino acid, etc.), a peptide-like compound (e.g., peptoid), and other variants known in the art. Gene products, such as extracellular matrix proteins (e.g., fibronectin, etc.), are usually in the form of a polypeptide. However, there may be a form of a polypeptide variant as long as it has the same function. Polypeptides having specific amino acid sequences include fragments, cognates, derivatives and variants thereof.

The terms "polynucleotide", "oligonucleotide", "nucleic acid molecule" and "nucleic acid" as used herein have the same meaning and refer to a nucleotide polymer having any length. This term also includes an "oligonucleotide derivative" or a "polynucleotide derivative". An "oligonucleotide derivative" or a "polynucleotide derivative" includes a nucleotide derivative, or refers to an oligonucleotide or a polynucleotide having linkages between nucleotides different from typical linkages, which are interchangeably used. Examples of such an oligonucleotide specifically include 2'-O-methyl-ribonucleotide, an oligonucleotide derivative in which a phosphodiester bond in an oligonucleotide is converted to a phosphorothioate bond, an oligonucleotide derivative in which a phosphodiester bond in an oligonucleotide is converted to a N3'-P5' phosphoroamidate bond, an oligonucleotide derivative in which a ribose and a phosphodiester bond in an oligonucleotide are converted to a peptide-nucleic acid bond, an oligonucleotide derivative in which uracil in an oligonucleotide is substituted with C-5 propynyl uracil, an oligonucleotide derivative in which uracil in an oligonucleotide is substituted with C-5 thiazole uracil, an oligonucleotide derivative in which cytosine in an oligonucleotide is substituted with C-5 propynyl cytosine, an oligonucleotide derivative in which cytosine in an oligonucleotide is substituted with phenoxazine-modified cytosine, an oligonucleotide derivative in which ribose in DNA is substituted with 2'-O-propyl ribose, and an oligonucleotide derivative in which ribose in an oligonucleotide is substituted with 2'-methoxyethoxy ribose. Unless otherwise indicated, a particular nucleic acid sequence also implicitly encompasses conservatively-modified variants thereof (e.g. degenerate codon substitutions) and complementary sequences, as well as the sequence explicitly indicated. Specifically, degenerate codon substitutions may be produced by generating sequences in which the third position of one or more selected (or all) codons is substituted with mixed-base and/or deoxyinosine residues (Batzer et al., Nucleic Acid Res. 19:5081(1991); Ohtsuka et al., J. Biol. Chem. 260:2605-2608 (1985); Rossolini et al.,

Mol. Cell. Probes 8:91-98(1994)).

As used herein the term "nucleotide" refers to a nucleoside in which the sugar moiety is a phosphate ester, and includes DNA, RNA and the like, and may be naturally occurring or non-naturally occurring. Nucleoside refers to a compound in which a base and a sugar are bound via N-glycoside bonding. "Nucleotide derivative" or "nucleotide analog" are interchangeably used herein to refer to a derivative or an analog which is different from a naturally occurring nucleotide but has a similar function as that of such a nucleotide. Such a nucleotide derivative and nucleotide analog is already well known in the art. Examples of such a nucleotide derivative and nucleotide analog include, for example, but are not limited to phosphorothioate, phosphoramidate, methyl phosphonate, chiral methyl phosphonate, 2-O-methyl ribonucleotide, peptide-nucleic acid (PNA). DNA includes cDNA, genomic DNA, and synthetic DNA.

In one embodiment, the variant refers to a naturally occurring allelic variant, non-naturally occurring variant, a variant having a deletion, substitution, or addition, and a polynucleotide sequence which does not substantially alter the function of the encoded polypeptide.

In another embodiment, variations such as a mutation of such amino acid sequences may occur in nature such as natural mutations, post-translational modifications and the like, but also may be artificially made using a naturally occurring gene such as specific genes of the present invention.

In another embodiment, the polypeptide comprises the allelic variants, homolog's, natural variants, having at least 70%, preferably at least 80%, more preferably at least 95 %, still more preferably at least 97 % homology with the naturally occurring polypeptide.

As used herein, the term "corresponding" amino acid or nucleic acid refers to an amino acid or nucleotide in a given polypeptide or polynucleotide molecule, which has, or is anticipated to have, a function similar to that of a predetermined amino acid or nucleotide in a polypeptide or polynucleotide as a reference for comparison. Particularly, in the case of enzyme molecules, the term refers to an amino acid which is present at a similar position in an active site and similarly contributes to it's catalytic activity. For example, in the case of the transposon sequence for a certain polynucleotide, the term refers to a similar portion in an ortholog corresponding to a particular portion of the transposon sequence.

As used herein, the term "corresponding" gene (e.g., a polypeptide or polynucleotide molecule) refers to a gene in a given species, which has, or is anticipated to have, a function similar to that of a predetermined gene in a species as a reference for comparison. When there are a plurality of genes having such a function, the term refers to a gene having the same evolutionary origin. Therefore, a gene corresponding to a given gene may be an ortholog of the given gene. Therefore, genes corresponding to those such as murine transposon and murine transposase can be found in other animals. Such a corresponding gene can be identified by techniques already well known in the art. Therefore, for example, a corresponding gene in a given animal can be found by searching a sequence database of the animal (e.g., human, rat, dog, cat) using the sequences such as murine transposon and murine transposase of a reference gene as a query sequence. Such corresponding genes can be readily obtained by those skilled in the art using genome databases. Methods for obtaining such genome sequences are well known in the art and described herein elsewhere. In the present invention, sequences obtained by such search can also be used.

As used herein, the term "fragment" with respect to a polypeptide or polynucleotide refers to a polypeptide or polynucleotide having a sequence length ranging from 1 to n-1 with respect to the full length of the reference polypeptide or polynucleotide (of length n). The length of the fragment can be appropriately changed depending on the purpose. For example, in the case of polypeptides, the lower limit of the length of the fragment includes 3, 4, 5, 6, 7, 8, 9, 10, 15, 20, 25, 30, 40, 50 or more nucleotides. Lengths represented by integers which are not herein specified (e.g., 11 and the like) may be appropriate as a lower limit. For example, in the case of polynucleotides, the lower limit of the length of the fragment includes 5, 6, 7, 8, 9, 10, 15, 20, 25, 30, 40, 50, 75, 100 or more nucleotides. Lengths represented by integers which are not herein specified (e.g., 11 and the like) may be appropriate as a lower limit. As used herein, the length of polypeptides or polynucleotides can be represented by the number of amino acids or nucleic acids, respectively. However, the above-described numbers are not absolute. The above-described numbers, as the upper or lower limits, are intended to include some greater or smaller numbers (e.g., $\pm$10%), as long as the same function is maintained. For this purpose, "about" may be herein put before the numbers. However, it should be understood that the interpretation of numbers is not affected by the presence or absence of "about" in the present specification. In the present invention, it should be understood that any fragment can be used as long as the fragment functions as possessing transposition activity (integrase = insertion activity into a genome, transcription activity and reverse transcription activity), murine transposase and the like, i.e., has transposition activity. As used herein the term integrase refers to an enzyme of catalyzing integration response of a genomic DNA into a DNA of a chromosome of a host, typically including IN protein of a retrovirus of INT protein ($\phi$80, P1, P2, P4, P22, 186 or the like) of a lambda phage. Such an activity can be determined by observing promotion of integration of a prophage or resection thereof from a chromosome of a bacteria.

As used herein, the term "biological molecule" refers to a molecule relating to an organism and an aggregation thereof.

As used herein, the term "biological" or "organism" refers to a biological organism, including, but not limited to, an animal, a plant, a fungus, a virus, and the like. A biological molecule includes a molecule extracted from an organism and an aggregation thereof, howvever the present invention is not limited to this. Any molecule capable of affecting an organism and an aggregation thereof, subsequently falls within the definition of a biological molecule. Therefore, low molecular weight molecules (e.g., low molecular weight molecule ligands, etc.) capable of being used as medicaments fall within the definition of biological molecule as long as an effect on an organism is intended. Examples of such a biological molecule include, but are not limited to, a protein, a polypeptide, an oligopeptide, a peptide, a polynucleotide, an oligonucleotide, a nucleotide, a nucleic acid (e.g., DNA such as cDNA and genomic DNA; RNA such as mRNA), a polysaccharide, an oligosaccharide, a lipid, a low molecular weight molecule (e.g., a hormone, a ligand, an information transmitting substance, a low molecular weight organic molecule, etc.), and a composite molecule thereof (glycolipids, glycoproteins, lipoproteins, etc.), and the like. A biological molecule may include a cell itself or a portion of tissue as long as it is intended to be introduced into a cell. Preferably, a biological molecule may include a nucleic acid (DNA or RNA) or a protein. In another preferred embodiment, a biological molecule is a nucleic acid (e.g., genomic DNA or cDNA, or DNA synthesized by PCR or the like). In another preferred embodiment, a biological molecule may be a protein. Preferably, such a biological molecule may be a hormone or cytokine.

As used herein "chemical synthesized substance" refers to any substance which may be synthesized using ordinary chemical technology. Accordingly, any chemical synthesized substance are within chemical substances. Substantially, all chemical substances may be synthesized. Such synthetic technology is well known in the art, and those skilled in the art can produce chemical synthesized substances appropriately combining such technologies.

As used herein, the term "biological activity" refers to activity possessed by an agent (e.g., a polynucleotide, a protein, etc.) within an organism, including activities exhibiting various functions (e.g., transposition activity, etc.). For example, when an agent is an antisense molecule, the biological activity thereof includes binding to a targeted nucleic acid molecule, suppression of expression thereby and the like. For example, when an agent is an enzyme, the biological activity thereof includes the enzymatic activity thereof. As for another example, when an agent is a ligand or a receptor, binding to the receptor or the ligand corresponding to the ligand or receptor, respectively, is included in the biological activity thereof. When the biological activity is transcriptional regulation activity, the activity refers to an activity for regulating transcriptional level or the variation thereof. For example, when the agent of interest is a retrotransposon, the activity thereof refers to an activity for transcription activity, reverse transcription activity, transposition activity and integrase activity and the like. Exemplifications, in which transposition activity is determined, include, for example, technologies described in the Examples Such biological activities may be determined by a well known technology in the art. An exemplification of such an assay for determining the activity includes, for example, that a cell obtains resistance against G418 by reconstructing the neo gene, which has been fragmented by means of introns, in the course of transposition reaction.

As used herein, "polynucleotides hybridizing under stringent conditions" refers to conditions commonly used and well known in the art. Such a polynucleotide can be obtained by conducting colony hybridization, plaque hybridization, Southern blot hybridization, or the like using a polynucleotide selected from the polynucleotides of the present invention. Specifically, a filter on which DNA derived from a colony or plaque is immobilized, is used to conduct hybridization at 65°C in the presence of 0.7 to 1.0 M NaCl. Thereafter, a 0.1 to 2-fold concentration of SSC (saline-sodium citrate) solution (1-fold concentration SSC solution composed of 150 mM sodium chloride and 15 mM sodium citrate) is used to wash the filter at 65°C. Polynucleotides identified by this method are referred to as "polynucleotides hybridizing under stringent conditions". Hybridization can be conducted in accordance with a method described in, for example, Molecular Cloning 2nd ed., Current Protocols in Molecular Biology, Supplement 1-38, DNA Cloning 1: Core Techniques, A Practical Approach, Second Edition, Oxford University Press (1995), and the like. Here, sequences hybridizing under stringent conditions exclude, preferably, sequences containing only A (adenine) or T (thymine).

As used herein, "hybridizable polynucleotide" refers to a polynucleotide which can hybridize to other polynucleotides under the above-described hybridization conditions. Specifically, the hybridizable polynucleotide includes at least a polynucleotide having a homology of at least 60% to the base sequence of DNA encoding a polypeptide having an amino acid sequence as specifically set forth herein, preferably a polynucleotide having a homology of at least 80%, and more preferably a polynucleotide having a homology of at least 95%.

As used herein, the term "probe" refers to a substance for use in searching-, which is used in a biological experiment, such as *in vitro* and/or *in vivo* -screening or the like, including, but not being limited to, for example, a nucleic acid molecule having a specific base sequence or a peptide containing a specific amino acid sequence.

Examples of a nucleic acid molecule as a common probe include one having a nucleic acid sequence having a length of at least 8 contiguous nucleotides, which is homologous or complementary to the nucleic acid sequence of a gene of interest. Such a nucleic acid sequence may be preferably a nucleic acid sequence having a length of at least 9 contiguous nucleotides, more preferably a length of at least 10 contiguous nucleotides, and even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 20 contiguous nucleotides, a length of at least 25 contiguous nucleotides, a length of at least 30 contiguous nucleotides, a length of at least 40 contiguous nucleotides, or a length of at least 50 contiguous nucleotides.

A nucleic acid sequence used as a probe includes a nucleic acid sequence having at least 70% homology to the above-described sequence, more preferably at least 80%, and even more preferably at least 90% or at least 95%.

As used herein, the term "search" indicates that a given nucleic acid sequence is utilized to find other nucleic acid base sequences having a specific function and/or property either electronically or biologically, or using other methods. Examples of an electronic search include, but are not limited to, BLAST (Altschul et al., J. Mol. Biol. 215:403-410 (1990)), FASTA (Pearson & Lipman, Proc. Natl. Acad. Sci., USA 85:2444-2448 (1988)), Smith and Waterman method (Smith and Waterman, J. Mol. Biol. 147:195-197 (1981)), and Needleman and Wunsch method (Needleman and Wunsch, J. Mol. Biol. 48:443-453 (1970)), and the like. Examples of a biological search include, but are not limited to, a macroarray in which genomic DNA is attached to a nylon membrane or the like or a microarray (microassay) in which genomic DNA is attached to a glass plate under stringent hybridization, PCR and in situ hybridization, and the like. In the present invention, retrotransposon identified by such search (e.g., IAP) may also be used.

The term "highly stringent conditions" refers to those conditions that are designed to permit hybridization of DNA strands whose sequences are highly complementary, and also to exclude hybridization of significantly mismatched DNAs. Hybridization stringency is principally determined by temperature, ionic strength, and the concentration of denaturizing agents such as formamide. Examples of "highly stringent conditions" for hybridization and washing are 0.0015 M sodium chloride, 0.0015 M sodium citrate at 65-68°C or 0.015 M sodium chloride, 0.0015 M sodium citrate, and 50% formamide at 42°C. See Sambrook, Fritsch & Maniatis, Molecular Cloning: A Laboratory Manual (2nd ed., Cold Spring Harbor Laboratory, N.Y., 1989); Anderson et al., Nucleic Acid Hybridization: A Practical Approach Ch. 4 (IRL Press Limited) (Oxford Express). More stringent conditions (such as higher temperature, lower ionic strength, higher formamide, or other denaturing agents) may be optionally used. Other agents may be included in the hybridization and washing buffers for the purpose of reducing non-specific and/or background hybridization. Examples are 0.1% bovine serum albumin, 0.1% polyvinylpyrrolidone, 0.1% sodium pyrophosphate, 0.1% sodium dodecylsulfate (NaDodSO$_4$ or SDS), Ficoll, Denhardt's solution, sonicated salmon sperm DNA (or other non-complementary DNA), and dextran sulfate, although other suitable agents can also be used. The concentration and types of these additives can be changed without substantially affecting the stringency of the hybridization conditions. Hybridization experiments are ordinarily carried out at pH 6.8-7.4; however, at typical ionic strength conditions, the rate of hybridization is nearly independent of pH. See Anderson et al., Nucleic Acid Hybridization: A Practical Approach Ch. 4 (IRL Press Limited, Oxford UK).

Agents affecting the stability of DNA duplex include base composition, length, and degree of base pair mismatch. Hybridization conditions can be adjusted by those skilled in the art in order to accommodate these variables and allow DNAs of different sequence relatedness to form hybrids. The melting temperature of a perfectly matched DNA duplexes can be subsequently estimated by the following equation:

$$Tm \ (°C) = 81.5 + 16.6 \ (log[Na^+]) + 0.41 \ (\% \ G+C) - 600/N - 0.72 \ (\% \ formamide)$$

where N is the length of the duplex formed, [Na$^+$] is the molar concentration of the sodium ion within the hybridization or washing solution, % G+C is the percentage of (guanine+cytosine) bases in the hybrid. For imperfectly matched hybrids, the melting temperature is reduced by approximately 1°C for each 1% mismatch.

The term "moderately stringent conditions" refers to conditions under which a DNA duplexes with a greater degree of base pair mismatching than could occur under "highly stringent conditions" is able to form. Typical Examples of "moderately stringent conditions" are 0.015 M sodium chloride, 0.0015 M sodium citrate at 50-65°C or 0.015 M sodium chloride, 0.0015 M sodium citrate, and 20% formamide at 37-50°C. By way of example, "moderately stringent conditions" of 50°C in 0.015 M sodium ion will allow about a 21% mismatch.

It will be appreciated by those skilled in the art that there is no absolute distinction between "highly stringent conditions" and "moderately stringent conditions". For example, at 0.015 M sodium ion (no formamide), the melting temperature of perfectly matched long DNA is about 71°C. With a wash at 65°C (at the same ionic strength), this would allow for approximately a 6% mismatch. To capture more distantly related sequences, those skilled in the art can simply lower the temperature or raise the ionic strength.

A good estimate of the melting temperature in 1 M NaCl for oligonucleotide probes up to about 20 nucleotides is given by the equation:

$$Tm = (2°C \ per \ A-T \ base \ pair) + (4°C \ per \ G-C \ base \ pair).$$

Note that the sodium ion concentration in 6X salt sodium citrate (SSC) is 1 M. See Suggs et al., Developmental Biology Using Purified Genes 683 (Brown and Fox, eds., 1981).

A naturally-occurring nucleic acid encoding a protein such as a retrotransposon (e.g., IAP element, in particular full length IAP element), a variant or fragment thereof, and a promoter sequence of the present invention may be readily isolated from a cDNA library having PCR primers and hybridization probes containing part of a nucleic acid sequence indicated by, for example, SEQ ID NO. 1, 6, 7 or the like. A preferable nucleic acid encoding a retrotransposase, or variants or fragments thereof, or the like is hybridizable to the whole or part of a sequence as set forth in SEQ ID NO: 1 or the like under low stringency conditions defined by hybridization buffer essentially containing 1% bovine serum albumin (BSA); 500 mM sodium phosphate ($NaPO_4$); 1mM EDTA; and 7% SDS at 42°C, and wash buffer essentially containing 2xSSC (600 mM NaCl; 60 mM sodium citrate); and 0.1% SDS at 50°C, more preferably under low stringency conditions defined by hybridization buffer essentially containing 1% bovine serum albumin (BSA); 500 mM sodium phosphate ($NaPO_4$); 15% formamide; 1 mM EDTA; and 7% SDS at 50°C, and wash buffer essentially containing $1 \times$ SSC (300 mM NaCl; 30 mM sodium citrate); and 1% SDS at 50°C, and most preferably under low stringency conditions defined by hybridization buffer essentially containing 1% bovine serum albumin (BSA); 200 mM sodium phosphate ($NaPO_4$); 15% formamide; 1 mM EDTA; and 7% SDS at 50°C, and wash buffer essentially containing $0.5 \times$ SSC (150 mM NaCl; 15 mM sodium citrate); and 0.1% SDS at 65°C.

As used herein, the term "probe" refers to a substance for use in searching, which is used in a biological experiment, such as *in vitro* and/or *in vivo* screening or the like, including, but not being limited to, for example, a nucleic acid molecule having a specific base sequence or a peptide containing a specific amino acid sequence.

Examples of a nucleic acid molecule as a common probe include one having a nucleic acid sequence having a length of at least 8 contiguous nucleotides, which is homologous or complementary to the nucleic acid sequence of a gene of particular interest. Such a nucleic acid sequence may be preferably a nucleic acid sequence having a length of at least 9 contiguous nucleotides, more preferably a length of at least 10 contiguous nucleotides, and even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 20 contiguous nucleotides, a length of at least 25 contiguous nucleotides, a length of at least 30 contiguous nucleotides, a length of at least 4D contiguous nucleotides, or a length of at least 50 contiguous nucleotides. A nucleic acid sequence used as a probe, includes a nucleic acid sequence having at least 70% homology to the above-described sequence, more preferably at least 80%, and even more preferably at least 90% or at least 95%. Such a probe may be used to obtain a transposon which can be used herein.

As used herein, the term "primer" refers to a substance required for initiation of a reaction of a macromolecule compound to be synthesized, in a macromolecule synthesis enzymatic reaction. In a reaction for synthesizing a nucleic acid molecule, a nucleic acid molecule (e.g., DNA, RNA, or the like) which is complementary to part of a macromolecule compound to be synthesized may be used.

A nucleic acid molecule which is ordinarily used as a primer includes one that has a nucleic acid sequence having a length of at least 8 contiguous nucleotides, which is complementary to the nucleic acid sequence of a particular gene of interest. Such a nucleic acid sequence preferably has a length of at least 9 contiguous nucleotides, more preferably a length of at least 10 contiguous nucleotides, even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 16 contiguous nucleotides, a length of at least 17 contiguous nucleotides, a length of at least 18 contiguous nucleotides, a length of at least 19 contiguous nucleotides, a length of at least 20 contiguous nucleotides, a length of at least 25 contiguous nucleotides, a length of at least 30 contiguous nucleotides, a length of at least 40 contiguous nucleotides, and a length of at least 50 contiguous nucleotides. A nucleic acid sequence used as a primer includes a nucleic acid sequence having at least 70% homology to the above-described sequence, more preferably at least 80%, even more preferably at least 90%, and most preferably at least 95%. An appropriate sequence as a primer may vary depending on the property of the sequence to be synthesized (amplified). Those skilled in the art can design an appropriate primer depending on the sequence of interest. Such a primer design is well known in the art and may be performed manually or using a computer program (e.g., LASERGENE, Primer Select, DNAStar).

As used herein, the term "epitope" refers to an antigenic determinant. Therefore, the term "epitope" includes a set of amino acid residues which are involved in recognition by a particular immunoglobulin. Further, in the context of T cells, those residues necessary for recognition by T cell receptor proteins and/or Major Histocompatibility Complex (MHC) receptors. This term is also used interchangeably with "antigenic determinant" or "antigenic determinant site". In the field of immunology, *in vivo* or *in vitro,* an epitope is the feature of a molecule (e.g., primary, secondary and tertiary peptide structure, and charge) that forms a site recognized by an immunoglobulin, T cell receptor or MHC (e.g. HLA) molecule. An epitope including a peptide comprises 3 or more amino acids in a spatial conformation which is unique to the epitope. Generally, an epitope consists of at least 5 such amino acids, and more ordinarily, consists of at least 6, 7, 8, 9 or 10 such amino acids. The greater the length of an epitope, the more the similarity of the epitope to the original peptide, i.e., longer epitopes are generally preferable. This is not necessarily the case when the conformation is taken into account. Methods of determining the spatial conformation of amino acids are already known in the art, and include for example, X-ray crystallography and two-dimensional Nuclear Magnetic Resonance (NMR) spectroscopy. Furthermore, the identification of epitopes in a given protein is readily accomplished using techniques well known in the art. See, also, Geysen et al., Proc. Natl. Acad. Sci. USA (1984) 81: 3998 (general method of rapidly synthesizing peptides to determine the location of immunogenic epitopes in a given antigen); U.S. Patent No. 4,708,871 (procedures for identifying and chemically synthesizing epitopes of antigens); and Geysen et al., Molecular Immunology (1986) 23: 709 (technique for identifying peptides with high affinity for a given antibody). Antibodies that recognize the same epitope can be identified in a simple immunoassay. Thus, methods for determining an epitope including a peptide are well known in the art. Such an epitope can be determined using a common technique well-known by those skilled in the art, on the proviso if the primary nucleic acid or amino acid sequence of the epitope is provided.

Therefore, an epitope including a peptide requires a sequence having a length of at least 3 amino acids, preferably at least 4 amino acids, more preferably at least 5 amino acids, at least 6 amino acids, at least 7 amino acids, at least 8 amino acids, at least 9 amino acids, at least 10 amino acids, at least 15 amino acids, at least 20 amino acids, and at least 25 amino acids. Epitopes may be determined by those skilled in the art by using a commercially available kit, such as PepSet™ (Kurabo). In the present invention, presenting a protein epitope playing a role in signal transduction may be used as a system for measuring signal transduction.

As used herein, the term "agent binding specifically to" a certain nucleic acid molecule or polypeptide refers to an agent which has a level of binding to the nucleic acid molecule or polypeptide equal to or higher than a level of binding to other nucleic acid molecules or polypeptides. Examples of such an agent include, but are not limited to, when a target is a nucleic acid molecule, a nucleic acid molecule having a complementary sequence to the nucleic acid molecule of particular interest, a polypeptide capable of binding to a nucleic acid sequence of interest (e.g., a transcription agent, etc.), and the like, and when a target is a polypeptide, an antibody, a single chain antibody, either of a pair of a receptor and a ligand, either of a pair of an enzyme and a substrate, and the like. As used herein, such an agent specifically binding to (such as an agent specifically binding to calcium, an antibody against a specific gene product and the like), can be used in measuring signal transduction.

As used herein, "agent" may be any substance or any other element (e.g., energy such as light, radioactivity, heat, electricity and the like) as long as the intended purpose is fulfilled thereby. Such a substance includes, but is not limited to: e.g., a protein, polypeptide, oligopeptide, peptide, polynucleotide, oligonucleotide, nucleotide, nucleic acid (e.g., including DNA such as cDNA, genomic DNA, and RNA such as mRNA), polysaccharide, oligosaccharide, lipid, organic low molecule (e.g., hormone, ligand, signal transduction substance, organic molecule having low molecular weight, molecules synthesized by means of combinatorial chemistry, low molecule which can be used as a pharmaceutical product (e.g., low molecular ligand or the like) and the like), a complex molecule thereof. An agent specific to a polynucleotide typically includes, but is not limited to, a polynucleotide having a complementarities with a certain degree of sequence homology (for example, sequence identity of 70 % or more) against the polynucleotide of particular interest, a polypeptide such as a transcription factor binding to a promoter region, and the like. Agents specific to a polypeptide typically include, but are not limited to for example, an antibody specifically directed to the polypeptide, or a derivative or homolog thereof (for example, single-stranded antibody), a ligand or receptor specific thereto when the polypeptide is a receptor or a ligand, respectively, and a substrate in the case of where the polypeptide is an enzyme, and the like.

As used herein, the term "compound" refers to any chemical substance or a molecule which is distinguishable, and includes, but is not limited to: low molecules, peptides, proteins, sugars, nucleotides, or nucleic acids, which may be naturally-occurring or synthetic.

As used herein, the term "organic low molecule" refers to an entity having relatively low molecular weight. Usually, an organic low molecule refers to a molecule weight having about 1000 Dalton or less, or alternatively may have a molecular weight of greater than this value. Organic low molecules may be usually synthesized by a method or a combination thereof already known in the art. Such an organic low molecule may be produced by a biological organism. Organic low molecules include, but are not limited to, for example, hormones, ligands, information signaling substances, molecules synthesized by combinatorial chemistry, low molecules which can be utilized as a pharmaceutical product (for example, low molecule ligand and the like) and the like.

As used herein the term "contact" refers to physically locating a compound in the vicinity of the polypeptide or polynucleotide in the present invention in a direct or indirect manner. Polypeptides or polynucleotides may be present in a number of buffers, salts or solutions, and the like. Contact includes locating a compound in a vessel such as beaker, microtiter plate, cell culture flask or microarray (such as a gene chip) comprising a nucleic acid molecule or a fragment or a polypeptide encoded thereby, and the like.

(Variation of polypeptides or polynucleotides)

In the present invention, when using a functional polypeptide such as a IAP element and the like, a variant thereof (as used herein it is called "functional variant") may be used as long as the variant can attain similar functional characteristics, such as transposition activity and the like.

A given amino acid may be substituted with another amino acid in a protein structure, such as a cationic region or a substrate molecule binding site, without a clear reduction or loss of interactive binding ability. A given biological function of a protein is defined by the interactive ability or other property of the protein. Therefore, a particular amino acid substitution may be performed in an amino acid sequence, or at the DNA code sequence level, to produce a protein which maintains its original property after the substitution. Therefore, various modifications of peptides as disclosed herein and DNA encoding such peptides may be performed without clear losses of biological usefulness.

When the above-described modifications are designed, the hydrophobicity indices of amino acids may be taken into consideration. Hydrophobic amino acid indices play an important role in providing a protein with an interactive biological function, which is generally recognized in the art (Kyte, J. and Doolittle, R.F., J. Mol. Biol. 157(1):105-132, 1982). The hydrophobic properties of an amino acid contributes to the secondary structure of a protein and facilitates interactions between the protein and other molecules (e.g., enzymes, substrates, receptors, DNA, antibodies, antigens, etc.). Each amino acid is given a hydrophobicity index based on the hydrophobicity and charge properties thereof as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamic acid (-3.5); glutamine (-3.5); aspartic acid (-3.5); asparagine (-3.5); lysine (-3.9); and arginine (-4.5).

It is well known that if a given amino acid is substituted with another amino acid having a similar hydrophobicity index, the resultant protein may still have a biological function similar to that of the original protein (e.g., a protein having an equivalent enzymatic activity). For such an amino acid substitution, the hydrophobicity index is preferably within $\pm 2$, more preferably within $\pm 1$, and even more preferably within $\pm 0.5$. It is understood in the art that such an amino acid substitution based on hydrophobicity is efficient.

A hydrophilicity index is also useful for modification of an amino acid sequence of the present invention. As described in US Patent No. 4,554,101, amino acid residues are given the following hydrophilicity indices: arginine (+3.0); lysine (+3.0); aspartic acid (+3.0$\pm 1$); glutamic acid (+3.0$\pm 1$); serine (+0.3); asparagine (+0.2); glutamine (+0.2); glycine (0); threonine (-0.4); proline (-0.5$\pm 1$); alanine (-0.5); histidine (-0.5); a cysteine (-1.0); methionine (-1.3); valine (-1.5); leucine (-1.8); isoleucine (-1.8); tyrosine (-2.3); phenylalanine (-2.5); and tryptophan (-3.4). It is further understood that an amino acid may be substituted with another amino acid, which has a similar hydrophilicity index and can still provide a biological equivalent. For such an amino acid substitution, the hydrophilicity index is preferably within $\pm 2$, more preferably $\pm 1$, and even more preferably $\pm 0.5$.

For example, it is well known in the art that the following RNA codon (in the corresponding DNA codon, T is replaced with U), can be interchangeably used for encoding each of the particular amino acids: phenylalanine (Phe or F): UUU or UUC; leucine (Leu or L): UUA, UUG, CUU, CUC, CUA or CUG; isoleucine (Ile or I): AUU, AUC or AUA; methionine (Met or M): AUG; valine (Val or V): GUU, GUC, GUA or GUG; serine (Ser or S): UCU, UCC, UCA, UCG, AGU or AGC; proline (Pro or P): CUU, CCC, CCA or CCG; threonine (Thr or T): ACU, ACC, ACA or ACG; alanine (Ala or A): GCU, GCG, GCA or GCC; tyrosine (Tyr or Y): UAU or UAC; histidine (His or H): CAU or CAC; glutamine (Gln or Q): CAA or CAG; asparagine (Asn or N): AAU or AAC; lysine (Lys or K): AAA or AAG; asparatic acid (Asp or D): GAU or GAC; glutamic acid (Glu or E): GAA or GAG; cystein (Cys or C): UGU or UGC; arginine (Arg or R): CGU, CGC, CGA, CGG, AGA or AGC; glycine (Gly or G): GGU, GGC, GGA or GGG; termination codon: UAA, UAG or UGA. Further, a specific DNA sequence is modified to employ a preferential codon for a specific cell type. For example, preferential codon usage of E. coli, is known in the art, as is the preferential codon usage of an animal and a human. Such a modification is well known in the art, and constitutes a part of the present invention.

Variants (e.g. retrotransposon) thus produced are also within the scope of the present invention, and any of such variants are used in the present invention.

(Antigen and antibody)

As used herein, the term "antibody" encompasses polyclonal antibodies, monoclonal antibodies, human antibodies, humanized antibodies, polyfunctional antibodies, chimeric antibodies, and anti-idiotype antibodies, and fragments thereof (e.g., F(ab')$_2$ and *Fab* fragments), and other recombinant conjugates. These antibodies may be fused with an enzyme (e.g., alkaline phosphatase, horseradish peroxidase, $\alpha$-galactosidase, and the like) via a covalent bond or by recombination.

As used herein, the term "monoclonal antibody" refers to an antibody composition having a group of homologous antibodies. This term is not limited by the production manner thereof. This term encompasses all immunoglobulin molecules and *Fab* molecules, F(ab')$_2$ fragments, *Fv* fragments, and other molecules having an immunological binding property of the original monoclonal antibody molecule. Methods for producing polyclonal antibodies and monoclonal antibodies are well known in the art, and will be more sufficiently described below.

Monoclonal antibodies are prepared by using standard techniques already well known in the art (e.g., Kohler and Milstein, Nature (1975) 256:495), or a modification thereof (e.g., Buck et al. (1982) In vitro 18:377). Representatively, a mouse or rat is immunized with a protein bound to a protein carrier, and boosted. Subsequently, the spleen (and optionally several large lymph nodes) are harvested and dissociated into a single cell suspension. If desired, the spleen cells may be screened (after removal of nonspecifically adherent cells) by applying the cell suspension to a plate or well coated with a protein antigen. B-cells that express membrane-bound immunoglobulin specific for the antigen bound to the plate, are not rinsed away with the rest of the suspension. Resulting B-cells, or all dissociated spleen cells, are then induced to fuse with myeloma cells to form hybridomas. Theses hybridomas are subsequently used to produce monoclonal antibodies.

As used herein, the term "antigen" refers to any substrate to which an antibody molecule may specifically bind. As used herein, the term "immunogen" refers to an antigen capable of initiating activation of the antigen-specific immune response of a lymphocyte. A-ccordingly, chemical receptors or downstream products thereof may be used as an antigen or immunogen, and uses antibody-antigen response to conduct selection of the genome variant product of the present invention using antigen-antibody reaction.

(Gene Engineering)

As used herein, the term " gene cassette" refers to a nucleic acid sequence comprising DNA encoding a gene, a nucleic acid sequence comprising a gene promoter operably linked thereto (such that it can control the expression of the DNA), a promoter, and optionally a heterologous gene operably linked thereto (i.e., in frame). It is intended that the use of this cassette optionally in combination with another regulatory element is encompassed in the present invention. Preferably expression cassettes are those which are amenable to specific restriction enzyme digestion and are feasible for recovery.

When a gene is mentioned herein, the term "vector" or "recombinant vector" refers to a vector transferring a polynucleotide sequence of interest to a target cell. Such a vector is capable of self-replication or incorporation into a chromosome of a host cell (e.g., a prokaryotic cell, yeast, an animal cell, a plant cell, an insect cell, an individual animal, and an individual plant, etc.), and contains a promoter at a site suitable for transcription of a polynucleotide of the present invention. A vector suitable for performing cloning is referred to as a "cloning vector". Such a cloning vector ordinarily contains a multiple cloning site (MCS) containing a plurality of restriction sites. Restriction enzyme sites and multiple cloning sites as described above are well known in the art and can be used as appropriate by those skilled in the art depending on the purpose in accordance with publications described herein (e.g., Sambrook et al., *supra*).

As used herein, the term "expression vector" refers to a nucleic acid sequence comprising a structural gene and a promoter for regulating expression thereof. In addition, they may contain various regulatory elements in a state that allows them to operate within host cells. The regulatory element may include, preferably, terminators, selectable markers such as drug-resistance genes, and enhancers. It is well known in the art that a type of an expression vector of a living organism such as an animal and a species of a regulatory element used may vary depending on the type of host cell used.

Examples of "recombinant vectors" for prokaryotic cells include, but are not limited to, pcDNA3(+), pBluescript-SK(+/-), pGEM-T, pEF-BOS, pEGFP , pHAT, pUC18, pFT-DEST™42GATEWAY (Invitrogen), and the like.

Examples of "recombinant vectors" for animal cells include, but are not limited to, pcDNAI/Amp, pcDNAI, pCDM8 (all commercially available from Funakoshi), pAGE107 [Japanese Laid-Open Publication No. 3-229 (Invitrogen), pAGE103 [J. Biochem., 101, 1307(1987)], pAMo, pAMoA [J. Biol. Chem., 268, 22782-22787(1993)], a retrovirus expression vector based on a murine stem cell virus (MSCV), pEF-BOS, pEGFP, and the like.

Examples of recombinant vectors for plant cells include, but are not limited to, pPCVICEn4HPT, pCGN1548, pCGN1549, pBI221, pBI121, and the like.

As used herein, the term "terminator" refers to a sequence that is located downstream of a protein-encoding region of a gene, and which is involved in the termination of transcription when DNA is transcribed into mRNA, and the addition of a poly-A sequence. It is known that a terminator contributes to the stability of mRNA, and has an influence on the amount of gene expression with the host cell.

As used herein, the term "promoter" or "promoter sequence" refers to a base sequence which determines the initiation site of transcription of a gene and is a DNA region which directly regulates the frequency of transcription. Transcription is started by RNA polymerase binding to a promoter. Accordingly, a portion having promoter function of a gene herein refers to "promoter moiety". A promoter region is usually located within about 2 kbp upstream of the first exon of a putative protein coding region. Therefore, it is possible to estimate a promoter region by predicting a protein coding region in a genomic base sequence using DNA analysis software. A putative promoter region is usually located upstream of a structural gene, but it is not limited thereto, and is dependant on the structural gene, i.e., a putative promoter region may be located downstream of a structural gene.

As used herein, the term "enhancer" refers to a sequence which is used as to enhance the expression efficiency of a gene of interest. One or more enhancers may be used, or no enhancer may be used.

As used herein, the term "silencer" refers to a sequence which has a function of suppressing and arresting the expression of a gene. Any silencer which has such a function may be herein used. No silencer may be used.

As used herein, the term "operably linked" indicates that a desired sequence is located such that expression (operation) thereof is under control of a transcription and translation regulatory sequence (e.g., a promoter, an enhancer, and the like) or a translation regulatory sequence. In order for a promoter to be operably linked to a gene, typically the promoter is located immediately upstream of the gene. However, a promoter is not necessarily adjacent to a structural gene. In the case of IAP, a promoter is preferably located directly immediately thereto for advantageous effects.

As used herein, technologies for introducing a nucleic acid molecule into a cell may be of any type, and includes for example, transformation, transduction, transfection and the like. Such a technology for introducing a nucleic acid molecule is well known in the art and is routinely used, and includes, for example, those described in Ausubel F. A. et al. ed. (1988), Current Protocols in Molecular Biology, Wiley, New York, NY; Sambrook J. et al. (1987) Molecular Cloning: A Laboratory Manual, 2nd Ed. and the third version thereof, Cold Spring Harbor Laboratory Press, Cold Spring Harbor, NY, Suppln. Experimental Medicine "Gene Introduction & Expression Analysis Experimental Procedure", Yodosha 1997. Furthermore, the introduction of genes may be confirmed by means of those described herein, such as Northern Blotting, Western Blotting analysis and other well known and routinely used technologies.

Methods of introducing a vector is also achieved by any of the above-mentioned methods for introducing a DNA into a cell, and include for example, transfection, transduction, transformation and the like, such as calcium phosphate, liposome methods, DEAE dextran methods, electroporation methods, particle gun methods (gene gun), and the like, lipofection, spheroplast Proc. Natl. Acad. Sci. USA, 84, 1929 (1978)],. lithium acetate method [J. Bacteriol., 153, 163 (1983)], a method described in Proc. Natl. Acad. Sci. USA, 75, 1929 (1978) and the like.

As used herein, the term "gene introduction reagent" refers to a reagent which is used in a gene introduction method so as to enhance introduction efficiency. Examples of gene introduction reagents include, but are not limited to, cationic polymers, cationic lipids, polyamine-based reagents, polyimine-based reagents, calcium phosphate, and the like. Specific examples of a reagent used in transfection include reagents available from various sources, such as, without limitation, Effectene Transfection Reagent (cat. no. 301425, Qiagen, CA), TransFast™ Transfection Reagent (E2431, Promega, WI), Tfx™-20 Reagent (E2391, Promega, WI), SuperFect Transfection Reagent (301305, Qiagen, CA), PolyFect Transfection Reagent (301105, Qiagen, CA), LipofectAMINE 2000 Reagent (11668-019, Invitrogen corporation, CA), JetPEI (x4) conc. (101-30, Polyplus-transfection, France) and ExGen 500 (R0511, Fermentas Inc., MD), and the like. In the present invention, such a gene introduction reagent may be used when introducing the nucleic acid molecule of the present invention into a cell.

Gene introduction efficiency may be calculated by measuring the cell number of introduced or exhibit the expression product of the introduced foreign substance (introduced gene) (for example, gene product of a reporter gene, fluorescence protein GFP and the like) per unit area (for example, 1 mm$^2$ and the like); intensity of total signal (in case of fluorescence protein, fluorescence).

As used herein, the term "transformant" refers to the whole or a part of an organism, such as a cell or a tissue, which is produced by transformation. Examples of transformants include a prokaryotic cell, yeast, an animal cell, a plant cell, an insect cell, and the like. Transformants may be referred to as transformed cells, transformed tissue, transformed hosts, or the like, depending on the subject, and may refer to any specific form depending on the context. Cells used in the present invention may be a transformant.

When a prokaryotic cell is used in genetic engineering in the present invention, prokaryotic cells include the following genera: Escherichia, Serratia, Bacillus, Brevibacterium, Corynebacterium, Microbacterium, Pseudomonas, for example, those species including Escherichia coli XL1-Blue, Escherichia coli XL2-Blue, *Escherichia coli* DH1. Alternatively, in the present invention, cells isolated from a naturally occurring substance may also be used.

Animal cells which can be used in genetic engineering or the like herein, include murine myeloma cells, rat myeloma cells, murine hybridoma cells, Chinese Hamster cells including CHO cell, BHK cell, African Green Monkey kidney cells, human leukemia cells, HBT5637 (see Japanese Laid-Open Publication 63-299), human colon cancer cell line and the like. Murine myeloma cells include ps20, NSO and the like; rat myeloma cells include YB2/0 and the like; human fetal kidney cells include HEK293 (ATCC: CRL-1573) and the like; human leukemia cells include BALL-1 and the like; African green monkey kidney cells include COS-1, COS-7 and the like; human colon cancer cell lines include HCT-15; human neuroblastoma cells include SK-N-SH, SK-N-SH-5Y and the like; murine neuroblastoma cells include Neuro2A and the like as examples. Alternatively, the present invention may use a primary cultured cell.

Plant cells which can be used in genetic engineering herein include callus, or a portion thereof and suspension culture cells, those cells from Solanaceae, Gramineae, Brassicaceae, Rosaceae, Leguminosae, Cucurbitaceae, Lamiacea, Liliaceae, Chenopodiaceae, Apiaceae and the like.

Gene expression (e.g., mRNA expression, polypeptide expression) may be "detected" or "quantified" by an appropriate method, including mRNA measurement and immunological measurement. Examples of molecular biological measurement methods include Northern blotting methods, dot blotting methods, PCR methods, and the like. Examples of immunological measurement method include ELISA methods, RIA methods, fluorescent antibody methods, Western blotting methods, immunohistological staining methods, and the like, where a microtiter plate may be used. Examples of quantification methods include ELISA methods, RIA methods, and the like. A gene analysis method using an array (e.g., a DNA array, a protein array, etc.) may be used. The DNA array is widely reviewed in Saibo-Kogaku [Cell Engineering], special issue, "DNA Microarray and Up-to-date PCR Method", edited by Shujun-sha. The protein array is described in detail in Nat Genet. 2002 Dec; 32 Suppl:526-32. Examples of methods for analyzing gene expression include, but are not limited to; RT-PCR methods, RACE methods, SSCP methods, immunoprecipitation methods, two-hybrid systems, *in vitro* translation methods, and the like in addition to the above-described techniques. Other analysis methods are described in, for example, "Genome Analysis Experimental Method, Yusuke Nakamura's Lab-Manual, edited by Yusuke Nakamura, Yodo-sha (2002), and the like. All of the above-described publications are herein incorporated by reference.

As used herein, the term "expression" of a gene product, such as a gene, a polynucleotide, a polypeptide, or the like, indicates that the gene or the like is affected by a predetermined action *in vivo* to be changed into another form. Preferably, the term "expression" indicates that genes, polynucleotides, or the like are transcribed and translated into polypeptides. In one embodiment of the present invention, genes may be transcribed into mRNA. More preferably, these polypeptides may have post-translational processing modifications.

As used herein, the term "expression level" refers to the amount of a polypeptide or mRNA expressed in a subject cell. The term "expression level" includes the level of protein expression of a polypeptide evaluated by any appropriate method using an antibody, including immunological measurement methods (e.g., an ELISA method, an RIA method, a fluorescent antibody method, a Western blotting method, an immunohistological staining method, and the like, or the mRNA level of expression of a polypeptide evaluated by any appropriate method, including molecular biological measurement methods (e.g., a Northern blotting method, a dot blotting method, a PCR method, and the like). The term "change in expression level" indicates that an increase or decrease in the protein or mRNA level of expression of a polypeptide evaluated by an appropriate method including the above-described immunological measurement method or molecular biological measurement method.

Accordingly, as used herein, "reduction" of "expression" of a gene, a polynucleotide, a polypeptide or the like refers to when an agent of the present invention is subjected to an action, and the amount of expression is significantly reduced compared to that when the agent is not subjected to an action. Preferably, the reduction of expression includes a reduction of the level of polypeptide expression. As used herein, the "increase" of "expression" of a gene, a polynucleotide, a polypeptide or the like refers to when an agent of the present invention is subjected to an action (or an agent relating to gene expression into a cell, for example, a gene to be expressed or an agent for regulating the same), resulting in the amount of expression is significantly increased compared to when the agent is not subjected to an action. Preferably, the increase of an expression includes a subsequent increase in the level of polypeptide expression. As used herein, the term "induction" of "expression" of a gene refers to an increase in the level of expression of the gene by acting an agent on a cell. Accordingly, the induction of expression encompasses the expression of the gene when no expression of the gene had been observed, and the increase in the level of expression of the gene when the level of the expression of the gene had already been observed.

As used herein, the term "specifically express(ing)" of a gene refers to expression in a different level (preferably in a higher level) in a specific site or period of time than that of the other site or period of time. Specific expression may refer to expression in a certain site (specific site) or may also refer to the expression including that in another site. Preferably, specific expression refers to the expression in the certain site only. A gene to be introduced into a biological organism by the present invention may be modified such that specific expression is thus achieved.

As used herein, the term "biological activity" refers to activity possessed by an agent (e.g., a polynucleotide, a protein, etc.) within an organism, including activities exhibiting various functions such as transcription promoting activity. When a collage interacts with the ligand thereof, the biological activity thereof encompasses formation of a conjugate or other biological change. In another embodiment, such a biological activity may be gene transposition activity and the like. Gene transposition activity may be determined by confirming the movement of a sequence encoding a gene of interest by any means. For example, when an agent is an enzyme, the biological activity thereof encompasses the enzymatic activity thereof. In another example, when an agent is a ligand, the activity encompasses the binding of the ligand to the receptor thereof. Such a biological activity may be determined by any well known technology in the art (see, for example, Molecular Cloning, Current Protocols, which is herein incorporated by reference).

As used herein, the term "kit" refers to a unit typically comprising two or more sections which provide portions (e.g., of a reagent, a particle, a cell, a nucleic acid and the like). When components are not provided as a mixture and are preferably mixed immediately before use, this form of the kit is preferable. It is advantageous that such a kit preferably comprises instructions describing how to treat a portion to be presented (for example, a reagent, a particle and the like). Such instructions may be of any medium, and includes, but is not limited to for example, paper-medium, transmitting medium, storage medium and the like. Transmitting media includes, but are not limited to the internet, intranet, extranet, LAN and the like. Storage media include, but are not limited to CD-ROM, CD-R, flexible disk, DVD-ROM, MD, mini-disc, MO, memory stick and the like.

(Transgenic biological organism)

A general technique for producing transgenic mice is described in International Publication WO91-13150 (Ludwig Inst. Cancer Res.). US Patent No. 4,873,191 (Wagner et al.) that teaches a mammal having an exogenous DNA, which was obtained by microinjection of the DNA into a mammalian zygote. Further, a method of efficiently producing mutants of an animal, a plant, or the like has been studied, in which a transposable genetic element (transposon) is inserted or transposed into endogenous DNA so that the structure of the DNA is changed and the DNA is thus inactivated. Transposons have been available for introduction, addition, and the like of a particular gene into a chromosome. In principle, these technologies can also be used with retrotransposons.

In addition, a variety of methods for producing transgenic biological organisms include, but are not limited to those described in, for example: M.Markkula et al., Rev.Reprod., 1, 97-106 (1996); R. T. Wall et al., J. Dairy Sci., 80, 2213-2224 (1997); J. C. Dalton, et al., Adv. Exp. Med. Biol., 411, 419-428 (1997); and H. Lubon et al., Transfus. Med. Rev., 10, 131-143 (1996), which are herein incorporated by reference, respectively.

In such circumstances, in the last ten years, analysis of transgenic (including knock-out, knock-in) animals via homologous recombination of embryonic stem (ES) cells is becoming of note for the purpose of analysis of gene functions.

In higher biological organisms, for example, efficient selection of recombinants by means of positive selection using the neomycin resistance gene, and negative selection using the HSV thymidine kinase gene or the diphtheria toxin gene are known. Homologous recombinants are selected by PCR or a Southern blotting method, i.e., a portion of a target gene is replaced with the neomycin resistance gene or the like for positive selection, and at the bottom thereof, targeting vectors in which the HSVTK gene or the like is linked for negative selection at the terminus, to introduce the same into an ES cell by electroporation, and selected in the presence of G418 and gancyclovir. The resultant colonies are isolated and selected for homologous recombinants by means of PCR or Southern blotting.

As such, a method for producing a transgenic (targeted gene recombination) mouse having substitution or disruption of a internal target gene, and having lost the function thereof or having altered the mutation, is useful since mutations are introduced only in the gene which is targeted for the analysis of the gene function.

After selection of a desired homologous recombinant, the resultant recombinant ES cell is mixed with a normal embryo by the blastocyst injection method or the collection chimeric method to produce a chimeric mouse between the ES cell and the host embryo. In the blastocyst injection method, ES cells are injected into the blastocyst by a glass pipette. In the collection chimeric method, the mass of ES cells and an embryo of the eight-cell phase, which has a removed clear zone, are subsequently fused. The blastocyst with the ES cell introduced therein is transferred to the uterus of a pseudo pregnant surrogate mother to produce a chimeric mouse. Since an ES cell has totipotency, it can differentiate into any type of cell including a germline cell *in vivo.* When a chimeric mouse having the germline cell derived from the ES cell and a normal mouse are crossbred,. Therefore, a mouse having the chromosome of ES cell in a heterologous manner, and a transgenic mouse having the modified chromosome of ES cell in a homologous manner shall be obtained by crossbreeding the mice to each other. In order to obtain a transgenic mouse having the modified chromosome in a homologous manner from the resultant chimeric mouse, a male chimeric mouse and a female wild-type mouse is crossbred to produce a F1 progeny of heterozygous mouse, and the resultant male and female heterozygous mice are crossbred and select homozygous mouse in the F2 progeny. Whether or not a desired gene mutation is introduced in each progeny of F1 and F2, any methods routinely used in the art such as Southern blotting, PCR, sequencing, and the like as in the assays for recombinant ES cells.

However, the production technology of a transgenic animal being presently conducted has a defect in that it is difficult to selectively analyze a variety of gene functions. There is also the disadvantage in that transgenic biological organisms cannot be readily produced.

Further, production of present transgenic animals requires disruption from initiation or disruption and replacement with respect to a desired gene after identification of such a gene as described above. Thus, it labor intensive and time consuming, and even those researchers familiar with the technology do not always succeed. Accordingly, it is still a labor-intensive operation.

As such, in order to overcome the problem in which a variety of gene functions cannot be selectively analyzed, it is of note that Cre recombinase cell-type specific expression and Cre-loxP site specific recombination are combined. Transgenic mice using Cre-loxP are produced by introducing the neomycin resistance gene in a location, such that the expression of a target gene is not blocked, further introducing a targeting vector into an ES cell, the vector containing the loxP sequence introduced therebetween in a manner such that the exon to be deleted is sandwiched, and isolating the homologous recombinant. The isolated clone is subsequently used to obtain a chimeric mouse, and a genetically modified mouse is produced. Next, when the mouse is cross bred with a transgenic mouse, the tissue-specifically expresses site specific recombinant enzyme *Cre* from P1 phage of *E. coli,* genes are disrupted only in the tissue expressing Cre (herein, Cre specifically recognizes loxP sequence (34bp) to raise recombination between the two lox P sequences, resulting in the disruption thereof. It is now possible to express Cre in an adult by crossbreeding the adult with a transgenic mouse expressing the Cre gene linked to an organ specific promoter or using a viral vector having the Cre gene.

The gene trapping (gene trap) method is of note as a method for analyzing a specific gene. In the gene trapping method, a reporter gene having no promoter is introduced into a cell, and once the gene is inserted into the genome in an accidental manner to express the reporter gene, it is then used to isolate (trap) a novel gene. The gene trapping method is a method for efficient insertion mutation and identification of unknown gene, based on mouse primary embryo operation method, embryonic stem cell culture method and a gene targeting method via homologous recombination (Stanford WL., et al., Nature Genetics 2:756-768(2001)). The gene trap method allows introduction of a gene, selection of a mutant and analysis of the phenotype with relative ease.

In the gene trap method, for example, a gene trapping vector having beta-geo, a fusion gene between *lacZ* and *neo,* has been linked between the splicing/acceptor sequence and the polyA addition signal. Subsequently it is introduced into an ES cell, and selected with G418 to allow selection of the clones which have accidentally trapped the gene expressed by the ES cell.

Production of a chimeic embryo from thus a obtained clone, a variety of X-gal stain patterns will be shown depending on the expression patterns of the genes. As such, in the gene trapping method, unknown genes will be isolated and analyzed for the gene expression patterns thereof, or the gene is disrupted. The present invention is used for enhancing transposition efficiency by methylation and the analytical efficiency of genes will also be greatly enhanced.

In the "retrotransposon-containing transgenic organism", the retrotransposon can be transposed to any site on the chromosomes as it is contained in a transposable manner. As such, it is possible to disrupt, reduce or activate a genetic function of any site on the chromosomes by means of this transposition.

In one embodiment, the biological organism of the present invention is induced from a stem cell or a fertilized egg having a retrotransposon. Therefore, "substantially all cells" have retrotransposon genes and should be copied and inserted. "Substantially all cells" is meant all cells except for such a particular cell(s). In each cell of the above-described biological organism, a retrotransposon is randomly transposed. For this reason, no uniform mutation is found in a whole individual among genetic mutations introduced by the retrotransposon.

In the present invention, a desired transgenic biological organism may be obtained by prescreening. As a prescreening method, a gene trap method can be used, for example (Zambrowicz et al.,; Nature, 392:608-611 (1998); Gossler, A. et al.; Science, 244:463-465 (1989); Skarnes, W.C. et al.; Genes Dev, 6:903-918 (1992); and Friedrich, G. et al.; Genes Dev, 5:1513-1523 (1991)). Thus, pre-screening is performed to select in advance transgenic biological organism desirable for clarification of gene function. Thereafter, crossbreeding over two or more generations or other appropriate means can be performed to obtain a transgenic biological organism in which both genes of a pair of chromosomes are mutated.

A method of analyzing the phenotype of a gene by disrupting the gene is an effective means for clarifying gene function. There are two big problems to be overcome in order to analyze phenotypes by exhaustive gene disruption for a individual mammal, particularly a mouse. The first problem is that there is no satisfactory technique for exhaustively disrupting genes so as to investigate gene function from phenotypes, i.e., so-called forward genetics. The second problem is that since there are a pair of genes (both alleles), a phenotype does not appear if only one member of the pair of genes is disrupted. Currently, individuals having one disrupted member of a pair of genes are crossbred in order to introduce a mutation into both alleles. In other words, a long time is required for crossbreeding to obtain an individual in which a mutation is introduced into both alleles.

The first problem can be overcome by a transposon system newly developed in the present invention. The second problem can be overcome by a method of rapidly introducing a mutation into both alleles. As a specific method for overcoming the second problem, a Bloom gene knockout mouse, in which cells having a mutation in both alleles frequently appear, can be used (G. Luo et al.; Nature Genetics, 26:424-429 (2000)). As an example of regulatable expression of Blood gene, means such as tetracyclin regulatable unit can be introduced in combination of retrotransposon system. For example, before crossbreeding, a means for regulatably expressing the Bloom gene is introduced into a fertilized egg or the like, into which a retrotransposon or the like is to be introduced. The obtained mouse having an introduced retrotransposon transposition site is treated with a means for inhibiting expression of the Bloom gene (e.g., administration of tetracycline) so that a genetic mutation obtained by a retrotransposon system is introduced into both alleles, thereby making it possible to rapidly determine a phenotype. In the present invention, when no selectable marker gene is used, DNA may be extracted from cells of a non-human mammal and may be then screened by investigating the presence or absence of transposition by Southern Blotting. According to the present invention, it is possible to achieve efficient transposition of a retrotransposon sequence in animals *in vivo*. According to a method for introducing a mutation using retrotransposon, it is now possible to randomly obtain in a more efficient manner for organisms having a variety of expression type. The transgenic organism of the present invention is extremely useful tool for clarifying complex life processes in gene function research since various genetic mutations can be introduced.

In the present invention, retrotransposon expression systems allows great enhancement in transposition efficiency of retrotransposon by forming an animal body or cell aggregates such as the tissue or organ thereof. It facilitates extreme enhancement of transposition efficiency of a retrotransposon.

According to an embodiment of the present invention, it is possible to screen a transgenic organism having an introduced transposon construct for individuals having a randomly introduced mutation using a marker or other means. This is useful as means for clarifying gene function. For exhaustive analysis of gene function, it is necessary to cause a transposon to be transposed to a greater number of sites on a genome.

According to the present invention, by producing mutated mice from different seed mice, it is possible to exhaustively introduce a mutation into substantially all genes, the number of which is believed to be at least about 30,000. Therefore, in analysis of non-human mammals having mutations, since the present invention can achieve a considerably high level of expression frequency of genetic mutations, a number of functional changes by mutations can be simultaneously analyzed from if a single organism individual having a plurality of mutations is obtained. Transposon (DNA-type) has the limitation in which sites to be transposed are limited, and thus exhaustiveness rate is limited. As such, the present invention readily allows utilization of a retrotransposon, also rendering exhaustive mutagenesis introduction.

According to the present invention, by obtaining and crossbreeding transgenic organisms, it is possible to obtain organisms having fixed transpositions, which are useful for clarification of gene function. As used herein, "fixed transposition" means that the number of signature sites produced by transposition of a retrotransposon is not increased due to the lack of an active retrotransposon. Specifically, this indicates either the case where at least one signature site and a retrotransposon are present but no inactivated retrotransposon is present. If such a transgenic organism individual is obtained, a type of gene function can be simply analyzed by investigating a corresponding single individual.

In the present invention, mutations are introduced by retrotransposons. Therefore, mutation introduction sites can be easily detected by an appropriate method, such as PCR or the like, using a signature sequence or a sequence derived from a retrotransposon construct, as compared to when a mutation is introduced using a mutation inducing substance or the like. In an embodiment of the present invention, by introducing a genetic mutation into a organism individual, but not cultured cells, it is possible to analyze gene function in individuals. It is also possible to introduce a genetic mutation into *in vivo* tissue of a non-human mammal individual, which is difficult to handle while the organism individual remains alive, without external manipulation. Further, transposition sites differ even within the same tissue, so that there are genetically different cells. Therefore, the lineage of cells, such as proliferation, differentiation, and the like, can be systematically investigated in any tissue and organs, such as the blood system, the immune system, and the like.

According to the present invention, a novel biological organism (particularly, a mouse) of the present invention provides a model system useful for clarification of gene function. This embodiment of the present invention may provide a model system of disease for studies on genetic disease in *in vivo* animal models. In the system, examples of disease genes to be introduced into animal models include human disease causative genes, homologous genes of biological organisms with the human disease causative genes, full-length cDNA genes, cDNA gene fragments, full-length genomic DNA genes, and genomic DNA gene fragments. Such a disease causative gene is not particularly limited. Any disease causative gene can be used as long as it can be introduced into biological organisms and the resultant transgenic biological organisms can be studied as animal models of human disease. However, Human disease causative genes are preferable. According to one embodiment of the present invention, when a retrotransposon containing various enhancers are transposed near proto-oncogenes, cancer is eventually expressed in the cells containing these genes. Therefore, it is possible to perform screening for proto-oncogenes. In particular, when a transgenic biological organism containing a retrotransposon sequence is used, cancer undergoes metastasis over the whole body as well as tissues since proto-oncogenes are clonally expressed. At the same time, reduction, disruption, or activation of gene function due to transposition randomly proceeds in each animal cell. It is expected that a plurality of cancers occur in the same individual. Therefore, clarification of gene function involved in cancer can be efficiently developed. Further, when a plurality of cancers are confirmed in the same individual, it is possible to investigate whether or not cancerous cells are derived from the same cell by investigating whether or not the insertion site of a retrotransposon vector is the same for the cancerous cells. Thus, the present invention may contribute to research on the mechanism of cancer metastasis.

In the present invention, the transgenic biological organism of the present invention may be used as a donor for organ transplantation. Examples of organs which are considered to be used as donors for heterograft to a human, include neurons, heart, lung, liver, pancreas, kidney, cornea, skin, and the like. In this case, as an introduced gene, a gene having a function of possibly reducing rejection or a gene having a function of expectedly increasing acceptance are preferable in heterograft, for example.

For production of transgenic biological organisms, refer also to: those references including, but not limited to: US patent Nos.: 5,464,764; 5,487,992; 5,627,059; Japanese Laid-Open Publication 2001-54337; Gossler, A. et al. (1989), Science 244, 463-465; Wurst, W. et al. (1995), Genetics 139, 889-899; Zambrowicz, B. P. et al. (1998), Nature 392, 608-611 Proc.Natl. Acad. Sci. USA, Vol. 86, 8932-8935, 1989; Nature, Vol. 342, 435-438, 1989; M. Muramatsu and M. Yamamoto ed. "Jikken Igaku Bessatsu, Shin-tei, Idenshi Kogaku Handobukku Kaitei Daisanhan" (Experimental Medicine, Suppl. New Revision, Gene Engineering Handbook, Third Edition" (1999, Yodosha), in particular, pages 239-256; S. Aizawa (1995) Jikken Igaku "jiin taagettingu - ES saibo wo mochiita hen'i mausu no sakusei" (Experimental Medicine, Gene Targeting - production of mutant mouse using ES cell) and the like.

As used herein the term "knock out", when referring to a gene, refers to rendering the disruption (deletion) of the gene or rendering the function of the gene deficient. Accordingly, the concept of a knock out is encompassed by transgenic animals.

As used herein, the term "knock-out biological organism" refers to a biological organism (for example, mouse) in which a gene is knocked out. Accordingly, the concept of knock-out biological organisms are encompassed by a transgenic biological organisms.

As used herein the term "biological organism" which is the object of the transgenic biological organism, encompasses any biological organism for which a transposon acts, and in which such a transgenic system can function. Such a biological organism includes, but is not limited to an animal, a plant, a bacteria and the like.

As used herein the term "animal" refers to any animal, which can be targeted by the introduction of a nucleic acid sequence (preferably a foreign sequence encoding a gene). Accordingly, an animal includes a vertebrate and invertebrate. An animal includes for example, mammals (for example, mouse, dog, cat, rat, monkey, pig, cattle, sheep, rabbit, dolphin, whale, goat, horse and the like), birds (for example, chicken, quail and the like), amphibian (for example, frog and the like), reptiles, insects (for example, Drosophila and the like), and the like. Preferably, an animal may be a mammal, and preferably, an animal, which is amenable to the production of a knock-out biological organism (for example, mouse). In another preferable embodiment, an animal may be an animal which is known to be appropriate as a human model animal (for example, monkey). In an embodiment, an animal may be, but is not limited to: non-human animal or non-human mammal. An animal may be, for example, pig, monkey, cattle, horse, goat, sheep, cat, dog, rabbit, mouse, rat, or hamster and the like, and more preferably, mouse or rat. As used herein, the biological organism of the present invention, unless otherwise stated, includes not only mammalian individuals, but also a part of an individual, or organs or tissue possessed by an individual. These may be useful as a human disease model or a donor for organ transplantation.

As used herein the term "plant" collectively refers to an organism belonging to the kingdom of *Plantae* and is typically characterized in chlorophyl, hard cell wall, presence of abundant permanent embryonal cells, and incapability of movement or the like. Typically, plant refers to *Phanerogamae* having formatino of cell walls, an anabolism action by chlorophyll. "Plant" encompasses both monocotyledonous plants and dicotyledonous plants. Preferably plants include, but are not limited to, for example, monocotyledonous plants belonging to Gramineae such as rice, wheat, maize, barley, sorghum, and the like. Preferably, plant may be rice. Rice includes but is not limited to *japonica* and *indica* variants. More preferably, rice may be *japonica* variant. As used herein variants of rice include but are not limited to, for example, Nipponbare, Nihonmasari, Kinmaze, Norin No. 22, Chiseiasahi, Koshihikari, Akitakomachi, Dontokoi, Hinohikari and the like. *Indica* variants include, but are not limited to Tetep, Basmati, IR8, Hunanzao, and the like. Preferable plants are not limited crops, but also flowers, trees, turfs, weeds and the like. Unless otherwise stated, plant refers to any part of a plant body, plant organ, plant tissue, plant cell, and seed. Examples of plant organs include root, leaf, stem and flower and the like. Examples of plant cells include callus and suspended culture cells.

Examples of Gramicear plants include plants belonging to Oryza, Hordenum, Secale, Scccharum, Echinochloa, or Zea, and include rice, barley, rye, Japanese millet, sorghum, maize and the like.

Plants used for a method for production according to the present invention are preferably monocotyledonous plants, and more preferably Gramineae plants. More preferably, it may be rice.

In the above-mentioned organisms, introduction technology of a gene includes a method selected from the group consisting of microinjection, a combination of a nucleic acid fragment and a cationic lipid vesicle or DNA aggregation reagent, and introduction of a nucleic acid fragment to a viral vector followed by contact with a cell with the virtual vector, and particle bombardment and electroporation.

Viral vectors which may be used herein, include but are not limited to: retroviral vectors, adenovirus vectors, herpes virus, and adeno-associated vectors, and the like.

As used herein the term "retrovirus" refers to a virus which has genetic information in the form of RNA, and synthesize a DNA from the information of the RNA via reverse transcipitase. Accordingly, "retroviral vector" refers to a form of a retrovirus which is used as a vector for a gene. "Retroviral vectors" as used herein include, but are not limited to, for example, retroviral type expression vector based on Moloney Murine Leukemia Virus (MMLV), Murine Stem Cell Virus (MSCV) and the like.

Preferably, retroviral vectors include, but are not limited to: pGen-, pMSCV and the like.

As used herein the term "gene trap (method)" refers to a method for identification of a gene using the fact that a desired cell is introduced with a reporter gene with lack of a promoter. For example, reporter activity is only detected when the reporter gene is inserted downstream of a promoter in an activated form in the chromosome. Such a gene trap is achieved by introducing a "gene trap vector" into the host chromosome of a eukaryotic organism and disrupting the host gene. A gene which was introduced with a reporter gene, expresses a complex protein with a reporter, and thus it is capable of identifying a gene by monitoring the protein. Accordingly, a reporter gene is incorporated into the original locus as in the homologous recombination, it is possible to produce a complete reporter system with respect to the transcription regulation. By means of these methods, it is possible to identify a gene which cannot be obtained by a method for isolation of a mutants via gene disruption. Accordingly, the present invention can use of these gene trapping method.

As used herein the term "gene trap vector" refers to a vector for selection of a vector inserted into a gene, using a phenomenon in which in the process of mRNA of a eukaryotic organism gene is matured into a mature mRNA, splicing mechanism is taken place. Gene trap vectors include, but are not limited to (1) a vector comprising a coding region of a reporter gene having no promoter, and a DNA sequence comprising splice-acceptor sites, or (2) a vector comprising a coding region of a reporter gene having a promoter, and a DNA comprising splice-donor sites, and (3) a vector comprising the DNA sequence of both (1) and (2), and the like.

Gene trapping vectors comprising splice/acceptor sequence as described above, may comprise polyA addition signal as necessary. A gene trapping vector comprising a splice/donor sequence may comprise enhancer region, and/or mRNA instability region, as necessary. PolyA addition signal includes, but is not limited to: "AATAAA".

Promoters used in the present invention include but are not limited to: MC1 promoter, RNA pol II promoter and the like.

Enhancers used in the present invention include but are not limited to polyoma viral enhancer (PYF441) and the like.

Splice donor sequences used in the present invention include but are not limited to murine *hprt* gene exon 8 splice donor.

Splice acceptor sequence used in the present invention include, but are not limited to human bcl-2 gene exon 3 splice acceptor.

As used herein the term "reporter" molecule or "reporter" gene refers to a molecule (e.g. polypeptide) or gene which can be used as an indicator of gene expression in a cell. Such a molecule may be of a known reporter protein, and includes, but is not limited to for example, chloramphenicol acetyl transferase (CAT), beta-glucuronidase (GUS), beta-D-galactosidase, luciferase, green fluorescence protein (GFP), or aequorin and the like. As used herein, a method for introducing a gene per se may be achieved by means of desired material using known technology in the art. In such a case, for example, an embryonic stem cell of interest was introduced with a reporter gene free of a promoter (e.g., luciferase, green fluorescence gene, beta-galactosidase gene (lacZ), alkaline phosphatase gene, Cre recombinase gene and the like), and reporter activity will only be detected when inserted downstream of an activated promoter on the chromosome. Vectors used may include, for example, the presently mentioned reporter gene, selectable marker gene (e.g., neomycin resistant gene, hygromycin resistant gene, puromycin resistant gene, rescue marker gene (e.g., ampicillin resistant gene and collicin E1 replication origin) and the like. A selectable marker gene is used for selecting a host with the vector. A rescue marker gene is used for rescuing a vector (see Joyner, A. L. ed. "Gene Targeting,2nd edition"(Oxford University Press,2000)). Using technologies as described above, an embryonic stem cell is produced. The modified embryonic stem cell has trapped a gene. As used herein the term "trap" refers to the state where an internal gene is disrupted by insertion of a trapping vector into the genome, and the gene disrupted by the gene is marked at the same time.

Preparation of an oligonucleotide having a specific sequence may be achieved by any well known technology in the art that includes, but are not limited to: e.g. those described in Joyner, A. L. ed. "Gene Targeting,2nd edition"(Oxford University Press,2000). Oligonucleotides are labeled as necessary with a fluorescence, radiolabel and the like. Such labeling methods are well known in the art, and described in the references herein cited.

(Screening)

As used herein, the term "screening" refers to selection of a target, such as an organism, a substance, or the like, a given specific property of interest from a population containing a number of elements using a specific operation/ evaluation method. For screening, a method or system of the present invention may be used. In the present invention, as a variety of transgenic biological organisms are produced, any nucleic acid molecule and a functional regulation agent may be screened.

In the present invention, any nucleic acid molecules may be screened by means of a nucleic acid molecule, a method or a system of the present invention. The present invention is also intended to comprise chemicals identified by the screening or the combination thereof.

A transposon system according to the present invention may be used in a variety of fields. For example, 1) the present invention is used to efficiently insert genetic material into a chromosome of a biological organism; 2)a transposon is used as an insertion mutation agent to identify, isolate and characterize the genes relating to growth, maintenance, regulation and development of an organism (e.g. Kaiser et al., 1995 "Eukaryotic transposable, elements as tools to study gene structure and function" Mobile Genetic Elements, IRL Press,pp.69-100); 3), in which it is possible to identify, isolate and characterize the transcriptional regulatory factors relating to growth, maintenance, regulation and development of an organism (e.g., Anderson et al., 1996, Mol. Mar. Biol. Biotech., 5, 105-113). As an example, a method and system of the present invention may be used to produce a germ-free transgenic mouse. Litter-mates having an activated gene are crossbred to allow production of germ-free ascendants for biological containment or maximizing the growth rate.

(Genetic Therapy)

Use of the present invention includes incorporation of a gene for genetic therapy to a cell by modifying a nucleic acid fragment. Such a gene is located under the control of a tissue-specific promoter or universal promoter, or under the control of one or more other expression controlling regions for expression of a gene in a cell requiring the gene. Genes used for genetic therapy include but are not limited to, for example, CFTR gene for cystic fibrosis, alpha-1-antitrypsin for lung diseases, adenosinaminase (ADA) for immunological diseases, Factor IX and interleukin-2 (IL-2) for blood cell diseases, and tumor necrosis factor (TNF) for cancer treatment and the like.

Gene sequence possibly used for genetic therapy can be obtained by searching a known database such as GenBank, DDBJ, EMBL and the like.

Further, the present invention may be used for operating or screening a library or a part thereof, evaluating a function of a sequence, or screening for protein expression, evaluating effects of a particular protein or a particular expression controlling region on a particular cell type. In one embodiment, libraries of recombinant sequences, for example, those products of combinatorial library or gene shuffling can be incorporated in to the nucleic acid fragments of the present invention to produce a library of nucleic acid fragments having a variety of nucleic acid sequences located between certain inverted repeat sequences. Next, this library is introduced into a cell with a transposase such as the SB protein as described above.

BEST MODE FOR CARRYING OUT THE INVENTION

Hereinafter, preferred embodiments for carrying out the present invention are described. The embodiments provided below are only intended for better understanding of the present invention, and thus it should be understood that the scope of the present invention should not be limited to the description of the following section.

(LTR-type retrotransposon nucleic acid construct)

In one aspect, the present invention provides an isolated nucleic acid construct comprising a nucleic acid sequence encoding an LTR-type retrotransposon. Conventionally, an LTR-type retrotransposon is known to be related to genomic abnormality. However, conventionally, it was believed that an active type LTR-type transposon derived a different factor present at an unknown site on the genome is necessary. As such, it has not been shown that an LTR-type retrotransposon which has actually been isolated, can be used alone to apply to the modification of the genome, transposition of a gene, and introduction of a foreign gene, and the like. As such, the present invention shows unexpectedly significant effects in that the moiety alone can allow practicing the use thereof.

Any LTR-type retrotransposon may be used as long as the retrotransposon has an LTR, and may be constructed by using a method of gene engineering of a nucleic acid construct having such a sequence, that is well known in the art. It is understood that such a nucleic acid construct has a variety of utilities as described above including the modification of the genome and the like.

In a preferable embodiment, the above-mentioned LTR-type retrotransposon comprises Intracisternal A particle (IAP) type retrotransposon, early transposon (ETn), virus-like 30S RNA(VL30) retrotransposon and the like.

In a preferable embodiment, the above-mentioned retrotransposon comprises the full length IAP. Conventionally, it is proposed that there is a so-called full length IAP element. In the present invention, the full length IAP refers to an IAP element having actual activities of transcription, reverse transcription and insertion into the genome. Accordingly, amongst what is conventionally so called full length IAP elements may not fall within the full length IAP element as defined by the present invention. However, without using the nucleic acid construct of the present invention, it was not possible to confirm the activity of such a retrotransposon (in particular, LTR-type). Further, it should be understood that what is already known also encompass those which fall within the IAP sequence of the present invention, and that such sequence may be used for the purpose of the present invention.

In a preferred embodiment, the present retrotransposon encodes a functional polypeptide. A method for assaying whether an agent is functional or not, may be confirmed by investigating activities of transcription, reverse transcription and insertion into the genome, and exemplified hereinbelow in the Examples. Accordingly, it is understood that functions comprise at least one, preferably at least two, more preferably all selected from the group consisting of transcription, reverse transcription and integrase activities, for example.

In another preferred embodiment, the retrotransposon of the present invention comprises at least one sequence corresponding to LTR (in particular the R region), *gag, pol* and tRNA binding site (these sequences are also called "consensus sequence"). As used herein the consensus sequence is preferably a consensus sequence relating to the functionality relating to an IAP.

As used herein a retrotransposon (for example, IAP element) is preferably from an animal, and more preferably from a mammal, and still more preferably from a rodent or a primate, and most preferably from a mouse, but are not limited thereto.

In another preferred embodiment, the retrotransposon used in the present invention has, in its nucleic acid sequence, at least one feature selected from the group consisting of the features of repeating of the sequence tccgggacgagaaaa (SEQ ID NO: 31) at the tRNA binding site immediately downstream of the 5' LTR, and two or more repeat sequence consisting of ttgcttcttgctctc (SEQ ID NO: 32) of the R region. The subject common sequence encodes a functional IAP. More preferably, the IAP sequence used herein comprises (a) the repeat of the sequence TCCGGGACGAGAAAA (SEQ ID NO: 31) in the tRNA binding site immediately downstream of the 5' side, and (b) the number of repeats, as many as five, consisting of the R region TTGCTTCTTGCTCTC (SEQ ID NO: 32). Although not wishing to be bound by theory, it is because in the IAP of the present invention, sequence specific for leukemia cells such as Q14 are found to have tandem repeats of - TGGTGCCGAATTCCGGG- (SEQ ID NO: 33), a tRNA binding site, and thereafter -AATCCGGGACGAGAA (SEQ ID NO: 34). This is a site of binding of the first tRNA-Phe as a primer in the first place of the reverse transcription. The germ-line IAP element have less conserved repeat sequences, whereas a specific IAP element insertion site identified in a tumor are all conserved. Thus it is believed that there is possibility of affecting the reverse transcription in the first course of action. Although not wishing to be bound by theory, with respect to (b) above, the R region has a repeat sequence consisting of TTGCTTCTTGC (SEQ ID NO: 35), and such a large number of repeat functions as a intermolecular switch such that the initial reverse transcript initiates the second reverse transcription. Thus it is believed that it affects the intermediate course of action of the reverse transcription.

These are feature which have not been observed in IAP elements isolated in Balb/c systems such as MIA14 and the like. Therefore, it is believed that this may be one of the reasons why the present invention attains these functions for the first time, but it is not always essential.

In a particularly preferred embodiment, the retrotransposon used in the present invention may comprise:

(a) a polynucleotide having a base sequence set forth in SEQ ID NO: 1 or a fragment sequence thereof;
(b) a polynucleotide encoding a polypeptide consisting of an amino acid sequence set forth in SEQ ID NO: 2, or 3 and 4, or a fragment thereof;
(c) a polynucleotide encoding a variant polypeptide consisting of an amino acid sequence set forth in SEQ ID NO: 2, or 3 and 4 with at least one mutation selected from consisting of at least one amino acid substitution, addition and deletion, or a fragment thereof, which possesses a biological activity;
(d) a polynucleotide being a splice variant or allelic variant of the base sequence set forth in SEQ ID NO: 1, or a fragment thereof;
(e) a polynucleotide encoding a species homolog of a polypeptide consisting of an amino acid sequence set forth in SEQ ID NO: 2, or 3 and 4, or a fragment thereof;
(f) a polynucleotide which hybridizes to any of polynucleotides (a) through (e) or the complement thereof under stringent conditions, and encoding a polypeptide having a biological activity; or
(g) a polynucleotide having at least 70 % identity to any of polynucleotides (a) through (e) or the complement thereof under stringent conditions, and encoding a polypeptide having a biological activity.

More preferably, the retrotransposon of the present invention may comprise the sequence set forth in SEQ ID NO: 1. Alternatively, a nucleic acid molecule comprising a nucleic acid sequence encoding the retrotransposon of the present invention may comprise the sequence set forth in SEQ ID NO: 1.

In one preferred embodiment, the number of substitutions, additions and deletions described in (c) above may be limited to, for example, preferably 50 or less, 40 or less, 30 or less, 20 or less, 15 or less, 10 or less, 9 or less, 8 or less, 7 or less, 6 or less, 5 or less, 4 or less, 3 or less, or 2 or less. The number of substitutions, additions and deletions is preferably small, but may be large as long as the biological activity is maintained (preferably, having similar or substantially identical activities as that of retrotransposon comprising the amino acid sequence set forth in SEQ ID NO: 2 or 3 and 4.).

In another preferable embodiment, biological activity possessed by the present modified polypeptide includes, but is not limited to for example, interaction with an antibody specific for the polypeptide consisting of the amino acid sequence set forth in SEQ ID NO: 2 , 3 or 4, or a fragment thereof, maintenance of un-differentiated state, interaction with a extracellular matrix, and the like. Preferably, such biological activity includes un-differentiation maintenance. In order to determine the activity, gene introduction experimentation, gene deletion experimentation, RNAi experimentation, protein function inhibition experimentation using an antibody and the like.

In a preferable embodiment, an allelic gene mutant preferably has at least 90 % homology to the nucleic acid sequence set forth in SEQ ID NO: 1. In the same lineage, for example, such an allelic gene mutant preferably has at least 99 % homology.

When there is a gene sequence database for the species, the species homologs may be identified by conducting a search or query using, an amino acid sequence of the entire or a portion of the retrotransposon polypeptide comprising the amino acid sequence set forth in SEQ ID NO: 2 or 3 and 4 of the present invention, or the entire or a portion of the nucleic acid sequence of a nucleic acid molecule encoding the retrotransposon including the nucleic acid set forth in SEQ ID NO: 1. Alternatively, such a homolog may be identified by screening a gene library of the species, using the entire or a portion of the nucleic acid sequence of the retrotransposon of the present invention as a probe or primer. Such an identification method is well known in the art, and is described in the literature described herein. Species homologs have preferably at least about 30 % homology with the nucleic acid sequence set forth in SEQ ID NO: 1, for example. Moreover, species homologs more preferably have at least about 50 % with the nucleic acid sequence set forth in SEQ ID NO: 1.

In a preferred embodiment, the identity to any one of the polynucleotides described in (a) to (e) above or a complementary sequence thereof may be at least about 80% identity, more preferably at least about 90% identity, even more preferably at least about 98% identity, and most preferably at least about 99% identity.

In a preferred embodiment, the nucleic acid molecule of the present invention or fragments and variants thereof may have a length of at least 8 contiguous nucleotides. The appropriate nucleotide length of the nucleic acid molecule of the present invention may vary depending on the purpose of use of the present invention. More preferably, the nucleic acid molecule of the present invention may have a length of at least 10 contiguous nucleotides, even more preferably at least 15 contiguous nucleotides, and still even more preferably at least 20 contiguous nucleotides. These lower limits of the nucleotide length may be present between the above-specified numbers (e.g., 9, 11, 12, 13, 14, 16, and the like) or above the above-specified numbers (e.g., 21, 22, ... 30, and the like). The upper limit of the length of the polypeptide of the present invention may be greater than or equal to the full length of the sequence as set forth in SEQ ID NO. 1 as long as the polynucleotide can be used for the intended purpose (e.g. marker). Alternatively, when the nucleic acid molecule of the present invention is used as a primer, the nucleic acid molecule typically may have a nucleotide length of at least about 8, preferably a nucleotide length of about 10. When used as a probe, the nucleic acid molecule typically may have a nucleotide length of at least about 15, and preferably a nucleotide length about 17.

In more preferable embodiments, the present invention may be (a) a polynucleotide having a base sequence set forth in SEQ ID NO: 1 or a fragment sequence thereof; or (b) a polynucleotide encoding a polypeptide consisting of an amino acid sequence set forth in SEQ ID NO: 2, or 3 and 4, or a subsequent fragment thereof.

In certain preferable embodiments, the nucleic acid molecule of the present invention includes, but is not limited to, a nucleic acid sequence wherein at least one domain is selected from the group consisting of LTR, *gag, pol* and tRNA binding site, or has a position corresponding to at least one feature selected from the group consisting of the repeat of a sequence of TCCGGGACGAGAAAA in the tRNA binding site immediately located at LTR at the 5' side, and inclusion of two or more repeat sequences TTGCTTCTTGCTCTC in the R region.

In a preferred embodiment, the identity to any one of the polynucleotides described in (a) to (b) above, or a complementary sequence thereof may be at least about 80%, more preferably at least about 90%, even more preferably at least about 98%, and most preferably at least about 99%.

In another preferred embodiment, the nucleic acid molecule of the present invention encoding a retrotransposon or fragments and variants thereof may have a length of at least 8 contiguous nucleotides. The appropriate nucleotide length of the nucleic acid molecule of the present invention may vary depending on the purpose of use of the present invention. More preferably, the nucleic acid molecule of the present invention may have a length of at least 10 contiguous nucleotides, even more preferably at least 15 contiguous nucleotides, and still even more preferably at least 20 contiguous nucleotides. These lower limits of the nucleotide length may be present between the above-specified numbers (e.g., 9, 11, 12, 13, 14, 16, and the like) or above the aforementioned-specified numbers (e.g., 21, 22, ... 30, and the like). The upper limit of the length of the polypeptide of the present invention may be greater than or equal to the full length of the sequence as set forth in SEQ ID NO. 1 as long as the polynucleotide can be used for the intended purpose (e.g. antisense, RNAi, marker, primer, probe, capable of interacting with a given agent). Alternatively, when the nucleic acid molecule of the present invention is used as a primer, the nucleic acid molecule typically may have a nucleotide length of at least about 8, preferably a nucleotide length of about 10. When used as a probe, the nucleic acid molecule typically may have a nucleotide length of at least about 15, and preferably a nucleotide length about 17.

In a particularly preferable embodiment, the retrotransposon of the present invention comprises SEQ ID NO: 1 (sequence set forth in the species which were shown to be effective).

(Nucleic acid construction of an LTR-type retrotransposon - promoter containing format)

In a preferred embodiment, the nucleic acid construct of the present invention comprises a promoter sequence in addition to the retrotransposon sequence. Any promoter may be used, as long as the promoter allows transcription, reverse transcription and insertion into the genome of retrotransposons. Such a promoter may be prepared in an organic synthesis or a biological manner once the sequence information thereof is provided.

In preferable embodiments, the promoter sequence used in the present invention exhibits at least 0.1 rlu (relative light unit), which is a value obtained by dividing the value obtained when introducing the promoter located upstream of a luciferase into an animal cell (for example, HeLa cell) by the value obtained by using the CMV promoter. That is, it will be advantageous that the promoter has at least about 10 % of that of CMV promoter, preferably at least about 25 % thereof, more preferably at least about 50 % thereof, still more preferably at least about 80 % thereof, at least about 90 % thereof, at least about 95 % thereof, and still more preferably at least equal thereto to or greater. By having such potent promoter activity, it is first possible to observe if an IAP has integration activity or not.

Such a potent promoter includes, but is not limited to, for example, CMV promoter, CA promoter or the like. Specific sequences include but are not limited to, for example, at least one nucleic acid sequence(s) selected from the group consisting SEQ ID NOs: 5, 6 and 7, or a variant thereof including a mutation selected from the group consisting of one or more addition(s), deletion(s) and substitution(s) thereto. It is understood that such a variant is within the scope of the present invention as long as the variant has at least about 10 % activity of that of the CMV promoter.

In more preferable embodiments, the promoter sequence used in the present invention is replaced with a portion of 5' LTR in the retrotransposon. The replacement is preferably conducted so as to reserve the promoter activity thereof. Such a replaced sequence may be produced according to well known technology in the art.

In a preferable embodiment, the promoter sequence used in the present invention is replaced with the entire or a portion of the U3 region in the 5' LTR in the retrotransposon. Any type of replacement of the U3 region may be conducted as long as the promoter sequence achieves the promoter activity thereof (preferably, 0.1 rlu or greater). In preferable embodiments, the promoter sequence used in the nucleic acid construct of the present invention is operably linked to a retrotransposon. As used herein, whether or not operable linkage is achieved may be confirmed by finding whether or not the promoter activity is achieved, for example, the presence or absence of transcription, reverse transcription or integrase activities and the like.

In more preferable embodiments, the present promoter sequences have advantageously the transcription initiation site thereof located in frame with the transcriptional initiation site of the retrotransposon. These embodiments are particularly preferable, when using an IAP. Although not wishing to be bound by theory, it is preferable to have high activity for IAP to have an important transcription initiation site of the retrotransposon. As such, to promote more effective transposition, such a frame location is preferred. Frame locations may be selected by linking a promoter sequence and the transcription initiation site of the retrotransposon to be transcribed directly without any intervening sequences.

(Nucleic acid construct of an LTR-type retrotransposon - a nucleic acid construct for transposition of a foreign gene)

In a preferable embodiment, the nucleic acid construct further comprises a sequence for encoding a foreign gene. The nucleic acid encoding a foreign gene may encode any genetic product and may be located within any site therein, and preferably may be within the retrotransposon.

In preferable embodiments, the foreign gene renders a host distinguishable property. Such a distinguishable property includes, but is not limited to, PCR primers, antibiotic resistance, complement of nutrition, fluorescence, chemiluminescence, dyes and the like. Such specific foreign genes include, but are not limited to: *neo,* GFP, *hyg, puro, zeo, bsr, lacZ,* CFP, YFP, RFP, BFP and hrGFP.

In preferable embodiments, the foreign gene to be included in the nucleic acid construct of the present invention is composed such that the foreign gene is first expressed only after transcription, reverse transcription and insertion into the genome is subjected to. Those skilled in the art would readily understand how to construct such a construct, that includes, for example those in which a foreign gene is reversely located, or a method for intervening an intron sequence is also contemplated.

Accordingly, when using the nucleic acid construct of the present invention, the foreign gene preferably includes an intron sequence, but is not limited thereto. Any intron sequence may be used, and includes, for example, a sequence of an intron derived from human gamma globin may be used, but the present invention is not limited thereto.

In preferable embodiments, the intron sequences used in the nucleic acid construct of the present invention are advantageously located in a forward location in terms of the retrotransposon, and in a reverse location in terms of the foreign gene. Subsequently, the intron will be removed by transcription and splicing from the promoter of the retrotransposon, and the insertion into the genome thereafter will achieve the expression of the foreign gene for the first time.

In preferable embodiments, the intron sequences used in the nucleic acid construct of the present application are advantageously located *in trans* with respect to the retrotransposon. By locating the same *in trans,* the effects of the same promoter is less amenable, and thus allowing the expression of a foreign gene independent of the expression of a protein encoding the retrotransposon. It is also further possible to confirm regardless of the movement of the retrotransposon, whether or not a foreign gene is introduced, by locating the same *in trans.*

Preferably, the intron sequence is advantageously sandwiched between a splice donor sequence and a splice acceptor sequence. As used herein, the way of sandwiching the two is preferably in a manner such that the splice donor and the splice acceptor are operably linked to each other.

The nucleic acid construct of the present invention is useful for a variety of uses, and can be used for, for example, modifying a genome for confirming whether or not a retrotransposon has transposition activity, for transposing a foreign gene, for introducing a foreign gene into a host and the like. Such uses are specific and feasible or enabled.

The modification of a genome may be at a cell level or a biological organism/individual level. In order to achieve genomic modification at an individual level, it is necessary to produce a transgenic organism. Such a transgenic organism may be produced by modifying a cellular genome of a germ-line cell using the nucleic acid construct of the present invention, and producing a founder cell using the cell, and thereafter producing a transgenic organism using a method for producing a transgenic organism well known in the art.

Alternatively, it is necessary to confirm transposition activity of a retrotransposon (in particular, LTR-type) by confirming transcription, reverse transcription and insertion into a genome are achieved in a detectable level. Although not wishing to be bound by theory, conventionally at least one of promoter activity or LTR retrotransposon activity is a insufficient.system for confirming the transposition activity of the LTR retrotransposon. The present invention attains an unexpectedly significant effect where whether or not LTR retrotransposon is active by providing potent promoter activity sufficient for confirming at least the activity of LTR retrotransposon. From different point of view, this means that the activity of a functional LTR-type retrotransposon can be detected for the first time by the present invention, and such a functional LTR-type retrotransposon is firstly provided. These two elements have only been provided by the inventors who have unexpectedly found the successful combination by their efforts. Once the present invention is completed as disclosed herein, those skilled in the art would understand how to can carry out any equivalent embodiments based on the description of the present specification.

In another preferred embodiment, it is understood that the host to be targeted by the introduction of a foreign gene by the nucleic acid construct of the present invention, may be any organism, and preferably a eukaryote, and more preferably mammalian, and still more preferably rodent or primate, and most preferably of a mouse, however not limited thereto.

(Vectors, compositions and cells)

In another aspect, the present invention provides a vector comprising the nucleic acid construct of the present invention. The nucleic acid construct included in such a vector may employ any embodiment of any nucleic acid construct as described above. Such a vector may include an additional element in addition to the nucleic acid construct of the present invention. Such an additional element includes, but is not limited to, for example, a regulation sequence (for example, promoter, enhance, silencer, origin of replication and the like), restriction enzyme digestion sites, or intron sequences and the like.

In another aspect, the present invention provides a composition comprising the nucleic acid construct of the present invention, and a carrier as necessary. Such a composition may be pharmaceutical composition, agricultural composition and the like, but is not limited thereto. Such a carrier includes, but is not limited to: an antioxidant, preservative, colorant, seasoning, diluent, emulsifying agent, suspending agent, solvent, filler, extender, buffer, delivery vehicle, diluting agent, excipient, and/or agricultural or pharmaceutical adjuvant and the like.

In another aspect, the present invention provides a cell, tissue, organism or a portion thereof comprising the nucleic acid construct of the present invention. Such a cell may be any cell, and preferably it is advantageous to be germ-line cell, which allows production of transgenic organism, but is not limited thereto. Alternatively, the cell preferably is a cell suitable for allowing confirmation of the activity of retrotranposon. For example, such a cell includes, but is not limited to, for example, NIH3T3 cell, HeLa cell, F9 cell, embryonic stem cell (ES cell), and the like. Such a tissue may also be any tissue or a portion thereof. The above-mentioned organism may also be any biological organism or a portion thereof. It is understood that those skilled in the art should understand that the cell, tissue, organism or a portion thereof is readily produced and used, in view of the disclosure of the present specification.

(Method and Kit for modifying the genome)

In one aspect, the present invention provides a method for modifying a genome in a cell. The present method comprises the steps of: A) providing a nucleic acid construct comprising an LTR-type retrotransposon; B) introducing the nucleic acid construct into the cell; C) culturing the cell for a predetermined period of time; and D) selecting a cell with a genome modified by means of the nucleic acid construct. The present invention unexpectedly completed modification of the genome by using an LTR-type retrotransposon which has been conventionally considered impossible to control and thus cannot be used for genomic modification. Further, the present invention achieved such modification at an unexpectedly significantly higher rate than what has been reported to achieve the genomic modification efficiency achieved by non-LTR-type retrotransposon.

As used herein, the nucleic acid construct comprising the nucleic acid sequence encoding an LTR-type retrotransposon used in the genome modification method of the present invention, may employ any embodiment for genomic modification as described in the above sections (Nucleic acid construct of an LTR-type retrotransposon) described herein above in detail.

In a method of genome modification of the present invention, any well known technology in the art may be used for introducing a nucleic acid construct into a cell. A method for introducing a nucleic acid or a vector may employ any method for introducing a DNA into a cell, and includes, for example, transfection, transduction, transformation, and the like (for example, calcium phosphate method, liposome method, DEAE dextran method, electroporation method, methods using particle gun (gene gun) and the like), lipofection method, spheroplast method, lithium acetate method, and the like. Conditions for introduction of a nucleic acid construct or a vector may vary depending on the properties of the cell and substances used, and such variation is well known in the art. Those skilled in the art will be able to appropriately specify proper nucleic acid introduction conditions based on the conditions given. Introduction of a gene may also be confirmed using the methods described herein or other well known routine technologies in the art such as Northern blotting, Western blotting and the like. Preferably, transfection is used but is not limited thereto. When using transfection, gene introduction is preferably used. Examples of such a gene introduction reagent include, but are not limited to, cationic polymers, cationic lipids, polyamine-based reagents, polyimine-based reagents, calcium phosphate, and the like. Specific examples of a reagent used in transfection include reagents available from various sources, such as, without limitation, Effectene Transfection Reagent (cat. no. 301425, Qiagen, CA), TransFast™ Transfection Reagent (E2431, Promega, WI), Tfx™-20 Reagent (E2391, Promega, WI), SuperFect Transfection Reagent (301305, Qiagen, CA), PolyFect Transfection Reagent (301105, Qiagen, CA), LipofectAMINE 2000 Reagent (11668-019, Invitrogen corporation, CA), JetPEI (x4) conc. (101-30, Polyplus-transfection, France) and ExGen 500 (R0511, Fermentas Inc., MD), and the like.

In yet another preferable embodiment, introduction is conducted in the presence of cationic lipids, polyamine reagents and the like, and more specifically, it is conducted under the conditions where using cationic lipids or polyamine lipids, 1-4 micrograms of DNA is introduced into a well with cultured cell on a six-well plate.

In a genome modification method of the present invention, any culture methods well known in the art may be used as a technology for culturing a cell for a predetermined period of time. Such a culture method includes, but is not limited to for example, a method for culturing in an appropriate medium under conditions of an appropriate temperature and humidity (for example, 37 degrees Celcius, 100 %, $CO_2$ 5% and the like). Accordingly, the method for culture may be conducted according to a conventional method used in a culture of a host. Culture medium for culturing a transformant obtained using a prokaryotic organism such as *E. coli* and the like, or a prokaryotic organism such as yeast as a host, includes, for example, carbon source which can be assimilated by the organism of the present invention (for example, glucose, fructose, sucrose, sugar or honey containing the same, starch, starch hydrolysate, organic acids such as acetic acid and propionic acid, alcohols such as ethanol and propanol and the like), nitrogen source (for example, ammonia, a variety of ammonium salts of inorganic or organic acid salt such as ammonium chloride, ammonium sulfate, ammonium acetate, ammonium phosphate, other nitrongen containing substance and the like, peptin, meat extract, yeast extract, corn steep liquid, casein hydrolysate, soybean powder, soybean powder hydrolysate, a variety of fermented bacterial body, and the digests thereof and the like), inorganic salts (for example, potassium primary phosphate, potassium secondary phosphate, magnesium phosphate, magnesium sulfate, sodium chloride, ferrous phosphate, manganese sulfate, copper sulfate, calcium carbonate and the like), and the like, and any medium which allows efficient culture of the transformant may be used including natural medium, synthetic medium (for example, RMPI1640 medium [The Journal -of the American Medical Association,199,519(1967)], Eagle's MEM medium [Science,122,501(1952)] DMEM medium [Virology,8,396(1959)],199 medium [Proceedings of the Society for the Biological Medicine,73,1(1950)], or such a culture medium supplemented with fetal bovine serum or the like) or the like. Culture is preferably conducted under aerobic conditions such as shaking culture or deep aeration vortex culture or the like, but is not limited thereto. Culture temperature is preferably from 15-40 degree Celsius. The period of time for culture is usually from five hours to seven days but is not limited thereto. The pH during the culture is kept from 3.0 to 9.0. The adjustment of the pH may be conducted by adding inorganic or organic acid or alkaline solution, urea, calcium carbonate, ammonia and the like. During the culture, antibiotics such as amphicillin or tetracycline or the like may be added as necessary.

In a genome modification method of the present invention, any methods well known and used in the art may be used as a technology for selecting a cell with the genome thereof modified by means of a nucleic acid construct of the present invention. Selection may preferably be conducted based on the phenotype of the host cell, which varies depending on the expression of the nucleic acid to be introduced. For example, when the nucleic acid introduced encodes a growth factor, the desired functional property is the particular cell or promotion of the growth of any cell. Moreover, if the nucleic acid introduced encodes an antibiotic resistance factor, the selection may be conducted by culturing the cell in the present of the antibiotic.

In a preferred embodiment, it is advantageous that the nucleic acid construct used in the method of genome modification of the present invention further comprises a promoter having activity of 0.1 rlu or greater as determined by luciferase assay in vitro, and that the predetermined period of time for culture is sufficient to allow transcription, reverse transcription and insertion into the genome. This is because such modification of the genome requires the transcription, reverse transcription and insertion into the genome to be achieved. Sufficient conditions for the transcription, reverse transcription and insertion into the genome may be arbitrarily determined by those skilled in the art. Such a period may be several hours (2-3 hours) to several days (5-10 days), preferably 3-7 days (for example, about 72 hours), and most preferably, about five days (about 120 hours).

Preferably, the promoter sequence used in the method of genome modification of the present invention is advantageously placed in frame with the transcription initiation site of the retrotransposon. Although not wishing to be bound by a theory, such an in frame location significantly enhances the transcription initiation from the site having no effects from the reverse transcription. As a result, the transcription activity is thus significantly enhanced.

The nucleic acid construct used in the method of genome modification of the present invention comprises a foreign gene operably located in the retrotransposon, and the selection is conducted by means of the expression of the foreign gene. Any foreign gene may be used as such a foreign gene as described in the above description (Nucleic Acid Construct for Transposition of Foreign Gene). The technology used for the selection, may appropriately be selected according to the foreign gene used.

In a preferred embodiment, the foreign gene used in the method of genome modification of the present invention is placed in a reverse direction with respect to the retrotransposon, and comprises a splice donor sequence and a splice acceptor sequence, and an intron located therebetween in a *cis* direction. The predetermined period of time is sufficient for conducting transcription, reverse transcription and insertion in to the genome. The selection is conducted according to the expression of the foreign gene. The reverse location may be conducted by any means well known in the art. Specifically, after confirming the transcription direction of a foreign gene or an expression cassette comprising the same, an appropriate restriction enzyme site was used to link the retrotransposon construct to an appropriate site thereof. Furthermore, a splice donor sequence and a splice acceptor sequence are well known in the art, and those skilled in the art can use any sequence herein. Such sequences includes, but is not limited to, for example, GTRAGT (R refers to purin), preferably GTAAGT for splice donor sequence, and for example, $(Y)_n$NCAG (n>11, N refers to any base), preferably $(T/C)_{15}$ACAG and the like.

In a preferred embodiment, the foreign gene used in the present invention encodes an agent selected from the group consisting of an antibiotic resistance gene, nutrition complement factor, enzyme gene, and fluorophore-coding gene (for example, *neo, hyg, puro, zeo, bsr, hisD* and the like). The selection is conducted according to the property of the cell expressing the agent. A Nutrition complement factor may be selected by the presence or absence of the corresponding nutrition. Antibiotic resistance genes may be selected according to the presence or absence of the corresponding antibiotic. Such a nutrition factor (for example, particular amino acid, vitamin or the like) or antibiotic factor (for example, neomycin, kanamycin, hygromycin, or the like), may be used in the vicinity of the lower limit of the effective concentration, and may be used at much higher concentration than the effective concentration, and as such the concentration is not limited.

In a preferred embodiment, an LTR retrotransposon used in the method for modifying a genome of the present invention comprises a sequence of an IAP element. The sequence of such an IAP element is desirably that of the full length IAP element, and thus preferably functional. IAP comprises *gap* and *pol. gag* and *pol* encode necessary group of enzymes for structural proteins and transposition of the virus protein, respectively. Accordingly, the sequence of the IAP element of the present invention is preferably to encode the full structural protein and the full reverse transcriptase groups for a method for modifying the genome.

In a certain embodiment of the method for modifying the genome of the present invention, selection may be conducted by confirming the sequence transposed by means of a ligation mediated PCR.

The cell targeted by the method for modifying the genome of the present invention may be any cell, and usually a cell from a prokaryote, yeast, animal, plant, insect and the like, and preferably a eukaryotic cell, more preferably a cell from a mammal, and still more preferably from a rodent (for example, mouse, rat and the like), or primate (human, gorilla, chimpanzee, apes or monkey and the like) are used in an advantageous manner.

Any cell may be used as a cell of the present invention, preferably includes a eukaryotic cell, more preferably a mammalian cell, more preferably, a rodent cell, but are not limited thereto. More preferably, it is useful to use a cell from a model animal such as a mouse, rat and the like. The cell of the present invention should be determined in relation to the property of the nucleic acid molecule to be introduced or purpose thereof for the host to which the nucleic acid molecule is to be introduced. The nucleic acid molecule to be included in the cell of the present invention may be the vector of the present invention.

The tissue of the present invention may be any type of tissue, and preferably includes a eukaryotic tissue, more preferably includes a mammalian tissue, still more preferably includes a rodent tissue, but is not limited thereto. More preferably, those of a model animal such as a mouse, or rat is useful. The tissue of the present invention should be determined in relation to the property of the nucleic acid molecule to be introduced, purpose thereof, the host to which the nucleic acid molecule is to be introduced. The nucleic acid molecule to be included in the tissue of the present invention may be the vector of the present invention.

The biological organism of the present invention may be of any type of biological organism, and preferably includes a eukaryotic biological organism, and more preferably includes a mammalian biological organism, and still more preferably, a rodent biological organism, but are not limited thereto. More preferably, those of a model animal such as a mouse, a rat model is useful. The organism of the present invention should be determined in relation to the property of the nucleic acid molecule to be introduced, purpose thereof, the host to which the nucleic acid molecule is to be introduced. The nucleic acid molecule to be included in the organism of the present invention may be the vector of the present invention.

The retrotransposon used in the method of the genome modification of the present invention may be of any cell, and usually includes any retrotransposon derived from a cell of a prokaryote, yeast, animal, plant, insect and the like, preferably from a eukaryotic cell, more preferably from a mammalian cell, and still more preferably from rodent (for example, mouse, rat and the like), or primate (for example, human, gorilla, chimpanzee, ape or monkey), which may be advantageous.

In certain embodiments, the retrotransposon used in the method of genome modification of the present invention (the natural host thereof) may be of the same or different species with respect to the targeted cell, and preferably from the same species, but is not limited thereto. Such a combination includes, but is not limited to a transposon from a mouse, a mouse cell (of the same species), a retrotransposon from a mouse, and a human cell (different species).

In another aspect, the present invention provides a kit for modifying the genome inside a cell. Such a kit comprises A) a nucleic acid molecule comprising a nucleic acid sequence encoding an LTR-type retrotransposon; B) means for introducing the nucleic acid construct into a cell; and C) means for selecting a cell the genome of which has been modified by means of the nucleic acid construct.

As used herein, any type of construct as described herein above sections (Nucleic acid construct of an LTR-type retrotransposon), may be used as the nucleic acid construct comprising a nucleic acid sequence encoding an LTR-type retrotransposon.

Any type of means for introducing a nucleic acid construct into a cell may be used, and for example, use of transfection reagent is preferable. The transfection reagent is selected from the group consisting of cationic macromolecules, cationic lipids, polyamine-based reagents, polyimine-based reagents and calcium phosphate. Specific examples of a reagent used in transfection include reagents available from various sources, such as, without limitation, Effectene Transfection Reagent (cat. no. 301425, Qiagen, CA), TransFast™ Transfection Reagent (E2431, Promega, WI), Tfx™-20 Reagent (E2391, Promega, WI), SuperFect. Transfection Reagent (301305, Qiagen, CA), PolyFect Transfection Reagent (301105, Qiagen, CA), LipofectAMINE 2000 Reagent (11668-019, Invitrogen corporation, CA), JetPEI (x4) conc. (101-30, Polyplus-transfection, France) and ExGen 500 (R0511, Fermentas Inc., MD), and the like.

In a preferred embodiment, means for selection may be any means for detecting the expression of the foreign gene (for example, in the case of antibiotic resistance, the antibiotic *per se,* in the case of complementarity of nutrition, the nutrition per se, and the like), or means for conducting PCR (for example, PCR primers for nested PCR with a sequence encoding the foreign gene, and that encoding a retrotransposon), and the like, but are not limited thereto.

It is preferable to attach a set of instructions describing experimental protocol, if necessary. Alternatively, a kit of the present invention may comprise these instructions describing a method of using the nucleic acid molecules and retrotransposon. The instructions may be of paper medium, and may be transfer medium (for example, information on a network). The instructions describe a variety of protocols relating to transgenic organism such as manipulation of nucleic acid molecules, transformation, culture, regeneration, incubation of transposon and the like. The description may be monolingual, but more than one languages may be accompanied thereto.

(Assay for transposition activity of a retrotransposon and the kits used therefor)

In another aspect, the present invention provides a method for assaying transposition activity of a retrotransposon. The present method comprises the steps of A) providing a nucleic acid construct comprising a nucleic acid sequence encoding a retrotransposon to be assayed, and a promoter sequence having activity of at least 0.1 rlu as determined by a luciferase assay *in vitro;* B) introducing the nucleic acid construct into the cell; C) culturing the cell for a predetermined period of time; and D) detecting the transposition by means of nucleic acid construct. The present invention uses an LTR-type retrotransposon, which was conventionally believed not to be possible to control, and thus it was not possible to observe transposition activity. The present invention achieved the unexpectedly significant effects thereof by incorporating the LTR-type retrotransposon under a particular promoter sequence into a cell to allow observation of the transposition activity thereof.

The nucleic acid construct comprising a nucleic acid sequence encoding a retrotransposon, which can be used in the assay of transposition activity of the retrotransposon of the present invention, can be used for any form of genomic modification, as described hereinabove in detail in the above sections (A nucleic acid structure of an LTR-type retrotransposon).

In an assay of transposition activity of the retrotransposon of the present application, any well known technology in the art may be used as a technology for introducing a nucleic acid construct into a cell. Such introduction technology of a nucleic acid construct have been described in detail in sections (method for modifying the genome) described herein.

The detection of transposition activity is preferably the use of ligation mediated PCR, because transposition activity can be digitized. In the case of using ligation mediated PCR in the present invention, the following procedures can be used:

An appropriate amount of genomic DNA is cleaved with a restriction enzyme such as EcoRV, HincII, MscI, ScaI and SmaI and the like, and the enzymes are deactivated by heat treatment, and an appropriated linker DNA is linked. Using the genomic DNA fragment with the linker linked thereto as a template, nested PCR is conducted using a primer specific for the linker and a primer specific for the neo cassette inside the target (IAP and the like). The primers used in the first round of PCR are a combination of a linker specific primer and a foreign gene (*neo* or the like) cassette specific primer. The primers used in the second round of PCR are a combination of linker specific primers and a foreign gene (*neo* or the like) cassette specific primer or foreign gene (*neo* or the like) cassette specific primers.

Conditions for the PCR may be appropriately determined. For example, for the first and second rounds, 94°C x five minutes, 94°C x 1 minute - 55°C x 1 minute - 68 ° C x two minutes) x 30 cycles, 68°C x seven minutes can be used for both. For example, Expand HiFi PCR system (Roche) may be used. Base sequence of the amplified band may be analyzed using a sequencer such as ABI PRISM 3100 or the like, and it can determine the site on the genome and genes present therein by means of databases such as Ensembl (http://www.ensembl.org/) and the like.

The detection of transposition activity by means of the retrotransposon of the present invention as described hereinabove, sequences obtained by the comparison between the ligation mediated PCR and a genomic database. Such a genomic database includes, other databases such as GenBank, DDBI and the like, in addition to Ensembl as described above. Comparison can be done using any tool, and tools attached to Ensembl, for example, can be used.

In another aspect, the present invention provides a kit for assaying transposition activity of a retrotransposon. The present kit comprises A) a nucleic acid construct comprising a nucleic acid sequence encoding a LTR-type retrotransposon, and a promoter having an activity of 0.1 rlu or greater as determined by a luciferase assay *in vitro;* B) means for introducing the nucleic acid construct into the cell; and C) means for detecting transposition by the nucleic acid construct. As used herein, any embodiments of individual nucleic acid construct, means for introduction and means for detection of transposition can be used as described in "a method for modifying the genome". The Kit can optionally include instructions describing experimental protocols, which is preferable.

In a preferable embodiment, means for selection is a means for conducting PCR, and such a means may include, but is not limited to, a sequence encoding a foreign gene, and a sequence encoding a retrotransposon, and PCR primers for nested PCR.

(Methods and Kits for producing a transgenic biological organism)

In another aspect, the present invention provides a method for producing a transgenic organism. The present method comprises the steps of A) providing a nucleic acid construct comprising a nucleic acid sequence encoding a LTR-type retrotransposon; B) introducing the nucleic acid construct into a germ-line cell of a desired biological organism; C) selecting a germ-line cell with the genome thereof modified in the germ-line cell; and D) regenerating the germ-line cell with the genome thereof modified into a biological organism.

A nucleic acid construct comprising a nucleic acid sequence encoding an LTR-type retrotransposon may be any type as described in the section herein above. Introduction of a nucleic acid molecule into a germ-line cell of a desired organism can also be achieved by well known technology in the art (for example, gene recombination technology described herein). Any method of the gene introduction technology described hereinabove may be employed as a means. Selection can also be conducted by means of any selection technology known in the art as described above, and such technology may vary depending on the nucleic acid construct to be introduced.

Regeneration of an organism using a transformed germ-line cell can also be achieved by any means and those skilled in the art can appropriately select an appropriate method depending on the organism to be used.

A mammal with the genome thereof modified can be produced using a positive negative selection method using homologous recombination, for example (United States Patent Nos. 5,464,764, 5,487,992, and 5,627,059 publications, Proc. Natl. Acad. Sci. USA, vol. 86, 8932-8935, 1989, Nature, Vol. 342, 435-438, 1989 and the like). Review of gene targeting is described in, for example, Masami MURAMATSU, Masa YAMAMOTO ed. "Experimental Medicine, Suppl., new edition, gene engineering handbook, revised version III" (1999, published by Yodo-sha, in particular pages 239-256), Shin'ichi AIZAWA (1995), Experimental Medicine, Suppl. "Gene Targeting - production of mutant mice using ES cells" and the like, which can all be used herein.

In a higher organism, for example, efficient selection of recombinants by means of positive selection using a neomycin resistant gene, and negative selection using HSV thymidine kinase gene or diphteria toxin gene. For example, homologous recombinants are conducted using knockout PCR or Southern blot method. That is, a part of a target gene is replaced with a neomycin resistant gene or the like, and a targeting vector is produced with linking at its end to HSVTK gene for negative selection, which is introduced into an ES cell by means of electroporation. Selection is achieved in the presence of G418 and gancyclovir to isolate resultant colonies to select homologous recombination by means of PCR or Southern blotting.

As such, a method for producing a mouse with a genomic modification (targeted gene recombination or gene disruption) having a mutation with modified functions, is useful for analysis of gene functions as only targeted genes have been introduced with the mutations.

After selecting a desired homologous recombinant, resultant recombinant ES cell is mixed with a normal embryo by means of blastocyst injection method or aggregation chimeric method to produce a chimeric mouse with ES cell and host embryo. In the blastocyst injection method, an ES cell is injected into a blastocyst by means of a glass pipette. In a aggregation chimeric method, aggregated ES cells and an embryo of eight-cell period with the zone pelucida removed therefrom are adhered to each other. The blastocyst with the ES cell introduced thereinto is implanted into the uterus of the host mother which has been pseudopregnant. ES cells have totipotency, and thus can be differentiated into any type of cell including germ-line cells. A chimeric mouse having the germ-line cell from the ES cell is crossbred with a normal mouse to obtain a mouse having the chromosome of the ES cell in a heterogenous manner. These crossbred mice are crossbred to each other to obtain a knowout mouse having the modified chromosome of the ES cell in a homozygous manner. In order to obtain a knockout mouse having the mutated chromosome in a homozygous manner from the resultant chimeric mouse, a male chimeric mouse and a female chimeric mouse are crossbred to produce a heterozygous mouse of the F1 generation, and the obtained male and female heterozygous mice are crossbred to produce and select a homozygous mouse of the F2 generation. In each of the F1 and F2 generations, whether or not a desired gene mutation has been introduced, may be analyzed by means of conventional methods well known and routine in the art such as Southern blotting, PCR, sequencing of the base sequence as in assays for the recombinant ES cell, and the like.

In a preferable embodiment, the transgenic organism of the present invention is a eukaryotic organism. This is because effects of retrotransposon attained by the present invention may be more efficiently achieved.

In a preferable embodiment, the organism targeted by the transgenic organism of the present invention includes a mammal. This is because effects of retrotransposon attained by the present invention may be more efficiently achieved. More preferably, the mammal is a rodent, and more preferably model animals such as a mouse or rat and the like.

In another aspect, the present invention provides a kit for producing a transgenic organism. The present kit comprises A) a nucleic acid construct comprising a nucleic acid sequence encoding an LTR-type retrotransposon; B) means for introducing the nucleic acid construct into a germ-line cell of a desired organism; C) means for selecting a germ-line cell with the genome thereof modified in the germ-line cell; and D) means for regenerating the germ-line with the genome thereof modified into an organism.

As used herein, a nucleic acid construct comprising a nucleic acid sequence encoding an LTR-type retrotransposon, may be any type as described above in the Sections (A nucleic acid construct of an LTR-type retrotransposon) herein above.

As means for introducing a nucleic acid construct into a germ-line cell, any technology appropriate for germ-line cells can be used amongst those described in the above described

(METHOD FOR GENOME MODIFICATION).

With respect to selection and regeneration, any means for such methods can be used as described herein in the present Section. Means for regeneration include a biological body or organism which can be a host. As such a host, any organism such as a mouse may be, which can be pseudopregnant state.

(Novel promoters)

The present invention further provides a novel promoter. The present promoters include cytomegalovirus enhancer and avian beta-actin promoter, wherein at least one of the cytomegalovirus enhance and the avian beta-actin promoter comprises a sequence shorter than the native full-length sequence thereof. Promoters having such shorter sequence than the native full-length sequence thereof are also called the CA promoter. Conventionally, it has been believed that no transcription activity can be achieved without the full length of the CAP promoter sequence. As such, it should be noted that a portion of the sequence achieved an activity substantially comparable to the CAG promoter by a partial sequence thereof.

In preferable embodiments, the shorter sequence in the present invention is due to the deletion of a sequence downstream of the transcription initiation site. Conventionally, it has not been evident that sequences after the transcription initiation site are unnecessary, and thus it can be recognized that the present invention provides a novel promoter sequence.

In a preferable embodiment, in the promoters of the present invention, all the sequence down stream of the transcription initiation site is deleted. It was unexpected that such sequences can be demonstrated to have promoter activity as potent as those before such deletion. Furthermore, absence of downstream of the transcription initiation sites, has provided a promoter sequence which can be used for direct linkage to a transcription initiation site. Such a promoter sequence has not been known to have potent promoter activity, for example, those which allow observation of retrotransposon transposition activity. As such, novel promoters of the present invention have achieved significant effects which cannot be achieved by conventional promoters.

In another embodiment, in the promoter of the present invention, a portion of a sequence downstream of the transcription initiation site and the promoter region is removed. In addition to the transcription initiation site, it was found that promoter activity is maintained even if a portion of the promoter region (for example, one, two or three base(s) or the like upstream of the transcription initiation site) is deleted. Accordingly, in such a case, the present invention is useful for the elements in which a portion of a promoter sequence is necessary, in particular.

The promoter sequence comprises a sequence set forth in SEQ ID NO: 36 as a cytomegalovirus enhancer, for example, in a specific manner. Further, the avian beta-actin promoter includes the sequence set forth in SEQ ID NO: 8.

Novel promoters of the present invention include, but are not limited to, those set forth in SEQ ID NO: 5, 6, or 7 and the like. Most preferably, the present invention comprises the sequence set forth in SEQ ID NO: 7 (without R region).

In another embodiment, the novel promoter of the present invention comprises the sequence set forth in SEQ ID NO: 6 (without R region and further deletion of a part of the promoter region).

(Variety of uses of an LTR-type retrotransposon)

In another aspect, the present invention provides use of an LTR-type retrotransposon for genomic modification. A variety of embodiments for an LTR-type retrotransposon used in the modification of the genome are described elsewhere herein in detail.

In another aspect, the present invention provides use of a promoter having an activity of 0.1 rlu or greater as determined by a luciferase assay *in vitro*, for modification of a genome. A variety of promoter embodiments for use in modification of the genome is described elsewhere herein in detail.

In another aspect, the present invention provides use of a promoter having an activity of 0.1 rlu or greater as determined by a luciferase assay *in vitro*, for confirmation of an LTR-type retrotransposon. A variety of promoter embodiments for use in modification of the genome is described elsewhere herein in detail.

The present invention achieves unexpected effects in comparison of the prior art in that exhaustive genome modification can be achieved in a simple manner, which had been impossible or even if it was possible with low efficiency.

References such as scientific literature, patents, patent applications and the like will be incorporated herein by reference as if the entirety thereof is specifically described herein.

Hereinafter, the present invention is described based on the Examples. The following Examples are provided only for the purpose of illustration. Accordingly, the scope of the claims of the present invention is not limited to the description described above or following examples, but only by the appended claims.

EXAMPLES

Hereinafter the present invention is described by way of examples in detail, but the present invention is not limited to the following examples. Reagents used herein below in the Examples, are obtained from Sigma (St. Louis, USA), Wako Pure Chemical (Osaka, Japan) and the like unless otherwise stated. Handling of animals has been conducted under the provisions defined in Osaka University, Medical School. The method for producing an expression vector used in the present invention will be described in with specific examples. It will be readily conducted for those skilled in the art to replace elements, such as these start plasmid, promoters and the like with equivalents thereof.

The scope of the claims of the present invention is not limited to the description described above or following examples, but only by the appended claims.

(EXAMPLE 1: Construction of IAP)

1. Production of IAP vector

(a) Isolation of the full-length IAP sequence from the genome

Amongst the leukemia cells induced by radioactive radiation to C3H/He mice, cells which have been observed to have transposition of the IAP, which had been believed to be full length amongst the base sequences (8065-AML cells, Ishihara & Tanaka, FEBS Lett. 418, 205-209, 1997) were used to isolate the IAP by means of PCR. Firstly, outside the genomic region of the IAP sequence, the following two primers were selected: 5'-GCAGCGGCCGCCGTGGTGGCACACACTTTTAGTCCCCGCAG-3' (SEQ ID NO: 9) and 5' - GGCGCACTAGTGATGCCCTCTCAGGCCTCCACTCAGGCACT-3' (SEQ ID NO: 10). Each has introduced NotI and SpeI, restriction enzyme sites which are not present in the PCR products, at the 5' terminus thereof. Conditions of PCR are as follows: 94°C x two minutes, (94°C x 15 seconds - 65°C x 30 seconds - 68°C x six minutes) for ten cycles, (4°C x 15 seconds - 65°C x 30 seconds - 68°C x (six minutes + five seconds/cycle)) for 20 cycles, and 72°C x seven minutes. Expand HiFi PCR system (Roche) was used.

The amplified bands were cleaved with NotI and SpeI, and have been cloned to NotI-SpeI sites of pBluescript II KS+ vector. Ten clones obtained were sequence for determining base sequences using ABI PRISM 3100 (Applied Biosystems), to compare the results of direct sequencing before cloning, each of 10 clones had base sequence substitution by means of PCR. Clones recognizing base sequence substitutions only within 2.5 kb BstEII-XbaI region only in the center of IAP, have been selected and subsequently conducted PCR using the following methods using a PCR product 2.5kb Bst-EII-XbaI region using Pfx polymerase having high fidelity, has been replaced. First, the above mentioned L8065-AML cell genome was used as a template to conduct PCR using the following primers: 5'-ATGCCCAGATTTCTTCCACGGCTATTAGGG-3' (SEQ ID NO: 11) and 5'-GATGCCCTCTCAGGCCTCCACTCAGGCACT-3' (SEQ ID NO: 12). Conditions of PCR as follows: 94°C x two minutes, (94°C x 15 seconds - 65°C x 30 seconds - 68°C x five minutes) for twenty cycles, and thereafter 68°C x two minutes, and Zero Blunt TOPO PCR cloning kit (invitrogen) has been used for cloning. Clones with no base sequence replacement introduced into the 2.5 kb BstEII-XbaI region have been identified, replaced with the same region as the above-mentioned clone to obtain a full length IAP vector, and the vector has been designated as pU3gp.

(b) Insertion of a neo cassette into the IAP vector

A cassette in which introns of gamma globin are inserted inside of the *neo* gene in a reverse direction against the *neo* gene, was isolated as an ApaLI-AccI fragment of pJM101/L1.3(Kimberland et al. Hum Mol Genet 8; 1557-1560, 1999), and inserted into the NdeI recognition site located downstream of the pol gene of the above pU3gp. Thereupon, a clone in which the introns of gamma globin are located in a forward direction in terms of pU3gp, was selected and designated as pU3gp-neo (Figure **4A**).

c) Replacement of the U3 region of 5' terminus with CMV promoter

PCR was conducted using the following primers using the CMV promoter derived from pcDNA (Invitrogen) as a template: hCMV-U3: 5'-CCAAGCGGCCGCTGGCCATTGCATACGTTGTATCCATATC-3' (SEQ ID NO: 13); hCMV-L3: 5'-GCGAGAAAAACGGTTCACTAAACGAGCTCTGCTTATATAG-3' (SEQ ID NO: 14). About 0.3 kb from the 5' terminus of the R region of the IAP to downstream of the U5 region thereof was amplified using the following PCR primers: R-U1: 5'-TTAGTGAACCGTTTTTCTCGCTCTCTTGCT-3' (SEQ ID NO: 15); R-L1: 5'-TCTGAAATGAAGTATCCCTCCTGCGCCAGT-3' (SEQ ID NO: 16). Both PCR used Pfx polymerase using the following PCR conditions: 94°C x two minutes, (94°C x 15 seconds - 55°C x 30 seconds - 68°C x one minutes) for twenty cycles, and thereafter 68°C x two minutes. The hCMV-L3 and the 5' side of R-U1 have complementary sequence to each other, and when conducing PCR using the mixture of both as a template using hCMV-U3 and R-L1 as primers, fusion product with the CMV promoter and the R region was obtained as a PCR product. The PCR conditions used therein were as follows: 94°C x two minutes, (94°C x 15 seconds - 55°C x 30 seconds - 68°C x one minute) for fifteen cycles, and thereafter 68°C x two minutes. The PCR product has been cloned by means of Zero Blunt TOPO PCR cloning kit (invitrogen) and clones with no base sequence replacement were identified by PCR. The NotI-BstEI fragment of the subject clone includes CMV-promoter region - the R region - the U5 region, and thus was replaced with the NotI-BstEI region of the above-described pU3gp and pU3gp-neo, to obtain a vector of the structure with the U3 region of the 5' side replaced with the CMV promoter, and designated as pCMVgp and pCMVgp-neo (Figure **4A**), respectively.

(d) The production of the IAP vector associated with deletion of pol or gag-pol (Figure **5A**)

A vector was produced in which the portion from BglII recognition site in the pol gene region of the IAP to the NdeI recognition site downstream of the pol gene have been deleted from the pCMVgp-neo, and designated as pCMVgp-neo-d1 (Figure **5A**). Similarly, another vector was produced in which the portion from the BstEII recognition site to the NdeI recognition site downstream of the pol gene has been deleted, and designated as pCMVgp-neo-d2 (Figure **5A**).

(RESULTS)

Schemes of production of the vectors of the present Example are shown in Figures **4** and **5**. Figure **4A** depicts the structure of a vector used in Example 1. pU3gp-neo is one in which a neo-cassette for detection of transposition was inserted into the IAP element from murine leukemia cell, which is expected to be full length. pCMVgp-neo is replaced the promoter region (U3 region) of IAP element with CMV promoter.

The juncture of the CMV promoter and the R region is shown in Figure **4B.** As described in Figure **4B,** the subject Example is constructed so as to coincide the transcription initiation site of the CMV promoter with the original transcription initiation site of the IAP (i.e. the 5' terminal moiety of the R region).

Figure **5A** shows the structure of a variety of vectors produced in the Examples. It shows that pCMVgp-neo is shown as a comparison target with respect to the same as described in Figure **4(A).** On the other hand, pCMVgp-neo-d1 and pCMVgp-neo-d2 have deletion in the 3' side from the BglII cleavage site of the *pol* gene, and the 3' side from the BstEII cleavage site of the gag gene. As shown, pCMVgp is a full length IAP having no neo cassette, and has the U3 region replaced with the CMV promoter, and thus expresses *gag-pol.*

This vector was used in the following

Examples.

(EXAMPLE 2: Transfection (introduction of a vector into a cell) and Drug Selection)

One day prior to transfection, 250,000 cells were plated in a six-well culture plate. Transfection was achieved by using 1.5 $\mu$g DNA using Effectene (QIAGEN) against a NIH 3T3 cell, and 4 $\mu$g of DNA in a HeLa cell, using LipofectAMINE (Invitrogen). Selection by means of G418, was initiated after 4-7 day passage of a cell after the transfection. The concentration of G418 used are 500 $\mu$g/ml against NIH 3T3, and 600 $\mu$g against HeLa. 12-14 days after the initiation, the number of G418 colonies have been counted. Fluorescent by means of GFP achieved detection under microscope after post-three day after the transfection.

(RESULTS)

The results of the Examples are shown in Figures **3C, 4** and **5.**

Figure **4C** depicts the principle of detection of transposition. Transcription and splicing result in the reconstitution of the neo gene by deletion of introns in the neo cassette. At this stage, the transcription and the neo gene are in a reverse direction, and thus the *neo* gene does not express the *neo* gene. Therefore, when the transcription and the insertion into the genome occur, then transcription occurs from the promoter possessed by the *neo* gene, thereby the neo gene causes expression rendering the cell G418 resistant, and thus the occurrence of transposition can be determined.

Examples of the results are shown in Figure **3C**. Figure 3C shows the appearance frequency of G418 resistant colonies. NIH3T3 cells were transfected with a vector and after four days, G418 selection was initiated. Twelve days after, staining was conducted. In IAP(i), a number of colonies have appeared, whereas (ii) the vector having mutation introduced into the *gag-pol* region, no colonies have been observed. Accordingly, the IAP used in the present Example has been demonstrated to have caused transposition by means of *Gag-Pol* which is encoded by itself. As a control, (iii) non-LTR type LINE1 having the neo cassette including the same intron which is transcribed by the CMV promoter in the same manner as in the IAP (gift from John Moran, Cell 110, 315, 2002) was used to demonstrate that the activity of the IAP used in the present Example, as the IAP made greater number of G418 resistant colonies than the LINE1. The present results are described from different point of view as follows:

Figure **4D** shows the results of the detection of transposition by means of transfection into the NIH 3T3 cells as the number of G418 resistant colonies derived from 5 x 10$^5$ cells, by means of the Examples. pJM101/L1.3 is a vector using LINE1, which is a different retrotransposon. As seen from the results shown herein, in the case of using the U3 region of the original IAP promoter, no G418 resistant colonies did not appear, whereas when using the CMV promoter, a number of colonies have been obtained, and thus the effects of the modification of the promoter has been elucidated. Furthermore, modified-type IAP vector has been turned out to have transposition ability as potent as or greater than the LINE1 vector.

Figure **5** shows that it can prove that the IAP which may be used in the present invention, has a full transcription ability, and that it can prove that separation of an expression unit of *gag-pol* allows control of transposition.

Figure **5B** shows assay of the activity of each vector by means of transfection into HeLa of the present Example. The colony number of G418 resistant colonies derived from 5x10$^5$ cells is shown. A number of G418 resistant colonies were observed in pCMVgp-neo, whereas no G418 resistant colonies were not found inpCMVgp-pol-d1 or CMVgp-pol-d2. As such, it was demonstrated that the gag-pol of the IAP of the present invention is critical for transposition. That is, the IAP of the present invention has an ability of causing transposition in an autonomous manner. The pCMVgp expressing the gag-pol is co-transfected to allow detection of the transposition by means of pCMVgp-pol-d1 and pCMVgp-pol-d2. That is to say, it is believed that the IAP vector allows regulation of transposition by separating the expression vector of the IAP vector and the *gag-pol* of the *gag-pol* deletion type.

(EXAMPLE 3: Determination of insertion sites into the genome of the IAP vector by means of Ligation-mediated PCR)

One hundred (100) ng of the genomic DNA were cleaved with restriction enzymes such as EcoRV, HincII, MscI, ScaI, SmaI and the like, and the enzymes were inactivated by heat treatment and a linker DNA was linked thereto.

Linker DNA were produced by complementing the following sequence:5'-CGAATCGTAACCGTTCGTACGAGAATTCGTACGAGAATCGCTGTCCTCTCCAACG AGCCAAGG-3' (SEQ ID NO: 17) and 5'-CCTTGGCTCGTTTTTTTTTGCAAAAA-3'(SEQ ID NO: 18). Using the genomic DNA fragment linked with a linker as a template, nested PCR was conducted using a primer specific for the linker and a primer specific for the neo cassette inside the IAP. The primers used in the first PCR are as follows: 5'-CGAATCGTAACCGTTCGTACGAGAA-3' (SEQ ID NO: 19) (linker specific primer) and 5'-GAGATGCATGCTTTGCATACTTCTGCCTGC-3' (SEQ ID NO: 20) (neo cassette specific primer). The primers used in the second PCR are as follows: 5'-TCGTACGAGAATCGCTGTCCTCTCC-3'(SEQ ID NO: 21) (linker specific primer) and 5'-GGAGCCTGGGGACTTTCCACACCTGGTTGC-3'(SEQ ID NO: 22) (neo cassette specific primer) or 5'-GGGGAGCCTGGGGACTTTCCACACCCTAAC-3' (SEQ ID NO: 23) (neo cassette specific primer). The conditions of the PCR are as follows for the first and second rounds: 94°C x five minutes, (94°C x one minute - 55°C x one minute - 68°C x two minutes) for thirty cycles, and thereafter 68°C x seven minutes using EXpand HiFi PCR system. The amplified bands were sequenced using ABI PRISM 3100 and the location on the genome and the gene present thereon were identified using Ensembl database (http://www.ensembl.org/) (Figures **6A** and **6B**).

Figure **6** shows an example in which an IAP vector is inserted into a gene by the present Example. Figure **6A** shows the region determined by ligation mediated PCR. Figure **6B** shows the results determined by the Ensembl database. The sequence determined was searched using the Ensembl to identify that the sequence has been inserted into the cytoglobin gene, and thus it was demonstrated that the IAP vector allows introduction of mutation into a gene.

As described above, the location, which was transposed in the present Example is elucidated to be the cytoglobin gene present in AL607039 in Chromosome 11. Accordingly, it was demonstrated that the present invention has an activity of actually allowing exhaustively transposing the genome.

(EXAMPLE 4: Replacement of the U3 region with the CA promoter)

Next, transposition activity of a retrotransposon using different promoter sequence was observed.

Using pCX-EGFP (Okabe et al., FEBS Lett. 407;313-319,1997)as a template, the portion from the human cytomegalovirus enhancer region to the chicken beta-actin promoter transcription initiation point in the CAG promoter sequence (Niwa et al., Gene 108; 193-199, 1991) was amplified by PCR. For a primer upstream of the 5' side, CA-U1 (5'-GCAATGCGGCCGCATTGATTATTGACTAGTTATTAATAG-3' (SEQ ID NO:24)) was used. It was reported that there were two transcription initiation points for the chicken beta-actin promoter (Kost et al., Nucleic Acids Res. 11: 8287-8301, 1983), the following two primers were used corresponding to the respective region for the 3' side primer: CA-L1,5'-CGAGAAAAACCGCCCGCCGCGCGCTTCGCTTTTTATAGG-3' (SEQ ID NO:25) and CA-L2,5'-CGAGAAAAACCCCGCCCGCCGCGCGCTTCGCTTTTTATAG-3' (SEQ ID NO:26). The region amplified thereby was designated type-1 CA promoter (CA1) and type-2 CA promoter (CA2), respectively. Pfx polymerase was used for the PCR, and the enhancer reagent attached to the polymerase was used at x 1 concentration. The conditions of the PCR were as follows: 94°C x two minutes, (94°C x fifteen seconds - 55°C x thirty seconds - 68°C x one minute) for thirty cycles, and thereafter 68°C x two minutes. The region of about 0.3 kb from the 5' terminus of the R region of the IAP to downstream of the U5 region thereof was amplified using the PCR primers: R-U3, 5'-CGCGGCGGGCGGTTTTTCTCGCTCTCTTGCTTCTTG-3' (SEQ ID NO: 27) and R-L1, 5'-TCTGAAATGAAGTATCCCTCCTGCGCCAGT-3' (SEQ ID NO: 28), or R-U4, 5'-CGGCGGGCGGGGTTTTTCTCGCTCTCTTGCTTCTTG-3' (SEQ ID NO: 29) and R-L1. Pfx polymerase was used for the PCR (without using enhancer reagents). The conditions of PCR were as follows: 94°C x two minutes, (94°C x fifteen seconds - 55°C x thirty seconds - 68°C x one minute) for fifteen cycles, and thereafter 68°C x two minutes. The PCR product by means of CA-U1 and CA-L1 and the PCR product by means of R-U3 and R-L1 were mixed and PCR was performed using CA-U1 and R-L1. Similarly, the PCR product by means of CA-U1 and CA-L2 and the PCR product by means of R-U4 and R-L1 were mixed and PCR was performed using CA-U1 and R-L1. CA-L1 and CA-L2, and R-U3 and R-U4 are designed to have complementary sequences to each other in the 5' sides. The subject PCR fuses the transcription initiation site of the beta-actin promoter and the 5' terminus of the R region. Pfx polymerase was used for the PCR and the enhancer reagent attached to the polymerase was used at the concentration of x 1, and the following conditions were used for amplification: 94°C x 2 minutes, (94°C x fifteen seconds - 55°C x thirty seconds - 68°C x 1 minute) for thirty cycles, and thereafter 68°C x 2 minutes. The PCR product has been cloned by means of Zero Blunt TOPO PCR cloning kit and clones with no base sequence replacement were identified by PCR. The subject clone was cleaved by NotI and BspI, and thereafter replaced with the NotI-BspI region of the pCMVgp-neo. The resultant vector is designated as pCA1gp-neo and pCA2gp-neo (Figure **7A**).

(RESULTS)

Figure **7** shows the effects of the CA promoter. (A) The U3 region, a promoter of IAP was replaced with cytomegalovirus enhancer and chicken beta-actin promoter to produce two vectors (pCA1gp-neo and pCA2gp-*neo*). Hereinafter, the promoters of each vector are designated as type 1 CA promoter (CA1) and type 2 CA promoter (CA2).

See (B) for detail. pCMVgp-neo is the same as in Figure **1.** (B) The sequence of juncture of the two CA promoters and the R region. There reported two sites for transcription initiation points of the chicken beta-actin promoter (see the item of (Methods) as described hereinabove). As such, depending on each case, design was achieved such that the transcription starts from the 5' terminus of the R region, and were designated as type 1 and type 2 CA promoters (CA1 and CA2). (C) Comparison of CA1, CA2 and CMV promoters. After transfection, G418 resistant colony number derived from NIH3T3 and HeLa cells (5 x 10⁵) were assayed. The CA2 promoter resulted in the largest number of colonies.

Figure **7B** shows type 1 CA promoter and type 2 CA promoter. As such, type 2 CA promoter is longer by two bases than type 1 CA promoter. There are reported to be two transcription initiation points of the chicken beta-actin promoter (see the above-mentioned Examples). Therefore, depending on each of the cases, it was designed such that transcription initiates at the 5' terminus of the R region, and designated as type 1 and type 2 CA promoters (CA1 and CA2).

Figure **7C** shows the results in which the number of G418 resistant colonies were counted. After transfection, $5 \times 10^5$ cells of NIH3T3- and HeLa-derived G418 resistant colonies were assayed. Most colonies were obtained by CA2 promoter. As shown therein, the transcription activity resulted by type 2 CA promoter is higher than CMV and the like. This is estimated to be comparable or greater than the activity which the CAG usually possesses. Therefore, it was demonstrated that the CA promoter of the present invention has subsequent potent promoter activity.

(EXAMPLE 5: Production of hr GFP cassette and insertion into IAP vector)

Next, an exemplification using the GFP gene is presented as a foreign gene.

A gamma-globin intron in the neo cassette of the previously described pJM101/L1.3 was inserted between the 192 base of the hrGFP gene (Stratagene) and the 193 base thereof (the base A of ATG, the translation initiation site is defined as the first base), in a reverse direction in terms of the hrGFP gene. Further, this hrGFP cassette was inserted into the NdeI site downstream of the *pol* gene of the previously described pCMVgp, and those having IAP and gamma globin intron in the same forward direction were identified and designated as pCMVgp-hrGFP (Figure **8**).

The measurement of the GFP expression was conducted using GFP specified Filter (Olympus, Tokyo, Japan) and Olympus fluorescence inverted microscope at x 100 - 400 magnification.

Figure **8** shows an exemplification of visualization of transposition using GFP. (A) The structure of the vectors: A GFP cassette with an intron located inside the hrGFP was produced and inserted into the IAP vector having the CA2 promoter. (B) The expression of GFP associated with transposition: the above-mentioned vector was transfected into a HeLa cell, and cells having fluorescence of the GFP were identified. Hence, the transposition of IAP were able to be visualized and thus it is believed that such a system is effective for detection of transposition in a model animal such as a mouse.

As such, cells having fluorescence of the GFP are limited to the cells with the vector actually including the retrotransposon introduced therein. It was then demonstrated that any foreign gene is observed to cause transposition for the GFP gene in addition to *neo* gene shown in Examples 2 and 4, and thus transposable for any foreign gene.

(EXAMPLE 6: Demonstration at biological level in the modification of the genome by means of retrotransposon)

The present Example confirms that the present inventors can use the retrotransposon may be actually used in a transgenic animal. A retrotransposon vector is injected in to a mouse fertilized egg. Alternatively, the retrotransposon vector is transfected into an ES cell and an ES cell is identified to the genome of which a vector DNA is inserted without transposition reaction.

(EXAMPLE 7: Production of transgenic mouse)

A system as described in Example 6 is used to produce a transgenic mouse. In brief, ES cell obtained in Example 6 is injected into blastocyst and the injected blastocyst is returned to the oviduct or uterus a pseudopregnant mouse to generate a mouse. Mutations are analyzed to confirm the effect of genomic modification of the retrotransposon in a transgenic animal.

(EXAMPLE 8: Assay of transposition of the IAP element in a mouse individual)

In the present Example, the DNA fragment of the vector shown in Figure **8A** is injected into a fertilized mouse egg, and transplanted into the oviduct of a pseudopregnant mouse. The resultant mouse is identified to be a founder mouse by screening with PCR using primers specific for hr GFP, and crossbred with a wild type mouse to established a lineage of a mouse. Specifically, a transgenic mouse having pCA2gp-hrGFP shown in Figure **8** is produced and PCR is subsequently conducted using primers corresponding to the location shown in Figure **8**, using DNA from a mouse tail as a template. The sequences of the primers are as follows:

SEQ ID NO: 37 AGGGCTGCGGCAAGGGCAACATCCTGTTCG (1st sense)
SEQ ID NO: 38 GCCGCCGTCCTCCACGTAGGTCTTCTCCAG (1st antisense)
SEQ ID NO: 39 GGCAACCAGCTGGTGCAGATCCGCGTGACC (2nd sense)
SEQ ID NO: 40 GTCCTTCACCACGCCCTTGCTCTTCATCAG (2nd antisense)

Once transposition of IAP occurs, intron inside the GFP will be disappeared. Therefore, it is expected that a 0.45 kb band will appear. As shown in Figure **8**, 0.45 kb band are detected in three out of thirteen lineages of mice, and thus it is demonstrated that transposition actually occurs in the living mice.

Child mice is observed with fluorescence substantial stereo-microscope with filter for GFP (LEICA, WILD M10 and the like), which will allow the identification of mice with no GFP signal but with the vector sequence. The resultant mouse is crossbred with a wild-type mouse to obtain a child mouse, and the mouse is observed using a similar fluorescence substantial stereo-microscope. Mice with fluorescence in the entire body are expected to have caused transposition in the course of germ-line production formation of the parent mice. Therefore, it is deemed to have a mutated mouse subsequently produced in the next generation.

In the vector shown in Figure **8A**, it is difficult to control transposition since there is all elements necessary for transposition in a single vector. Therefore, in order to allow control of the transposition, the following experiments can be conducted. Firstly, a vector (corresponding to pCA2gp-hrGFP-M1 as used in the next Example 9) with a mutation introduced into the initiation codon of the *gag* gene is prepared. Next, injection of the DNA fragment to a fertilized egg, followed by the oviduct implantation of the egg in a pseudopregnant mouse leads to establishments of a mouse lineage. The IAP element introduced into the mouse has a deletion in the *pol* gene, and thus it is believed that it cannot cause transposition in an autonomous manner (that is non-self type). On the other hand, a vector for expression of *gag-pol* is produced in which the *gag-pol* gene fragment has been resected from the IAP element to be placed downstream under a potent promoter (for example, CAG promoter), and the mouse having the subject vector is established in a similar manner. The resultant mouse is crossbred with a mouse having the non-autonomous type vector. The resultant fetal mouse is=used to identify a mouse having both vectors, and crossbred with a wild-type mouse. The resultant fetal mouse is observed under fluorescent stereoscopic microscope. Mice exhibiting fluorescence in the entire body are expected to have caused transposition in the course of production of the germ-line cell in the parent mice, and thus it is deemed to have produced a mutant mouse in the following generation. The resultant mutant mouse and a wild-type mouse is crossbred to identify a mouse having a non-autonomous IAP vector and having the *gag-pol* expression vector deleted therefrom. This mouse is considered to have the non-autonomous type IAP vector which is lack of transposition ability. As described above, it is possible to confirm whether transposition can be controlled in a mouse individual.

(EXAMPLE 9: The first fifteen amino acids of the GAG protein are preferable)

Next, the first fifteen amino acids of the GAG protein is demonstrated to be preferable for transposition. The specific scheme is shown in Figure **10**.

Figure **10A** shows the structure of the vector used. As shown, in comparison with pCA2gp-hrGFP exhibiting autonomous transposition in Figure **8**, pCA2gp-hrGFP-M1 introduced mutation therein at the initiation codon of the gag gene, and as a result, it is believed that the translation shall start from the second ATG, at fifteen amino acids downstream therefrom.

Next, as shown in Figure **10B**, transposition efficiency can be studied. The vector shown in Figure **10A** is used to transfect HeLa cells with three combinations as shown therein. After seven days, the ratio of GFP positive cells is analyzed by FACS. As a result, pCA2gp-hrGFP-M1 in which a mutation has been introduced in the ATG of the original translation initiation site of the *gag* gene, has subsequently lost its transposition ability. However, when the same vector is co-transfected with pCA2gp, an expression vector of the *gag-pol* full length, the transposition ability will be recovered. Hence, it is shown that it is important to have the fifteen amino acids from the translation initiation site of the GAG protein for effecting the transposition activity. As such, it is firstly elucidated that it is important to have the first fifteen amino acids of the GAG protein for the full length of the IAP, which is believed to be preferable for attaining the genome modification effects.

(EXAMPLE 10: It is preferable in the transposition of non-autonomous vector, that the GAG protein is translated from *per se*.)

Next, in order to demonstrate that it is preferable that the translation of the GAG protein is achieved from *per se* in the transposition of a non-autonomous vector, experiments shown in Figure **10** have been conducted.

The structure of the vectors used therein is shown in Figure **11A**. The first three vectors and the *gag-pol* expression vector are the same as those in Figure **10**. In pCA2gp-hrGFP-M2 and pCA2gp-hrGFP-M3, termination codon is introduced immediately downstream of the second ATG of the *gag*, and thus the GAG protein can only be expressed as a short fragment. Four vectors having mutation in the GAG protein are defined as a non-autonomous vector since they cannot cause transposition per se.

Next, as shown in Figure **11B**, transposition efficiency is studied. The transfer vector of Figure **11A** is transfected into a HeLa cell in the presence or absence of the *gag-pol* expression vector (pCA2gp) or alternatively using pBluescript, to analyze the appearance frequency of the GFP-positive cells seven days after by FACS. As a result, in the three non-autonomous vectors with the translation of the GAG protein suppressed, significantly lowers the transposition under the presence of the *gag-pol* expression vector. On the other hand, transposition is only observed at a high frequency only under the presence of the *gag-pol* expression vector for pCA2gp-hrGFP-M1 which causes full length translation after the first fifteen amino acids. Hence, it is shown that the translation of the *gag* protein from itself is preferable for transposition of a non-autonomous vector.

Although certain preferred embodiments have been described herein, it is not intended that such embodiments be construed as limitations on the scope of the invention except as set forth in the appended claims. Various other modifications and equivalents will be apparent to and can be readily made by those skilled in the art, after reading the description herein, without departing from the scope and spirit of this invention. All patents, published patent applications and publications cited herein are incorporated by reference as if set forth fully herein.

INDUSTRIAL APPLICABILITY

The present invention is used to efficiently conduct genomic modification even if sites far from the site of interest. Such an organism relating thereto is useful as a model animal, for screening and pharmacological experiment and the like.

**Claims**

1. An isolated nucleic acid construct comprising a nucleic acid sequence encoding an LTR-type retrotransposon.

2. A nucleic acid construct according to Claim 1 wherein the LTR-type retrotransposon comprises Intracisternal A particle (IAP)-type retrotransposon.

3. A nucleic acid construct according to Claim 1 wherein the retrotransposon comprises a full-length IAP element.

4. A nucleic acid construct according to Claim 1 wherein the retrotransposon encodes a polypeptide having a function.

5. A nucleic acid construct according to Claim 1 wherein the function comprises at least one activity selected from the group consisting of transcription activity, reverse transcription activity and integrase activity.

6. A nucleic acid construct according to Claim 1 wherein the retrotransposon is an IAP element and at least -one domain selected from the group consisting of LTR, *gag, pol* and tRNA binding site, which is conserved against SEQ ID NO: 1.

7. A nucleic acid construct according to Claim 1 wherein the retrotransposon is an IAP element, wherein the nucleic acid thereof has at least one feature selected from the group consisting of repeat of a sequence of TCCGGGACGAGAAAA in the tRNA binding site immediately located at LTR at the 5' side, and inclusion of two or more repeat sequences TTGCTTCTTGCTCTC in the R region.

8. A nucleic acid construct according to Claim 1 wherein the retrotransposon comprises:

(a) a polynucleotide having a base sequence set forth in SEQ ID NO: 1 or a fragment sequence thereof;
(b) a polynucleotide encoding a polypeptide consisting of an amino acid sequence set forth in SEQ ID NO: 2, or 3 and 4, or a fragment thereof;
(c) a polynucleotide encoding a variant polypeptide consisting of an amino acid sequence set forth in SEQ ID NO: 2, or 3 and 4 with at least one mutation selected from consisting of at least one amino acid substitution, addition and deletion, or a fragment thereof, which possesses a biological activity;
(d) a polynucleotide being a splice variant or allelic variant of the base sequence set forth in SEQ ID NO: 1, or a fragment thereof;
(e) a polynucleotide encoding a species homolog of a polypeptide consisting of an amino acid sequence set forth in SEQ ID NO: 2, or 3 and 4, or a fragment thereof;
(f) a polynucleotide which hybridizes to any of polynucleotides (a) through (e) or the complement thereof under stringent conditions, and encoding a polypeptide having a biological activity; or

(g) a polynucleotide having at least 70 % identity to any of polynucleotides (a) through (e) or the complement thereof under stringent conditions, and encoding a polypeptide having a biological activity.

**9.** A nucleic acid construct according to Claim 1 wherein the nucleic acid sequence encoding the retrotransposon comprises SEQ ID NO: 1.

**10.** A nucleic acid construct according to Claim 1 further comprising a promoter sequence.

**11.** A nucleic acid construct according to Claim 10 wherein the promoter sequence has an activity of 0.1 rlu or greater when determined by a luciferase assay *in vitro*.

**12.** A nucleic acid construct according to Claim 10 wherein the promoter sequence is selected from the group consisting of CMV, CA and the variants thereof.

**13.** A nucleic acid construct according to Claim 10 wherein the promoter sequence partially substitutes a portion of 5'LTR of the LTR-type retrotransposon.

**14.** A nucleic acid construct according to Claim 13 wherein the promoter sequence substitutes an entirety or portion of U3 region in the 5'^LTR in the LTR-type retrotransposon.

**15.** A nucleic acid construct according to Claim 10 wherein the promoter sequence is operably linked to the retrotransposon.

**16.** A nucleic acid construct according to Claim 10 wherein the promoter sequence is located in frame to a transcription initiation site of the retrotransposon at the transcription initiation site of the promoter sequence.

**17.** A nucleic acid construct according to Claim 10 wherein the promoter sequence is a base sequence set forth in any of SEQ ID NO: 5-7, or a portion or variant thereof, and comprises a nucleic acid sequence having promoter activity.

**18.** A nucleic acid construct according to Claim 10 wherein the promoter sequence consists of a nucleic acid sequence set forth in SEQ ID NO: 6 or 7.

**19.** A nucleic acid construct according to Claim 1 further comprising a sequence encoding a foreign gene.

**20.** A nucleic acid construct according to Claim 19 wherein the sequence encoding the foreign gene is placed in said retrotransposon.

**21.** A nucleic acid construct according to Claim 19 wherein the foreign gene renders a host a distinguishable property.

**22.** A nucleic acid construct according to Claim 21 wherein the distinguishable property is selected from the group consisting of PCR primer, antibiotic resistance, complement of nutrition, enzymatic activity and fluorescence.

**23.** A nucleic acid construct according to Claim 19, wherein the foreign gene is selected from the group consisting of neo, GFP, hyg, puro, zeo, bsr, lacZ, CFP, YFP, RFP, BFP and hrGFP.

**24.** A nucleic acid construct according to Claim 19, wherein the foreign gene is composed such that the foreign gene is first expressed only after transcription, reverse transcription and insertion into the genome it is subjected to.

**25.** A nucleic acid construct according to Claim 19, wherein the foreign gene comprises an intron sequence.

**26.** A nucleic acid construct according to Claim 25, wherein the intron sequence is located in the same transcription direction (forward) with respect to the retrotransposon.

**27.** A nucleic acid construct according to Claim 25, wherein the intron sequence is located between a splice donor sequence and a splice acceptor sequence.

**28.** A nucleic acid construct according to Claim 1 for use in genomic modification.

**29.** A nucleic acid construct according to Claim 11 which is for confirming whether or not the retrotransposon has transposition ability.

**30.** A nucleic acid construct according to Claim 19 which is for transposing the foreign gene.

**31.** A nucleic acid construct according to Claim 19 which is used for introducing the foreign gene into a host.

**32.** A method for modifying a genome in a cell, comprising the steps of:

> A) providing a nucleic acid construct comprising an LTR-type retrotransposon;
> B) introducing the nucleic acid construct into the cell;
> C) culturing the cell for a predetermined period of time; and
> D) selecting a cell with a genome modified by means of the nucleic acid construct.

**33.** A method according to Claim 32, further comprising a promoter having an activity of 0.1 rlu or greater as determined by a luciferase assay *in vitro,* wherein the predetermined period of time is sufficient for transcription, reverse transcription and insertion into the genome.

**34.** A method according to Claim 32, wherein the promoter sequence is located in frame to a transcription initiation site of the retrotransposon at the transcription initiation site of the promoter sequence.

**35.** A method according to Claim 32, wherein the nucleic acid construct comprises a foreign gene located in an operable manner in the retrotransposon, and the selection is achieved by the expression of the foreign gene.

**36.** A method according to Claim 32, wherein the foreign gene is located in the reverse direction with respect to the transcription direction of the retrotransposon, and comprises a splice donor sequence and splice acceptor sequence, and an intron sequence located cis-direction sandwiched therebetween, wherein said predetermined period of time is sufficient for achieving transcription, reverse transcription and insertion into the genome, and wherein the selection is achieved by the expression of the foreign gene.

**37.** A method according to Claim 36, wherein the foreign gene encodes an agent selected from the group consisting of a antibiotic resistance gene, nutrient supplement agent, enzyme and fluorophore, and the selection is achieved by the property of the cell expressing the agent.

**38.** A method according to Claim 32, wherein the LTR-type retrotransposon comprises an IAP element.

**39.** A method according to Claim 32, wherein the LTR-type retrotransposon comprises a full-length IAP element.

**40.** A method according to Claim 32, wherein the selection is achieved by confirming the transposed sequence by means of ligation mediated PCR.

**41.** A method according to Claim 32, wherein the introduction comprises a format selected from the group consisting of transfection, transformation and transduction.

**42.** A method according to Claim 32, wherein the introduction is achieved in the presence of at least one substance selected from the group consisting of cationic lipids and polyamine reagents.

**43.** A method according to Claim 32, wherein the cell is of the same species as that of the natural host of the retrotransposon.

**44.** A method according to Claim 32, wherein the cell is of the different species as that of the natural host of the retrotransposon.

**45.** A method for assaying transposition activity of a retrotransposon, comprising the steps of:

> A) providing a nucleic acid construct comprising a nucleic acid sequence encoding a retrotransposon to be assayed, and a promoter sequence having activity of at least 0.1 rlu as determined by a luciferase assay *in vitro*;
> B) introducing the nucleic acid construct into the cell;

C) culturing the cell for a predetermined period of time; and
D) detecting the transposition by means of nucleic acid construct.

**46.** A method according to Claim 45, wherein the detection comprises the step of ligation mediated PCR.

**47.** A method according to Claim 45, wherein the detection comprises the step of comparing a genomic database and the sequence obtained by the ligation mediated PCR.

**48.** A method for producing the transgenic organism, comprising the steps of:

A) providing a nucleic acid construct comprising a nucleic acid sequence encoding a LTR-type retrotransposon;
B) introducing the nucleic acid construct into a germ-line cell of a desired biological organism;
C) selecting a germ-line cell with the genome thereof modified in the germ-line cell; and
D) regenerating the germ-line cell with the genome thereof modified into a biological organism.

**49.** A kit for modifying the genome of a cell, comprising:

A) a nucleic acid construct comprising a nucleic acid sequence encoding a LTR-type retrotransposon;
B) means for introducing the nucleic acid construct into a germ-line cell of a desired biological organism; and
C) means for selecting a germ-line cell with the genome thereof modified in the germ-line cell.

**50.** A kit according to Claim 49, wherein the means for introducing the nucleic acid construct into the cell comprises a transfection reagent.

**51.** A kit according to Claim 48, wherein the transfection reagent is selected from the group consisting of cationic macromolecule, cationic lipid, polyamine reagent, polyimine reagent, and calcium phosphate.

**52.** A kit according to Claim 50, wherein the transfection reagent is selected from the group consisting of cationic lipid and polyamine reagent.

**53.** A kit according to Claim 49, wherein the means for selection comprises at least one of means for detection corresponding to one selected from the group consisting of a PCR primer, antibiotic resistance, complement of nutrition, enzymatic activity and fluorescence.

**54.** A kit for assaying transposition activity of a retrotransposon, comprising:

A) a nucleic acid construct comprising a nucleic acid sequence encoding a LTR-type retrotransposon, and a promoter having an activity of 0.1 rlu or greater as determined by a luciferase assay *in vitro;*
B) means for introducing the nucleic acid construct into the cell; and
C) means for detecting transposition by the nucleic acid construct.

**55.** A kit according to Claim 54, wherein the means for detecting comprises at least one means selected from means for detection of at least one of the group consisting of PCR primer, antibiotic resistance, complement of nutrition, enzymatic activity and fluorescence.

**56.** A kit for producing a transgenic organism, comprising:

A) a nucleic acid construct comprising a nucleic acid sequence encoding an LTR-type retrotransposon;
B) means for introducing the nucleic acid construct into a germ-line cell of a desired organism;
C) means for selecting a germ-line cell with the genome thereof modified in the germ-line cell; and
D) means for regenerating the germ-line with the genome thereof modified into an organism.

**57.** A kit according to Claim 56, wherein the means for regenerating the organism comprises an organism as a host.

**58.** A promoter comprising a cytomegalovirus enhancer and avian beta-actin promoter, wherein at least one of the cytomegalovirus enhancer and the avian beta-actin promoter comprises a sequence shorter than the native full-length thereof.

**59.** A promoter according to Claim 58, wherein the shorter sequence is due to the deletion of a sequence downstream of the transcription initiation site.

**60.** A promoter according to Claim 58, wherein all the sequence down stream of the transcription initiation site is deleted.

**61.** A promoter according to Claim 58, wherein a portion of a sequence downstream of the transcription initiation site and the promoter region is deleted.

**62.** A promoter according to Claim 58, wherein the cytomegalovirus enhancer comprises a sequence set forth in SEQ ID NO: 36 and a variant thereof.

**63.** A promoter according to Claim 58, wherein the avian beta-actin promoter comprises a sequence set forth in SEQ ID NO: 8 or a variant thereof.

**64.** A promoter according to Claim 58, comprising the sequence set forth in SEQ ID NO: 6.

**65.** A promoter according to Claim 58, comprising the sequence set forth in SEQ ID NO: 7.

**66.** A promoter according to Claim 58, consisting of the sequence set forth in SEQ ID NO: 6.

**67.** A promoter according to Claim 58, consisting of the sequence set forth in SEQ ID NO: 7.

**68.** Use of an LTR-type retrotransposon for genomic modification.

**69.** Use of a promoter having an activity of 0.1 rlu or greater as determined by a luciferase assay *in vitro,* for modification of a genome.

**70.** Use of a promoter having an activity of 0.1 rlu or greater as determined by a luciferase assay *in vitro,* for confirmation of an LTR-type retrotransposon.

DNA-type transposon       RNA-type transposon

ex) Sleeping Beauty        =(retrotransposon)

                              ex) IAP element

Transposon

Genome

Transposase enzyme        Insertion into a genome

Transcription             Reverse transcription

                    AAAAAA

              RNA

**Fig. 1**

**A**

5' LTR

U3 | R | U5

*gag*

*pol*

3' LTR

U3 | R | U5

Region to be transcribed

**B**

5' LTR

The genomic DNA of
the host cell

*gag*

*pol*

3' LTR

Insertion into
the genomic DNA
of the host cell

①

②

⑤

mRNA for production of *Gag-Pol*

IAP genomic
RNA

DNA
DNA

Gag-Pol

③

DNA
RNA

④ Reverse transcription

RNA

Formation of IAP

**Fig. 2**

Fig. 3

A

The *neo* cassette with an intron located in
a reverse direction to the neo gene in a coding
region of the *neo* gene, wherein the *neo* casstte
is inserted into the IAP.

original U3 of IAP

Replacing U3 region
with CMV promoter

B

CMV promoter

a predicted transcription
initiation site

--- GAGCTCGTTAGTGAACCGTTTTTCTCGCTCTCTTGC ---

R region

C

D

| Vector | The number of G418 resistant colonies |
|---|---|
| Mock | 0 |
| pU3gp-neo | 0 |
| pCMVgp-neo | 110 |
| pJM101/L1.3(LINE1) | 22 |

# Fig. 4

A

B

| Vector | The number of G418 resistant colonies |
|--------|---------------------------------------|
| Mock | 0 |
| pCMVgp-neo | 264 |
| pCMVgf-neo-d1 | 0 |
| pCMVgf-neo-d2 | 0 |
| pCMVgf-neo-d1 + CMVgp | 43 |
| pCMVgf-neo-d2 + CMVgp | 28 |

# Fig. 5

A

IAP

Genome DNA

5' LTR          *gag*   *pol*          oʍn   3' LTR

a region identified with
ligation-mediated PCR

B

Search of a insertion site
using the Ensemble database

Chr 11          RL607039 >

Blast hit

Direction of insertion of IAP
into a genome

Cygb

Direction of cytoglobin gene

# Fig. 6

A

Replacing a U3 region
with a type 1 CA promoter

Replacing a U3 region
with a type 2 CA promoter

Replacing a U3 region
with a CMV promoter

B

Type 1 CA promoter   ···GAAGCGCGCGGCGGGCGGTTTTTTCTCGCTCTCTTGC···

Type 2 CA promoter   ···GAAGCGCGCGGCGGGCGGGGTTTTTCTCGCTCTCTTGC···

R region

Type 2 CA promoter being
longer than type 1 by 2 bp

C

| Vector | The number of G418 resistant colonies | |
|---|---|---|
| | NIH3T3 | HeLa |
| pCA1gp-neo | 65 | 95 |
| | 92 | 223 |
| pCA2gp-neo | 173 | 230 |
| | 124 | 185 |
| pCMVgp-neo | 92 | 208 |
| | 90 | 177 |

# Fig. 7

A

B

Bright-field          Fluorescence-field

# Fig. 8

Fig. 9

A

pCA2gp-hrGFP

pCA2gp-hrGFP-M1

pCA2gp
(expression vector for gap-pol)

The first ATG in a gag gene,
which is modified in pCA2gp-hrGFP-M1
into ATT.

The second ATG in a gag gene,
from which translation in pCA2gp-hrGFP-M1
is predicted to start.

WT
(pCA2gp-hrGFP)

ATG AAT TCA GAA CTT TTC AGC TGG GGA ACG AGA GTA CCA GTG AGT ATG TTT GCC CTT GAA
Met Asn Ser Glu Leu Phe Ser Trp Gly Thr Arg Val Pro Val Ser Met Phe Gly Leu Glu

B

| Vector | % GFP-positive |
|---|---|
| pCA2gp-hrGFP | 0.29 |
| pCA2gp-hrGFP-M1 | 0 |
| pCA2gp-hrGFP-M1 + pCA2gp | 0.22 |

Fig. 10

A

Transfer vector

gag-pol expression vector

B

| Transfer vector | % GFP-positive | |
| --- | --- | --- |
| | +pBluescript | +pCA2gp |
| pCA2gp-hrGFP | 0.29 | not done |
| pCA2gp-hrGFP-M1 | 0 | 0.215 |
| pCA2gp-hrGFP-M2 | 0 | 0.005 |
| pCA2gp-hrGFP-M3 | 0 | 0.04 |
| pCA2gp-hrGFP-M4 | 0 | 0.015 |

Fig. 11

## INTERNATIONAL SEARCH REPORT

| International application No. |
| --- |
| PCT/JP2004/017307 |

**A. CLASSIFICATION OF SUBJECT MATTER**
    Int.Cl⁷ C12N15/09, C12Q1/02, C12Q1/68, A01K67/027

According to International Patent Classification (IPC) or to both national classification and IPC

**B. FIELDS SEARCHED**

Minimum documentation searched (classification system followed by classification symbols)
    Int.Cl⁷ C12N15/09, C12Q1/02, C12Q1/68, A01K67/027

Documentation searched other than minimum documentation to the extent that such documents are included in the fields searched

Electronic data base consulted during the international search (name of data base and, where practicable, search terms used)
    BIOSIS/WPI(DIALOG), MEDLINE(STN), JSTPlus/JST7580(JOIS),
    SwissProt/PIR/GeneSeq, GenBank/EMBL/DDBJ/GeneSeq

**C. DOCUMENTS CONSIDERED TO BE RELEVANT**

| Category* | Citation of document, with indication, where appropriate, of the relevant passages | Relevant to claim No. |
| --- | --- | --- |
| X/Y | Ishihara, H. et al., Mus musculus DNA, full-length type-I intracisternal A-particle element, complete sequence, clone:Q14, 11 July, 2003 (11.07.03), DATABASE GenBank, ACCESSION AB099818 | 1-11,15-16, 28-29/12-14, 17-27,30-44, 48-53,56-57, 68 |
| X/Y | Mietz, J.A. et al., Nucleotide sequence of a complete mouse intracisternal A-particle genome: relationship to known aspects of particle assembly and function, J.Virol, 1987, Vol.61, No.10, pages 3020 to 3029 | 1-6,8-11, 15-16,28-29 /7,12-14, 17-27,30-44, 48-53,56-57, 68 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 01 April, 2005 (01.04.05) | 19 April, 2005 (19.04.05) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

## INTERNATIONAL SEARCH REPORT

| | International application No. |
|---|---|
| | PCT/JP2004/017307 |

C (Continuation). DOCUMENTS CONSIDERED TO BE RELEVANT

| Category* | Citation of document, with indication, where appropriate, of the relevant passages | Relevant to claim No. |
|---|---|---|
| X/Y | Heldmann, O. et al., Retrotransposition of a mouse IAP sequence tagged with an indicator gene, Cell, 1991, Vol.64, No.1, pages 159 to 170 | 1-2,4-6, 8-11,15-16, 19-38,40-44, 68/3,7, 12-14,17-18, 39,48-53, 56-57,68 |
| Y | Largaespada, D.A. et al., Generating and manipulating transgenic animals using transposable elements, Reprod Biol. Endocrinol, 07 November, 2003 (07.11.03), Vol.1, No.1, 80 | 48-53,56-57 |
| P,X | Dewannieux, M. et al., Identification of autonomous IAP LTR retrotransposons mobile in mammalian cells, Nat Genet, 2004 May, Vol.36, No.5, pages 534 to 539 | 1-44,48-53, 56-57,68 |
| P,X | Kyozo HORIE et al., "Transposon ni yoru Shinka, Hen'i Donyu no Seibutsugakuteki Igi Mouse ni Okeru Transposon Kenkyu no Shinten", Protein, Nucleic acid and Enzyme, 2004 Nen 10 Gatsu, Vol.49, No.13, pages 2117 to 2122 | 1-44,48-53, 56-57,68 |

Form PCT/ISA/210 (continuation of second sheet) (January 2004)

## INTERNATIONAL SEARCH REPORT

| International application No. |
|---|
| PCT/JP2004/017307 |

**Box No. II**     **Observations where certain claims were found unsearchable (Continuation of item 2 of first sheet)**

This international search report has not been established in respect of certain claims under Article 17(2)(a) for the following reasons:

1. ☐   Claims Nos.:
   because they relate to subject matter not required to be searched by this Authority, namely:

2. ☐   Claims Nos.:
   because they relate to parts of the international application that do not comply with the prescribed requirements to such an extent that no meaningful international search can be carried out, specifically:

3. ☐   Claims Nos.:
   because they are dependent claims and are not drafted in accordance with the second and third sentences of Rule 6.4(a).

**Box No. III**     **Observations where unity of invention is lacking (Continuation of item 3 of first sheet)**

This International Searching Authority found multiple inventions in this international application, as follows:
(1) The inventions according to claims 1 to 44, 48 to 53, 56 to 57 and 68 relate to a nucleic acid construct containing a nucleic acid sequence encoding an LTR retrotransposon and a method of using the construct. (2) The inventions according to claims 45 to 47 and 54 to 55 relate to a method of calibrating the transfer activity of a retrotransposon. (3) The inventions according to claims 58 to 67 relate to a promoter containing a cytomegarovirus enhancer and an avian β-actin promoter wherein at least one of the enhancer and the promoter contains a sequence shorter than the natural full-length sequence. (4) The invention according to claim 69 relates a method of using a promoter, which has an activity of 0.1 rlu or higher     (continued to extra sheet)

1. ☐   As all required additional search fees were timely paid by the applicant, this international search report covers all searchable claims.

2. ☐   As all searchable claims could be searched without effort justifying an additional fee, this Authority did not invite payment of any additional fee.

3. ☐   As only some of the required additional search fees were timely paid by the applicant, this international search report covers only those claims for which fees were paid, specifically claims Nos.:

4. ☒   No required additional search fees were timely paid by the applicant. Consequently, this international search report is restricted to the invention first mentioned in the claims; it is covered by claims Nos.:
   Claims 1 to 44, 48 to 53, 56 to 57 and 68

**Remark on Protest**     ☐   The additional search fees were accompanied by the applicant's protest.

                                  ☐   No protest accompanied the payment of additional search fees.

**INTERNATIONAL SEARCH REPORT**

International application No.

PCT/JP2004/017307

Continuation of Box No.III of continuation of first sheet(2)

observed in luciferase assay (*in vitro* system), in modifying genome.
(5) The invention according to claim 70 relates to a method of using
a promoter, which has an activity of 0.1 rlu or higher observed in luciferase
assay (*in vitro* system), in confirming an LTR retrotransposon.

Since there is no technical relationship among these groups of
inventions involving one or more of the same or corresponding special
technical features, these groups of inventions are not considered as
being so linked as to form a single general inventive concept.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6150160 A
- US 20030121063 A
- US 4708871 A
- US 4554101 A
- JP 3000229 A
- WO 9113150 A
- US 4873191 A
- US 5464764 A
- US 5487992 A
- US 5627059 A
- JP 2001054337 A

### Non-patent literature cited in the description

- **RADICE, A.D. et al.** *Mol. Gen. Genet.,* 1994, vol. 244, 606-612
- **OOSUMI et al.** *Nature,* 1995, vol. 378, 873
- **IVICS et al.** *Mol. Gen. Genet.,* 1995, vol. 247, 312-322
- **KOGA et al.** *Nature,* 1996, vol. 383, 30
- **LAM et al.** *J. Mol. Biol.,* 1996, vol. 257, 359-366
- **LAM, W. L. et al.** *Proc. Natl. Acad. Sci. USA,* vol. 93, 10870-10875
- **PLASTERK, RHA.** *TIG,* 1999, vol. 15, 326-332
- **PLASTERK RHA.** *Curr. Top. Microbiol. Immunol.,* 1996, vol. 204, 125-143
- **OSTERTAG, E. M. et al.** *Nat Genet.,* 2002, vol. 32, 655-660
- **HEIDMANN O. et al.** *Cell,* 1991, vol. 64, 159-170
- **MIETZ, J. A. et al.** *J. Virol.,* 1987, vol. 61, 3020-3029
- **KOSUGA M. et al.** *Cell Transplant,* September 2000, vol. 9 (5), 675-680
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- Molecular Cloning 2nd ed., Current Protocols in Molecular Biology. DNA Cloning 1: Core Techniques, A Practical Approach. Oxford University Press, 1995, 1-38
- **ALTSCHUL et al.** *J. Mol. Biol.,* 1990, vol. 215, 403-410
- **PEARSON ; LIPMAN.** *Proc. Natl. Acad. Sci., USA,* 1988, vol. 85, 2444-2448
- **SMITH ; WATERMAN.** *J. Mol. Biol.,* 1981, vol. 147, 195-197
- **NEEDLEMAN ; WUNSCH.** *J. Mol. Biol.,* 1970, vol. 48, 443-453
- **SAMBROOK ; FRITSCH ; MANIATIS.** Molecular Cloning: A Laboratory Manual. Cold Spring Harbor Laboratory, 1989
- **ANDERSON et al.** Nucleic Acid Hybridization: A Practical Approach. IRL Press Limited) (Oxford Express
- 683. **SUGGS et al.** Developmental Biology Using Purified Genes. 1981
- **GEYSEN et al.** *Proc. Natl. Acad. Sci. USA,* 1984, vol. 81, 3998
- **KYTE, J. ; DOOLITTLE, R.F.** *J. Mol. Biol.,* 1982, vol. 157 (1), 105-132
- **KOHLER ; MILSTEIN.** *Nature,* 1975, vol. 256, 495
- **BUCK et al.** *In vitro,* 1982, vol. 18, 377
- *J. Biochem.,* 1987, vol. 101, 1307
- *J. Biol. Chem.,* 1993, vol. 268, 22782-22787
- Current Protocols in Molecular Biology. Wiley, 1988
- **SAMBROOK J. et al.** Molecular Cloning: A Laboratory Manual. Cold Spring Harbor Laboratory Press, 1987
- Gene Introduction & Expression Analysis Experimental Procedure. **YODOSHA.** Experimental Medicine. 1997
- *Nat Genet.,* December 2002, vol. 32, 526-32
- **M.MARKKULA et al.** *Rev.Reprod.,* 1996, vol. 1, 97-106
- **R. T. WALL et al.** *J. Dairy Sci.,* 1997, vol. 80, 2213-2224
- **DALTON et al.** *Adv. Exp. Med. Biol.,* 1997, vol. 411, 419-428
- **H. LUBON et al.** *Transfus. Med. Rev.,* 1996, vol. 10, 131-143
- **STANFORD WL. et al.** *Nature Genetics,* 2001, vol. 2, 756-768

- **ZAMBROWICZ et al.** *Nature,* 1998, vol. 392, 608-611
- **GOSSLER, A. et al.** *Science,* 1989, vol. 244, 463-465
- **SKARNES, W.C. et al.** *Genes Dev,* 1992, vol. 6, 903-918
- **FRIEDRICH, G. et al.** *Genes Dev,* 1991, vol. 5, 1513-1523
- **G. LUO et al.** *Nature Genetics,* 2000, vol. 26, 424-429
- **WURST, W. et al.** *Genetics,* 1995, vol. 139, 889-899
- **ZAMBROWICZ, B. P. et al.** *Nature,* 1998, vol. 392, 608-611
- *Proc.Natl. Acad. Sci. USA,* 1989, vol. 86, 8932-8935
- *Nature,* 1989, vol. 342, 435-438
- Jikken Igaku Bessatsu, Shin-tei, Idenshi Kogaku Handobukku Kaitei Daisanhan. **YODOSHA.** Experimental Medicine, Suppl. New Revision, Gene Engineering Handbook. 1999, 239-256
- **S. AIZAWA.** jiin taagettingu - ES saibo wo mochiita hen'i mausu no sakusei. *Jikken Igaku,* 1995
- Gene Targeting. Oxford University Press, 2000
- Eukaryotic transposable, elements as tools to study gene structure and function. **KAISER et al.** Mobile Genetic Elements. IRL Press, 1995, 69-100
- **ANDERSON et al.** *Mol. Mar. Biol. Biotech.,* 1996, vol. 5, 105-113
- *The Journal -of the American Medical Association,* 1967, vol. 199, 519
- *Science,* 1952, vol. 122, 501
- *Virology,* 1959, vol. 8, 396
- *Proceedings of the Society for the Biological Medicine,* 1950, vol. 73, 1
- *Proc. Natl. Acad. Sci. USA,* 1989, vol. 86, 8932-8935
- Experimental Medicine. gene engineering handbook, revised version III. Yodo-sha, 1999, 239-256
- **ISHIHARA ; TANAKA.** *FEBS Lett.,* 1997, vol. 418, 205-209
- **KIMBERLAND et al.** *Hum Mol Genet,* 1999, vol. 8, 1557-1560
- **JOHN MORAN.** *Cell,* 2002, vol. 110, 315
- **NIWA et al.** *Gene,* 1991, vol. 108, 193-199
- **KOST et al.** *Nucleic Acids Res.,* 1983, vol. 11, 8287-8301